(19) Europäisches Patentamt / European Patent Office / Office européen des brevets

(11) **EP 4 729 510 A1**

(12) **EUROPEAN PATENT APPLICATION**
published in accordance with Art. 153(4) EPC

(43) Date of publication:
**22.04.2026 Bulletin 2026/17**

(21) Application number: **24835406.0**

(22) Date of filing: **04.07.2024**

(51) International Patent Classification (IPC):
***C07D 209/88*** *(2006.01)* ***C07D 209/90*** *(2006.01)*
***A61K 31/403*** *(2006.01)* ***A61P 17/00*** *(2006.01)*

(52) Cooperative Patent Classification (CPC):
**A61K 31/403; A61P 17/00; C07D 209/88; C07D 209/90**

(86) International application number:
**PCT/CN2024/103648**

(87) International publication number:
**WO 2025/007928 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.07.2023 CN 202310814278**
**06.03.2024 CN 202410257182**
**25.03.2024 CN 202410340315**

(71) Applicant: **Suzhou Merna Therapeutics Co. Ltd**
**Suzhou, Jiangsu 215000 (CN)**

(72) Inventors:
- **WANG, Jingfang**
  **Suzhou, Jiangsu 215000 (CN)**
- **PENG, Cheng**
  **Suzhou, Jiangsu 215000 (CN)**
- **SONG, Zhidong**
  **Suzhou, Jiangsu 215000 (CN)**
- **WANG, Ying**
  **Suzhou, Jiangsu 215000 (CN)**
- **TONG, Siyu**
  **Suzhou, Jiangsu 215000 (CN)**

(74) Representative: **karo IP**
**Patentanwälte PartG mbB**
**Steinstraße 16-18**
**40212 Düsseldorf (DE)**

# (54) INDOLE COMPOUND, PREPARATION METHOD THEREFOR AND USE THEREOF

(57) A compound of formula I, or a stereoisomer, a tautomer, a solvate, a hydrate, a prodrug, a stable isotope derivative, and a pharmaceutically acceptable salt thereof is provided:

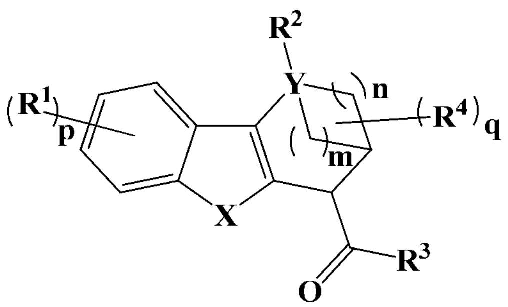

I

.

The compound has activity in preventing and/or treating a skin-related disease.

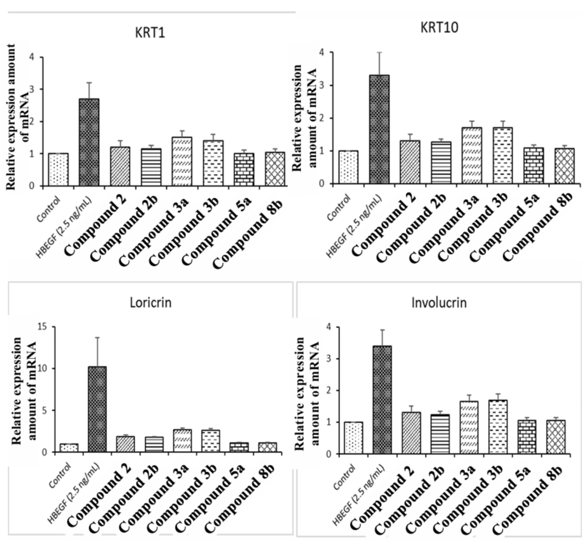


FIG. 7

EP 4 729 510 A1

## Description

### CROSS REFERENCE TO RELATED APPLICATION

The present disclosure claims priority to Chinese Patent Application No. 202310814278.1, entitled "INDOLE COMPOUND, PREPARATION METHOD THEREFOR AND USE THEREOF", filed with the China National Intellectual Property Administration on July 5, 2023, Chinese Patent Application No. 202410257182.4, entitled "INDOLE COMPOUND, PREPARATION METHOD THEREFOR AND USE THEREOF", filed with the China National Intellectual Property Administration on March 6, 2024, and Chinese Patent Application No. 202410340315.4, entitled "INDOLE COMPOUND, PREPARATION METHOD THEREFOR AND USE THEREOF", filed with the China National Intellectual Property Administration on March 25, 2024, the entire contents of the above applications are incorporated herein by reference.

### FIELD OF TECHNOLOGY

The present disclosure relates to the field of biomedicine, and more particularly relates to use of an indole compound in prevention and/or treatment of a skin-related disease, especially a skin hyperkeratosis-related disease.

### BACKGROUND

The epidermis is the outermost layer of tissues of the skin, and the stratum corneum is the outermost layer of the epidermis and is derived from basal cells of the skin. The stratum corneum serves as the first line of defense between the human body and the external environment, and has primary effects including retaining moisture, obtaining mechanical strength, selectively transporting molecules, and protecting against external infections. Hyperkeratosis refers to excessive thickening of the mucosa or the stratum corneum of the skin, which is mainly related to aberrant keratogenesis of an outer layer of the skin. Generally, an increase in the thickness of the stratum corneum is caused by exogenous factors (such as chronic physical or chemical damage) and endogenous factors (such as chronic inflammations and side effects of drugs) (Maria Vastarella et al. Drug Safety 2020, 43(5), 395-408). The hyperkeratosis is commonly observed in various skin diseases, such as acanthosis, eczema, ichthyosis, psoriasis, keratosis, systemic lupus erythematosus, and various types of dermatitis, and may also occur as a side effect of various tumor therapies and medications, such as chemotherapeutic drugs, kinase inhibitors, immunomodulators, and immune checkpoint inhibitors (Austin Greco et al. Cancers 2019, 11(12), 1950; Samantha R. Ellis et al. Journal of the American Academy of Dermatology 2022, 83(4), 1130-1143). Currently, there are no drugs targeting hyperkeratosis, and only alleviation can be achieved clinically.

m6A methylation modification is a most prevalent mRNA modification in mammals, which can regulate multiple signaling pathways and cellular processes (such as growth, development, and diseases), thus playing critical biological actions (Frye M., et al. Science 2018, 361, 2073-2092; Yang C., et al. Cell Death & Disease 2020, 11, 960; Meyer K.D. & Jaffrey S.R. Nature Review Molecular Cell Biology 2014, 15, 313-326). The m6A methylation modification of mRNA, miRNA, circRNA, and lncRNA is a dynamically reversible process, in which methyltransferases (such as METTL3, METTL14, and METTL16) bind methyl to RNA, while demethylases (FTO and ALKBH5) can remove the methyl from RNA, thereby forming an m6A regulation basis. m6A affects processes, such as processing, translation, and degradation, of RNA by recruiting specific binding proteins (such as YTHDF1, YTHDF2, YTHDC1, and IGF2BP), thereby resulting in changes in downstream protein functions and cellular biological behaviors (Hsu P.J., et al. Journal of Biological Chemistry 2019, 294, 19889-19895; Yao Y., et al. FASEB Journal 2019, 33, 7529-7544).

Existing studies indicate that m6A is closely associated with the occurrence and development of skin-related diseases. The mRNA expression levels of METTL3 and ALKBH5 in tumor tissues of patients with acral melanoma are significantly higher than those in para-carcinoma tissues, and the mRNA expression level of METTL3 in patients with advanced acral melanoma is significantly higher than that in early-stage patients (Le Zhanghui, Preliminary Study on the Pathogenesis of LncRNA and RNA m6A Methylation in Acral Melanoma, Dissertation, 2019). In terms of diagnosis of melanoma, m6A-specific binding proteins YTHDF1 and HNRNPA2B1 can serve as novel biomarkers (Li T.D., et al. Cancer Cell 2020, 20, 239). In keratinocytes, long-term and low-level arsenic exposure can inhibit m6A in cells and result in selective autophagy, thereby inducing the occurrence of skin tumors (Cui Y.H., et al. Nature Communications 2021, 12, 2183). After knockout of m6A methylases, the induced proliferation of epidermal cells is suppressed, while m6A binding protein YTHDC1 can affect epidermal autophagy by regulating the stability of mRNA in the keratinocytes (Lee J. et al., EMBO J. 2021, 40(8): e106276; Liang D.F. et al., Autophage 2022, 18(6): 1318-1337). In a mouse model with psoriasis, m6A can regulate the expression of cytokines IL-17A and TNFalpha (Wang Y. et al., Front. Cell. Dev. Biol. 2020, 8, 591629) to significantly decrease the m6A level in skin tissues of mice with psoriasis and obviously increase the protein content of m6A demethylase FTO, thereby playing a protective role against psoriasis (Cui L. et al., J. Investig. Dermatol. 2020, 140(7): S6). Additionally, the m6A methylases, the demethylases, and the binding proteins are also considered blood

markers for systemic lupus erythematosus (Li L. et al., Mol. Immunol. 2018, 93, 55-63). However, currently, there are no methods for preventing or treating skin diseases by regulating RNA m6A methylation.

## SUMMARY

Problems to be solved

To solve the above problems, the present disclosure provides an indole compound, where the compound can be used for preventing and/or treating a skin-related disease.

Solutions to solve the problems

The present disclosure provides a compound of formula I, or a stereoisomer, a tautomer, a solvate, a hydrate, a prodrug, a stable isotope derivative, and a pharmaceutically acceptable salt thereof:

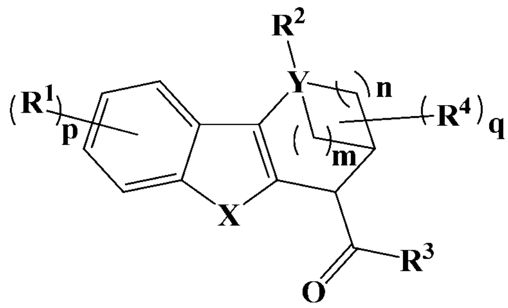

I

where,

- X is selected from $NR^a$, O, and S;
- Y is selected from C and N;
- $R^1$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^1$;
- $R^2$ is selected from hydrogen, deuterium, halogen, $-S(O)_2-R^d$, $-C(O)-R^e$, $-NR^iR^j$, substituted or unsubstituted alkyl, alkoxy, haloalkyl, cycloalkyl, aryl, heteroaryl, and heterocyclyl, where the substituted means substituted with one or more $Q^2$;
- $R^3$ is selected from hydrogen, deuterium, hydroxyl, $-NR^iR^j$, substituted or unsubstituted alkyl, alkoxy, haloalkyl, cycloalkyl, aryl, heteroaryl, and heterocyclyl, where the substituted means substituted with one or more $Q^3$;
- $R^4$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^4$;
- $R^a$ is selected from hydrogen, deuterium, alkyl, and haloalkyl;
- each $R^b$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^5$;
- each $R^c$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^6$;
- each of $R^i$ and $R^j$ is respectively and independently selected from hydrogen, deuterium, $-S(O)_2-R^d$, substituted or unsubstituted alkyl, alkoxy, haloalkyl, cycloalkyl, aryl, heteroaryl, and heterocyclyl, where the substituted means substituted with one or more $Q^7$;
- each $R^d$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^8$;
- each $R^e$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^9$;

each of $Q^1$, $Q^2$, $Q^3$, $Q^4$, $Q^5$, $Q^6$, $Q^7$, $Q^8$, and $Q^9$ is independently selected from hydrogen, deuterium, halogen, cyano, hydroxyl, mercapto, amino, substituted or unsubstituted alkyl, alkoxy, haloalkyl, cycloalkyl, aryl, heteroaryl, and heterocyclyl, where the substituted means substituted with one or more $Q^{10}$;
$Q^{10}$ is selected from hydrogen, deuterium, halogen, alkyl, alkoxy, haloalkyl, cycloalkyl, aryl, heteroaryl, and heterocyclyl;
each of m and n is independently 0, 1, or 2; and
each of p and q is independently 0, 1, 2, 3, or 4.

Beneficial effects of the present disclosure

The compound of formula I provided by the present disclosure can be used for preventing and/or treating an abnormal RNA m6A methylation-related disease, where the abnormal RNA m6A methylation-related disease is a skin-related disease. The skin-related disease is a skin hyperkeratosis-related disease, including but not limited to rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

## BRIEF DESCRIPTION OF THE DRAWINGS

FIG. 1 describes exemplary results of a cellular m6A level and an NAP1L2 m6A level determined by nanopore sequencing after administration of sorafenib, capecitabine, docetaxel, and osimertinib to HaCat keratinocytes for 24 hours.
FIG. 2 describes exemplary results of a cellular m6A level and an NAP1L2 m6A level in skin tissues of hind limb toes of mice in Example 15.
FIG. 3 describes exemplary results of an NAP1L2 mRNA expression amount determined by RT-PCR after administration of a human recombinant METTL3 protein and a human recombinant FTO protein or transfection with METTL3 siRNA and FTO siRNA to HaCat keratinocytes for 24 hours.
FIG. 4 describes exemplary results of mRNA and protein expression amounts of matrix metalloproteinases determined by RT-PCR and Western blot after administration of a human recombinant NAP1L2 protein, sorafenib, capecitabine, docetaxel, and osimertinib or transfection with NAP1L2 siRNA to HaCat keratinocytes for 24 hours.
FIG. 5 describes exemplary results of mRNA and protein expression amounts of keratinocyte differentiation markers KRT1, KRT10, Loricrin, and Involucrin determined by RT-PCR and Western blot after administration of a human recombinant HBEGF protein and an HBEGF antibody or transfection with METTL3 and NAP1L2 siRNA to HaCat keratinocytes for 24 hours.
FIG. 6 describes exemplary results of a cellular m6A methylation inhibition level determined by LC-MS after administration of compounds 1, 2, 2b, 5a, 8b, nicotinamide, and UZH2 to THP-1 cells for 24 hours.
FIG. 7 describes exemplary results of mRNA expression amounts of keratinocyte differentiation markers KRT1, KRT10, Loricrin, and Involucrin determined by RT-PCR after administration of a human recombinant HBEGF protein and compounds 2, 2b, 3a, 3b, 5a, and 8b to HaCaT keratinocytes for 24 hours.
FIG. 8 describes exemplary stratum corneum thickness measurement results of paw plantar skin of rats in Example 22.
FIG. 9 describes exemplary histopathological results of epithelial blisters, inflammatory cell infiltration, and skin tissue congestion situations of paw plantar skin of rats after tissue staining in Example 22.
FIG. 10 describes exemplary histopathological scoring results of the epithelial blisters, the inflammatory cell infiltration, and the skin tissue congestion situations of the paw plantar skin of the rats after tissue staining in Example 22.
FIG. 11 describes exemplary results of the skin of hind limb toes of mice in Example 23.
FIG. 12 describes exemplary results of the degree of swelling of hind limb toes of mice in Example 23.
FIG. 13 describes exemplary histopathological results of skin tissues of hind limb toes of mice in Example 23.
FIG. 14 describes exemplary immunohistochemical results of cytokine IL-8 in skin tissues of hind limb toes of mice in capecitabine modeling series groups in Example 23.
FIG. 15 describes exemplary immunohistochemical results of cytokine IL-6 in skin tissues of hind limb toes of mice in docetaxel modeling series groups in Example 23.
FIG. 16 describes exemplary results of the skin of hind limb toes of mice in Example 24.
FIG. 17 describes exemplary results of the degree of swelling of hind limb toes of mice in Example 24.
FIG. 18 describes exemplary histopathological results of skin tissues of hind limb toes of mice in Example 24.

FIG. 19 describes exemplary immunohistochemical results of cytokine IL-1β in skin tissues of hind limb toes of mice in sorafenib modeling series groups in Example 24.

FIG. 20 describes exemplary immunohistochemical results of cytokine IL-1β in skin tissues of hind limb toes of mice in osimertinib modeling series groups in Example 24.

## DESCRIPTION OF THE EMBODIMENTS

To make the technical solutions and beneficial effects of the present disclosure more apparent and understandable, the following provides a detailed description by the way of listing specific examples. The drawings are not necessarily drawn to scale, and local features may be enlarged or reduced to more clearly show details of the local features. Unless otherwise defined, technical and scientific terms used herein have the same meanings as those in the technical field to which the present application belongs.

In one aspect, the present disclosure provides a compound of formula I, or a stereoisomer, a tautomer, a solvate, a hydrate, a prodrug, a stable isotope derivative, and a pharmaceutically acceptable salt thereof:

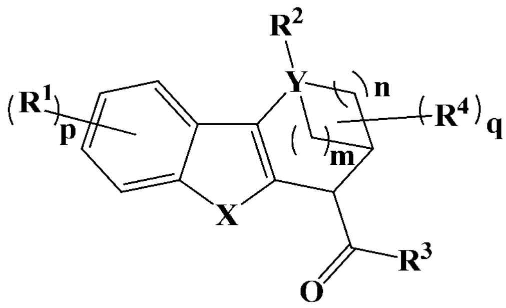

I

where,

X is selected from $NR^a$, O, and S;

Y is selected from C and N;

$R^1$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^1$;

$R^2$ is selected from hydrogen, deuterium, halogen, $-S(O)_2-R^d$, $-C(O)-R^e$, $-NR^iR^j$, substituted or unsubstituted alkyl, alkoxy, haloalkyl, cycloalkyl, aryl, heteroaryl, and heterocyclyl, where the substituted means substituted with one or more $Q^2$;

$R^3$ is selected from hydrogen, deuterium, hydroxyl, $-NR^iR^j$, substituted or unsubstituted alkyl, alkoxy, haloalkyl, cycloalkyl, aryl, heteroaryl, and heterocyclyl, where the substituted means substituted with one or more $Q^3$;

$R^4$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^4$;

$R^a$ is selected from hydrogen, deuterium, alkyl, and haloalkyl;

each $R^b$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^5$;

each $R^c$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^6$;

each of $R^i$ and $R^j$ is respectively and independently selected from hydrogen, deuterium, $-S(O)_2-R^d$, substituted or unsubstituted alkyl, alkoxy, haloalkyl, cycloalkyl, aryl, heteroaryl, and heterocyclyl, where the substituted means substituted with one or more $Q^7$;

each $R^d$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^8$;

each $R^e$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^9$;

each of $Q^1$, $Q^2$, $Q^3$, $Q^4$, $Q^5$, $Q^6$, $Q^7$, $Q^8$, and $Q^9$ is independently selected from hydrogen, deuterium, halogen, cyano, hydroxyl, mercapto, amino, substituted or unsubstituted alkyl, alkoxy, haloalkyl, cycloalkyl, aryl, heteroaryl, and heterocyclyl, where the substituted means substituted with one or more $Q^{10}$;
$Q^{10}$ is selected from hydrogen, deuterium, halogen, alkyl, alkoxy, haloalkyl, cycloalkyl, aryl, heteroaryl, and heterocyclyl;
each of m and n is independently 0, 1, or 2; and
each of p and q is independently 0, 1, 2, 3, or 4.

In certain embodiments, X is selected from $NR^a$ and O.
In certain embodiments, X is selected from $NR^a$.
In certain embodiments, $R^a$ is selected from hydrogen, deuterium, and alkyl.
In certain embodiments, $R^a$ is selected from hydrogen and $C_{1-3}$ alkyl.
In certain embodiments, $R^a$ is selected from hydrogen and methyl.
In certain embodiments, $R^1$ is selected from hydrogen, deuterium, halogen, $-OR^b$, $-NR^iR^j$, substituted or unsubstituted alkyl, cycloalkyl, aryl, heteroaryl, and heterocyclyl.
In certain embodiments, $R^1$ is selected from hydrogen, deuterium, halogen, $-OR^b$, $-NR^iR^j$, substituted or unsubstituted $C_{1-6}$ alkyl, $C_{3-7}$ cycloalkyl, $C_{6-10}$ aryl, 5-10 membered heteroaryl, and 3-10 membered heterocyclyl.
In certain embodiments, $R^1$ is selected from hydrogen, deuterium, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl.
In certain embodiments, $R^1$ is selected from hydrogen, fluorine, chlorine, $-OR^b$, $-NR^iR^j$, substituted or unsubstituted methyl, and cyclopropyl.
In certain embodiments, $Q^1$ is selected from hydrogen, halogen, cyano, hydroxyl, amino, and substituted or unsubstituted alkyl.
In certain embodiments, $Q^1$ is selected from hydrogen, halogen, and substituted or unsubstituted $C_{1-3}$ alkyl.
In certain embodiments, $Q^1$ is selected from hydrogen and halogen.
In certain embodiments, $Q^1$ is selected from hydrogen.
In certain embodiments, $R^b$ is selected from hydrogen, substituted or unsubstituted alkyl, and cycloalkyl.
In certain embodiments, $R^b$ is selected from substituted or unsubstituted $C_{1-6}$ alkyl and $C_{3-7}$ cycloalkyl.
In certain embodiments, $R^b$ is selected from substituted or unsubstituted $C_{1-3}$ alkyl and $C_{3-6}$ cycloalkyl.
In certain embodiments, $R^b$ is selected from substituted or unsubstituted methyl, cyclopropyl, and cyclohexyl.
In certain embodiments, $Q^5$ is selected from hydrogen, halogen, cyano, hydroxyl, amino, and substituted or unsubstituted heteroaryl.
In certain embodiments, $Q^5$ is selected from hydrogen, halogen, and substituted or unsubstituted 5-10 membered heteroaryl.
In certain embodiments, $Q^5$ is selected from hydrogen, halogen, and substituted or unsubstituted 5-6 membered heteroaryl, where the heteroaryl contains 1-4 heteroatoms selected from N, O, and S.
In certain embodiments, $Q^5$ is selected from hydrogen and substituted or unsubstituted pyridyl.
In certain embodiments, $R^c$ is selected from hydrogen, substituted or unsubstituted alkyl, and cycloalkyl.
In certain embodiments, $R^c$ is selected from substituted or unsubstituted $C_{1-6}$ alkyl and $C_{3-7}$ cycloalkyl.
In certain embodiments, $R^c$ is selected from substituted or unsubstituted $C_{1-3}$ alkyl and $C_{3-6}$ cycloalkyl.
In certain embodiments, $R^c$ is selected from substituted or unsubstituted methyl, cyclopropyl, and cyclohexyl.
In certain embodiments, $Q^6$ is selected from hydrogen, halogen, cyano, hydroxyl, amino, and substituted or unsubstituted heteroaryl.
In certain embodiments, $Q^6$ is selected from hydrogen, halogen, and substituted or unsubstituted 5-10 membered heteroaryl.
In certain embodiments, $Q^6$ is selected from hydrogen, halogen, and substituted or unsubstituted 5-6 membered heteroaryl, where the heteroaryl contains 1-4 heteroatoms selected from N, O, and S.
In certain embodiments, $Q^6$ is selected from hydrogen and substituted or unsubstituted pyridyl.
In certain embodiments, $R^i$ is selected from hydrogen, $-S(O)_2-R^d$, substituted or unsubstituted alkyl, cycloalkyl, aryl, and heteroaryl.
In certain embodiments, $R^i$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.
In certain embodiments, $R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.
In certain embodiments, $R^i$ is selected from hydrogen, $-S(O)_2-R^d$, substituted or unsubstituted methyl, ethyl, cyclopentyl, phenyl, pyridyl, furanyl, and imidazolyl.

In certain embodiments, $R^j$ is selected from hydrogen, $-S(O)_2-R^d$, substituted or unsubstituted alkyl, cycloalkyl, aryl, and heteroaryl.

In certain embodiments, $R^j$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^j$ is selected from hydrogen, $-S(O)_2-R^d$, substituted or unsubstituted methyl, ethyl, cyclopentyl, phenyl, pyridyl, furanyl, and imidazolyl.

In certain embodiments, $Q^7$ is selected from hydrogen, halogen, cyano, hydroxyl, amino, and substituted or unsubstituted heteroaryl.

In certain embodiments, $Q^7$ is selected from hydrogen, halogen, and substituted or unsubstituted 5-10 membered heteroaryl.

In certain embodiments, $Q^7$ is selected from hydrogen, halogen, and substituted or unsubstituted 5-6 membered heteroaryl, where the heteroaryl contains 1-4 heteroatoms selected from N, O, and S.

In certain embodiments, $Q^7$ is selected from hydrogen and pyridyl.

In certain embodiments, $R^d$ is selected from hydrogen, substituted or unsubstituted alkyl, cycloalkyl, aryl, and heteroaryl.

In certain embodiments, $R^d$ is selected from hydrogen, substituted or unsubstituted $C_{1-6}$ alkyl, $C_{3-7}$ cycloalkyl, $C_{6-10}$ aryl, and 5-10 membered heteroaryl.

In certain embodiments, $R^d$ is selected from hydrogen, substituted or unsubstituted $C_{1-3}$ alkyl, $C_{3-6}$ cycloalkyl, and $C_{6-10}$ aryl.

In certain embodiments, $R^d$ is selected from methyl, cyclopropyl, and phenyl.

In certain embodiments, $Q^8$ is selected from hydrogen, halogen, cyano, hydroxyl, mercapto, and amino.

In certain embodiments, $Q^8$ is selected from hydrogen, halogen, and amino.

In certain embodiments, $Q^8$ is selected from hydrogen, fluorine, and chlorine.

In certain embodiments, $Q^8$ is selected from hydrogen.

In certain embodiments, $R^2$ is selected from hydrogen, halogen, $-S(O)_2-R^d$, $-C(O)-R^e$, $-NR^iR^j$, substituted or unsubstituted alkyl, cycloalkyl, aryl, and heteroaryl.

In certain embodiments, $R^2$ is selected from hydrogen, halogen, $-S(O)_2-R^d$, $-C(O)-R^e$, substituted or unsubstituted $C_{1-6}$ alkyl, and $C_{3-7}$ cycloalkyl.

In certain embodiments, $R^2$ is selected from hydrogen, halogen, $-S(O)_2-R^d$, $-C(O)-R^e$, $C_{1-3}$ alkyl, and $C_{3-6}$ cycloalkyl.

In certain embodiments, $R^2$ is selected from hydrogen, fluorine, $-S(O)_2-R^d$, $-C(O)-R^e$, methyl, isopropyl, and cyclopropyl.

In certain embodiments, $Q^2$ is selected from hydrogen, halogen, cyano, hydroxyl, mercapto, and amino.

In certain embodiments, $Q^2$ is selected from hydrogen, halogen, and amino.

In certain embodiments, $Q^2$ is selected from hydrogen, fluorine, and chlorine.

In certain embodiments, $Q^2$ is selected from hydrogen.

In certain embodiments, $R^e$ is selected from hydrogen, substituted or unsubstituted alkyl, aryl, and heterocyclyl.

In certain embodiments, $R^e$ is selected from hydrogen, substituted or unsubstituted $C_{1-6}$ alkyl, $C_{6-10}$ aryl, and 3-10 membered heterocyclyl.

In certain embodiments, $R^e$ is selected from hydrogen, substituted or unsubstituted $C_{1-3}$ alkyl, $C_{6-10}$ aryl, and 3-6 membered heterocyclyl.

In certain embodiments, $R^e$ is selected from hydrogen, substituted or unsubstituted methyl, ethyl, phenyl, and piperidinyl.

In certain embodiments, $Q^9$ is selected from hydrogen, halogen, substituted or unsubstituted alkyl, aryl, and heteroaryl.

In certain embodiments, $Q^9$ is selected from hydrogen, halogen, substituted or unsubstituted $C_{1-6}$ alkyl, $C_{6-10}$ aryl, and 5-10 membered heteroaryl.

In certain embodiments, $Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S.

In certain embodiments, $Q^9$ is selected from hydrogen, fluorine, methyl, and pyrrolyl.

In certain embodiments, $Q^{10}$ is selected from hydrogen, halogen, and substituted or unsubstituted alkyl.

In certain embodiments, $Q^{10}$ is selected from hydrogen and halogen.

In certain embodiments, $Q^{10}$ is selected from hydrogen and fluorine.

In certain embodiments, $R^3$ is selected from hydrogen, hydroxyl, $-NR^iR^j$, substituted or unsubstituted alkyl, and alkoxy.

In certain embodiments, $R^3$ is selected from hydrogen, hydroxyl, and $-NR^iR^j$.

In certain embodiments, $R^3$ is selected from $-NR^iR^j$.

In certain embodiments, $Q^3$ is selected from hydrogen, halogen, cyano, hydroxyl, mercapto, and amino.

In certain embodiments, $Q^3$ is selected from hydrogen, halogen, and amino.

In certain embodiments, $Q^3$ is selected from hydrogen, fluorine, and chlorine.

In certain embodiments, $Q^3$ is selected from hydrogen.

In certain embodiments, $R^4$ is selected from hydrogen, halogen, $-OR^b$, and $-NR^iR^j$.

In certain embodiments, $R^4$ is selected from hydrogen and halogen.

In certain embodiments, $R^4$ is selected from hydrogen, fluorine, chlorine, and bromine.

In certain embodiments, $R^4$ is selected from hydrogen and fluorine.

In certain embodiments, $Q^4$ is selected from hydrogen, halogen, cyano, hydroxyl, amino, and substituted or unsubstituted alkyl.

In certain embodiments, $Q^4$ is selected from hydrogen, halogen, and substituted or unsubstituted $C_{1-3}$ alkyl.

In certain embodiments, $Q^4$ is selected from hydrogen and halogen.

In certain embodiments, $Q^4$ is selected from hydrogen.

In certain embodiments, m is 0 or 1.

In certain embodiments, n is 1 or 2.

In certain embodiments, p is 1, 2, or 3.

In certain embodiments, q is 0, 1, or 2.

In certain embodiments, X is selected from $NR^a$;

$R^a$ is selected from hydrogen and $C_{1-3}$ alkyl;

$R^1$ is selected from hydrogen, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl, where the substituted means substituted with one or more $Q^1$;

$Q^1$ is selected from hydrogen and halogen;

$R^b$ is selected from substituted or unsubstituted $C_{1-3}$ alkyl and $C_{3-6}$ cycloalkyl, where the substituted means substituted with one or more $Q^5$;

$Q^5$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, the substituted means substituted with one or more $Q^7$;

$Q^7$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^d$ is selected from hydrogen, substituted or unsubstituted $C_{1-3}$ alkyl, $C_{3-5}$ cycloalkyl, and $C_{6-10}$ aryl, where the substituted means substituted with one or more $Q^8$;

$Q^8$ is selected from hydrogen, fluorine, and chlorine;

$R^2$ is selected from hydrogen, halogen, $-S(O)_2-R^d$, $-C(O)-R^e$, $C_{1-3}$ alkyl, and $C_{3-6}$ cycloalkyl;

$R^e$ is selected from hydrogen, substituted or unsubstituted $C_{1-3}$ alkyl, $C_{6-10}$ aryl, and 3-6 membered heterocyclyl, where the substituted means substituted with one or more $Q^9$;

$Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S, and the substituted means substituted with one or more $Q^{10}$;

$Q^{10}$ is selected from hydrogen and halogen;

$R^3$ is selected from $-NR^iR^j$;

$R^4$ is selected from hydrogen, fluorine, chlorine, and bromine;

m is 0 or 1;

n is 1 or 2;

p is 1, 2, or 3; and

q is 0, 1, or 2.

In certain embodiments, X is selected from $NR^a$;

$R^a$ is selected from hydrogen and methyl;
$R^1$ is selected from hydrogen, fluorine, chlorine, $-OR^b$, $-NR^iR^j$, methyl, and cyclopropyl;
$R^b$ is selected from substituted or unsubstituted methyl, cyclopropyl, and cyclohexyl, where the substituted means substituted with one or more $Q^5$;
$Q^5$ is selected from hydrogen and substituted or unsubstituted pyridyl, where the substituted means substituted with one or more $Q^{10}$;
$R^i$ is selected from hydrogen, $-S(O)_2-R^d$, substituted or unsubstituted methyl, ethyl, cyclopentyl, phenyl, pyridyl, furanyl, and imidazolyl, where the substituted means substituted with one or more $Q^7$;
$R^j$ is selected from hydrogen, $-S(O)_2-R^d$, substituted or unsubstituted methyl, ethyl, cyclopentyl, phenyl, pyridyl, furanyl, and imidazolyl, where the substituted means substituted with one or more $Q^7$;
$Q^7$ is selected from hydrogen and pyridyl;
$R^d$ is selected from methyl, cyclopropyl, and phenyl;
$R^2$ is selected from hydrogen, fluorine, $-S(O)_2-R^d$, $-C(O)-R^e$, methyl, isopropyl, and cyclopropyl;
$R^e$ is selected from hydrogen, substituted or unsubstituted methyl, ethyl, phenyl, and piperidinyl, where the substituted means substituted with one or more $Q^9$;
$Q^9$ is selected from hydrogen, fluorine, methyl, and pyrrolyl;
$Q^{10}$ is selected from hydrogen and fluorine;
$R^3$ is selected from $-NR^iR^j$;
$R^4$ is selected from hydrogen and fluorine;
m is 0 or 1;
n is 1 or 2;
p is 1, 2, or 3; and
q is 0, 1, or 2.

In another aspect, the present disclosure provides a compound of formula I-1, or a stereoisomer, a tautomer, a solvate, a hydrate, a prodrug, a stable isotope derivative, and a pharmaceutically acceptable salt thereof:

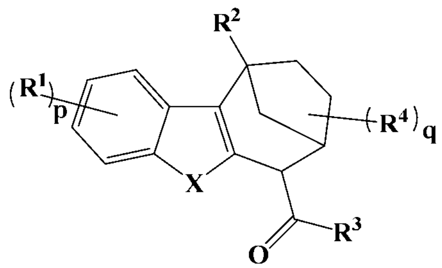

I-1

where,

X is selected from $NR^a$, O, and S;
$R^1$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^1$;
$R^2$ is selected from hydrogen, deuterium, halogen, $-S(O)_2-R^d$, $-C(O)-R^e$, $-NR^iR^j$, substituted or unsubstituted alkyl, alkoxy, haloalkyl, cycloalkyl, aryl, heteroaryl, and heterocyclyl, where the substituted means substituted with one or more $Q^2$;
$R^3$ is selected from hydrogen, deuterium, hydroxyl, $-NR^iR^j$, substituted or unsubstituted alkyl, alkoxy, haloalkyl, cycloalkyl, aryl, heteroaryl, and heterocyclyl, where the substituted means substituted with one or more $Q^3$;
$R^4$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^4$;
$R^a$ is selected from hydrogen, deuterium, alkyl, and haloalkyl;
each $R^b$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^5$;
each $R^c$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or

more $Q^6$;

each of $R^i$ and $R^j$ is respectively and independently selected from hydrogen, deuterium, $-S(O)_2-R^d$, substituted or unsubstituted alkyl, alkoxy, haloalkyl, cycloalkyl, aryl, heteroaryl, and heterocyclyl, where the substituted means substituted with one or more $Q^7$;

each $R^d$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^8$;

each $R^e$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^9$;

each of $Q^1$, $Q^2$, $Q^3$, $Q^4$, $Q^5$, $Q^6$, $Q^7$, $Q^8$, and $Q^9$ is independently selected from hydrogen, deuterium, halogen, cyano, hydroxyl, mercapto, amino, substituted or unsubstituted alkyl, alkoxy, haloalkyl, cycloalkyl, aryl, heteroaryl, and heterocyclyl, where the substituted means substituted with one or more $Q^{10}$;

$Q^{10}$ is selected from hydrogen, deuterium, halogen, alkyl, alkoxy, haloalkyl, cycloalkyl, aryl, heteroaryl, and heterocyclyl; and

p and q are each independently 0, 1, 2, 3, or 4.

In certain embodiments, X is selected from $NR^a$ and O.

In certain embodiments, X is selected from $NR^a$.

In certain embodiments, $R^a$ is selected from hydrogen, deuterium, and alkyl.

In certain embodiments, $R^a$ is selected from hydrogen and $C_{1-3}$ alkyl.

In certain embodiments, $R^a$ is selected from hydrogen and methyl.

In certain embodiments, $R^1$ is selected from hydrogen, deuterium, halogen, $-OR^b$, $-NR^iR^j$, substituted or unsubstituted alkyl, cycloalkyl, aryl, heteroaryl, and heterocyclyl.

In certain embodiments, $R^1$ is selected from hydrogen, deuterium, halogen, $-OR^b$, $-NR^iR^j$, substituted or unsubstituted $C_{1-6}$ alkyl, $C_{3-7}$ cycloalkyl, $C_{6-10}$ aryl, 5-10 membered heteroaryl, and 3-10 membered heterocyclyl.

In certain embodiments, $R^1$ is selected from hydrogen, deuterium, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl.

In certain embodiments, $R^1$ is selected from hydrogen, fluorine, chlorine, $-OR^b$, $-NR^iR^j$, substituted or unsubstituted methyl, and cyclopropyl.

In certain embodiments, $Q^1$ is selected from hydrogen, halogen, cyano, hydroxyl, amino, and substituted or unsubstituted alkyl.

In certain embodiments, $Q^1$ is selected from hydrogen, halogen, and substituted or unsubstituted $C_{1-3}$ alkyl.

In certain embodiments, $Q^1$ is selected from hydrogen and halogen.

In certain embodiments, $Q^1$ is selected from hydrogen.

In certain embodiments, $R^b$ is selected from hydrogen, substituted or unsubstituted alkyl, and cycloalkyl.

In certain embodiments, $R^b$ is selected from substituted or unsubstituted $C_{1-6}$ alkyl and $C_{3-7}$ cycloalkyl.

In certain embodiments, $R^b$ is selected from substituted or unsubstituted $C_{1-3}$ alkyl and $C_{3-6}$ cycloalkyl.

In certain embodiments, $R^b$ is selected from substituted or unsubstituted methyl, cyclopropyl, and cyclohexyl.

In certain embodiments, $Q^5$ is selected from hydrogen, halogen, cyano, hydroxyl, amino, and substituted or unsubstituted heteroaryl.

In certain embodiments, $Q^5$ is selected from hydrogen, halogen, and substituted or unsubstituted 5-10 membered heteroaryl.

In certain embodiments, $Q^5$ is selected from hydrogen, halogen, and substituted or unsubstituted 5-6 membered heteroaryl, where the heteroaryl contains 1-4 heteroatoms selected from N, O, and S.

In certain embodiments, $Q^5$ is selected from hydrogen and substituted or unsubstituted pyridyl.

In certain embodiments, $R^c$ is selected from hydrogen, substituted or unsubstituted alkyl, and cycloalkyl.

In certain embodiments, $R^c$ is selected from substituted or unsubstituted $C_{1-6}$ alkyl and $C_{3-7}$ cycloalkyl.

In certain embodiments, $R^c$ is selected from substituted or unsubstituted $C_{1-3}$ alkyl and $C_{3-6}$ cycloalkyl.

In certain embodiments, $R^c$ is selected from substituted or unsubstituted methyl, cyclopropyl, and cyclohexyl.

In certain embodiments, $Q^6$ is selected from hydrogen, halogen, cyano, hydroxyl, amino, and substituted or unsubstituted heteroaryl.

In certain embodiments, $Q^6$ is selected from hydrogen, halogen, and substituted or unsubstituted 5-10 membered heteroaryl.

In certain embodiments, $Q^6$ is selected from hydrogen, halogen, and substituted or unsubstituted 5-6 membered heteroaryl, where the heteroaryl contains 1-4 heteroatoms selected from N, O, and S.

In certain embodiments, $Q^6$ is selected from hydrogen and substituted or unsubstituted pyridyl.

In certain embodiments, $R^i$ is selected from hydrogen, $-S(O)_2-R^d$, substituted or unsubstituted alkyl, cycloalkyl, aryl, and heteroaryl.

In certain embodiments, $R^i$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^i$ is selected from hydrogen, $-S(O)_2-R^d$, substituted or unsubstituted methyl, ethyl, cyclopentyl, phenyl, pyridyl, furanyl, and imidazolyl.

In certain embodiments, $R^j$ is selected from hydrogen, $-S(O)_2-R^d$, substituted or unsubstituted alkyl, cycloalkyl, aryl, and heteroaryl.

In certain embodiments, $R^j$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^j$ is selected from hydrogen, $-S(O)_2-R^d$, substituted or unsubstituted methyl, ethyl, cyclopentyl, phenyl, pyridyl, furanyl, and imidazolyl.

In certain embodiments, $Q^7$ is selected from hydrogen, halogen, cyano, hydroxyl, amino, and substituted or unsubstituted heteroaryl.

In certain embodiments, $Q^7$ is selected from hydrogen, halogen, and substituted or unsubstituted 5-10 membered heteroaryl.

In certain embodiments, $Q^7$ is selected from hydrogen, halogen, and substituted or unsubstituted 5-6 membered heteroaryl, where the heteroaryl contains 1-4 heteroatoms selected from N, O, and S.

In certain embodiments, $Q^7$ is selected from hydrogen and pyridyl.

In certain embodiments, $R^d$ is selected from hydrogen, substituted or unsubstituted alkyl, cycloalkyl, aryl, and heteroaryl.

In certain embodiments, $R^d$ is selected from hydrogen, substituted or unsubstituted $C_{1-6}$ alkyl, $C_{3-7}$ cycloalkyl, $C_{6-10}$ aryl, and 5-10 membered heteroaryl.

In certain embodiments, $R^d$ is selected from hydrogen, substituted or unsubstituted $C_{1-3}$ alkyl, $C_{3-6}$ cycloalkyl, and $C_{6-10}$ aryl.

In certain embodiments, $R^d$ is selected from methyl, cyclopropyl, and phenyl.

In certain embodiments, $Q^8$ is selected from hydrogen, halogen, cyano, hydroxyl, mercapto, and amino.

In certain embodiments, $Q^8$ is selected from hydrogen, halogen, and amino.

In certain embodiments, $Q^8$ is selected from hydrogen, fluorine, and chlorine.

In certain embodiments, $Q^8$ is selected from hydrogen.

In certain embodiments, $R^2$ is selected from hydrogen, halogen, $-S(O)_2-R^d$, $-C(O)-R^e$, $-NR^iR^j$, substituted or unsubstituted alkyl, cycloalkyl, aryl, and heteroaryl.

In certain embodiments, $R^2$ is selected from hydrogen, halogen, $-S(O)_2-R^d$, $-C(O)-R^e$, substituted or unsubstituted $C_{1-6}$ alkyl, and $C_{3-7}$ cycloalkyl.

In certain embodiments, $R^2$ is selected from hydrogen, halogen, $-S(O)_2-R^d$, $-C(O)-R^e$, $C_{1-3}$ alkyl, and $C_{3-6}$ cycloalkyl.

In certain embodiments, $R^2$ is selected from hydrogen, fluorine, $-S(O)_2-R^d$, $-C(O)-R^e$, methyl, isopropyl, and cyclopropyl.

In certain embodiments, $Q^2$ is selected from hydrogen, halogen, cyano, hydroxyl, mercapto, and amino.

In certain embodiments, $Q^2$ is selected from hydrogen, halogen, and amino.

In certain embodiments, $Q^2$ is selected from hydrogen, fluorine, and chlorine.

In certain embodiments, $Q^2$ is selected from hydrogen.

In certain embodiments, $R^e$ is selected from hydrogen, substituted or unsubstituted alkyl, aryl, and heterocyclyl.

In certain embodiments, $R^e$ is selected from hydrogen, substituted or unsubstituted $C_{1-6}$ alkyl, $C_{6-10}$ aryl, and 3-10 membered heterocyclyl.

In certain embodiments, $R^e$ is selected from hydrogen, substituted or unsubstituted $C_{1-3}$ alkyl, $C_{6-10}$ aryl, and 3-6 membered heterocyclyl.

In certain embodiments, $R^e$ is selected from hydrogen, substituted or unsubstituted methyl, ethyl, phenyl, and piperidinyl.

In certain embodiments, $Q^9$ is selected from hydrogen, halogen, substituted or unsubstituted alkyl, aryl, and heteroaryl.

In certain embodiments, $Q^9$ is selected from hydrogen, halogen, substituted or unsubstituted $C_{1-6}$ alkyl, $C_{6-10}$

aryl, and 5-10 membered heteroaryl.

In certain embodiments, $Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S.

In certain embodiments, $Q^9$ is selected from hydrogen, fluorine, methyl, and pyrrolyl.

In certain embodiments, $Q^{10}$ is selected from hydrogen, halogen, and substituted or unsubstituted alkyl.

In certain embodiments, $Q^{10}$ is selected from hydrogen and halogen.

In certain embodiments, $Q^{10}$ is selected from hydrogen and fluorine.

In certain embodiments, $R^3$ is selected from hydrogen, hydroxyl, $-NR^iR^j$, substituted or unsubstituted alkyl, and alkoxy.

In certain embodiments, $R^3$ is selected from hydrogen, hydroxyl, and $-NR^iR^j$.

In certain embodiments, $R^3$ is selected from $-NR^iR^j$.

In certain embodiments, $Q^3$ is selected from hydrogen, halogen, cyano, hydroxyl, mercapto, and amino.

In certain embodiments, $Q^3$ is selected from hydrogen, halogen, and amino.

In certain embodiments, $Q^3$ is selected from hydrogen, fluorine, and chlorine.

In certain embodiments, $Q^3$ is selected from hydrogen.

In certain embodiments, $R^4$ is selected from hydrogen, halogen, $-OR^b$, and $-NR^iR^j$.

In certain embodiments, $R^4$ is selected from hydrogen and halogen.

In certain embodiments, $R^4$ is selected from hydrogen, fluorine, chlorine, and bromine.

In certain embodiments, $R^4$ is selected from hydrogen and fluorine.

In certain embodiments, $Q^4$ is selected from hydrogen, halogen, cyano, hydroxyl, amino, and substituted or unsubstituted alkyl.

In certain embodiments, $Q^4$ is selected from hydrogen, halogen, and substituted or unsubstituted $C_{1-3}$ alkyl.

In certain embodiments, $Q^4$ is selected from hydrogen and halogen.

In certain embodiments, $Q^4$ is selected from hydrogen.

In certain embodiments, p is 1, 2, or 3.

In certain embodiments, q is 0, 1, or 2.

In certain embodiments, X is selected from $NR^a$;

$R^a$ is selected from hydrogen and $C_{1-3}$ alkyl;

$R^1$ is selected from hydrogen, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl, where the substituted means substituted with one or more $Q^1$;

$Q^1$ is selected from hydrogen and halogen;

$R^b$ is selected from substituted or unsubstituted $C_{1-3}$ alkyl and $C_{3-6}$ cycloalkyl, where the substituted means substituted with one or more $Q^5$;

$Q^5$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$Q^7$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^d$ is selected from hydrogen, substituted or unsubstituted $C_{1-3}$ alkyl, $C_{3-5}$ cycloalkyl, and $C_{6-10}$ aryl, where the substituted means substituted with one or more $Q^8$;

$Q^8$ is selected from hydrogen, fluorine, and chlorine;

$R^2$ is selected from hydrogen, halogen, $-S(O)_2-R^d$, $-C(O)-R^e$, $C_{1-3}$ alkyl, and $C_{3-6}$ cycloalkyl;

$R^e$ is selected from hydrogen, substituted or unsubstituted $C_{1-3}$ alkyl, $C_{6-10}$ aryl, and 3-6 membered heterocyclyl, where the substituted means substituted with one or more $Q^9$;

$Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S, and the substituted means substituted with one or more $Q^{10}$;

$Q^{10}$ is selected from hydrogen and halogen;

$R^3$ is selected from $-NR^iR^j$;

$R^4$ is selected from hydrogen, fluorine, chlorine, and bromine;
p is 1, 2, or 3; and
q is 0, 1, or 2.

In certain embodiments, X is selected from $NR^a$;

$R^a$ is selected from hydrogen and methyl;
$R^1$ is selected from hydrogen, fluorine, chlorine, $-OR^b$, $-NR^iR^j$, methyl, and cyclopropyl;
$R^b$ is selected from substituted or unsubstituted methyl, cyclopropyl, and cyclohexyl, where the substituted means substituted with one or more $Q^5$;
$Q^5$ is selected from hydrogen and substituted or unsubstituted pyridyl, where the substituted means substituted with one or more $Q^{10}$;
$R^i$ is selected from hydrogen, $-S(O)_2-R^d$, substituted or unsubstituted methyl, ethyl, cyclopentyl, phenyl, pyridyl, furanyl, and imidazolyl, where the substituted means substituted with one or more $Q^7$;
$R^j$ is selected from hydrogen, $-S(O)_2-R^d$, substituted or unsubstituted methyl, ethyl, cyclopentyl, phenyl, pyridyl, furanyl, and imidazolyl, where the substituted means substituted with one or more $Q^7$;
$Q^7$ is selected from hydrogen and pyridyl;
$R^d$ is selected from methyl, cyclopropyl, and phenyl;
$R^2$ is selected from hydrogen, fluorine, $-S(O)_2-R^d$, $-C(O)-R^e$, methyl, isopropyl, and cyclopropyl;
$R^e$ is selected from hydrogen, substituted or unsubstituted methyl, ethyl, phenyl, and piperidinyl, where the substituted means substituted with one or more $Q^9$;
$Q^9$ is selected from hydrogen, fluorine, methyl, and pyrrolyl;
$Q^{10}$ is selected from hydrogen and fluorine;
$R^3$ is selected from $-NR^iR^j$;
$R^4$ is selected from hydrogen and fluorine;
p is 1, 2, or 3; and
q is 0, 1, or 2.

In another aspect, the present disclosure provides a compound of formula I-2, or a stereoisomer, a tautomer, a solvate, a hydrate, a prodrug, a stable isotope derivative, and a pharmaceutically acceptable salt thereof:

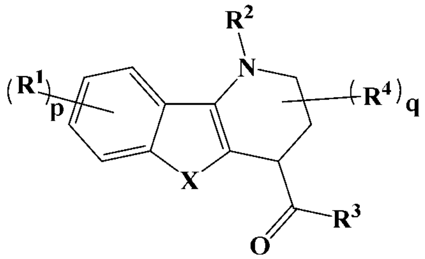

I-2

where,

X is selected from $NR^a$, O, and S;
$R^1$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^1$;
$R^2$ is selected from hydrogen, deuterium, halogen, $-S(O)_2-R^d$, $-C(O)-R^e$, $-NR^iR^j$, substituted or unsubstituted alkyl, alkoxy, haloalkyl, cycloalkyl, aryl, heteroaryl, and heterocyclyl, where the substituted means substituted with one or more $Q^2$;
$R^3$ is selected from hydrogen, deuterium, hydroxyl, $-NR^iR^j$, substituted or unsubstituted alkyl, alkoxy, haloalkyl, cycloalkyl, aryl, heteroaryl, and heterocyclyl, where the substituted means substituted with one or more $Q^3$;
$R^4$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^4$;
$R^a$ is selected from hydrogen, deuterium, alkyl, and haloalkyl;
each $R^b$ is respectively and independently selected from hydrogen, deuterium, substituted or unsubstituted alkyl,

alkoxy, haloalkyl, cycloalkyl, aryl, heteroaryl, and heterocyclyl, where the substituted means substituted with one or more $Q^5$;

each $R^c$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^6$;

each of $R^i$ and $R^j$ is respectively and independently selected from hydrogen, deuterium, $-S(O)_2-R^d$, substituted or unsubstituted alkyl, alkoxy, haloalkyl, cycloalkyl, aryl, heteroaryl, and heterocyclyl, where the substituted means substituted with one or more $Q^7$;

each $R^d$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^8$;

each $R^e$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^9$;

each of $Q^1$, $Q^2$, $Q^3$, $Q^4$, $Q^5$, $Q^6$, $Q^7$, $Q^8$, and $Q^9$ is independently selected from hydrogen, deuterium, halogen, cyano, hydroxyl, mercapto, amino, substituted or unsubstituted alkyl, alkoxy, haloalkyl, cycloalkyl, aryl, heteroaryl, and heterocyclyl, where the substituted means substituted with one or more $Q^{10}$;

$Q^{10}$ is selected from hydrogen, deuterium, halogen, alkyl, alkoxy, haloalkyl, cycloalkyl, aryl, heteroaryl, and heterocyclyl; and

p and q are each independently 0, 1, 2, 3, or 4.

In certain embodiments, X is selected from $NR^a$ and O.

In certain embodiments, X is selected from $NR^a$.

In certain embodiments, $R^a$ is selected from hydrogen, deuterium, and alkyl.

In certain embodiments, $R^a$ is selected from hydrogen and $C_{1-3}$ alkyl.

In certain embodiments, $R^a$ is selected from hydrogen and methyl.

In certain embodiments, $R^1$ is selected from hydrogen, deuterium, halogen, $-OR^b$, $-NR^iR^j$, substituted or unsubstituted alkyl, cycloalkyl, aryl, heteroaryl, and heterocyclyl.

In certain embodiments, $R^1$ is selected from hydrogen, deuterium, halogen, $-OR^b$, $-NR^iR^j$, substituted or unsubstituted $C_{1-6}$ alkyl, $C_{3-7}$ cycloalkyl, $C_{6-10}$ aryl, 5-10 membered heteroaryl, and 3-10 membered heterocyclyl.

In certain embodiments, $R^1$ is selected from hydrogen, deuterium, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl.

In certain embodiments, $R^1$ is selected from hydrogen, fluorine, chlorine, $-OR^b$, $-NR^iR^j$, substituted or unsubstituted methyl, and cyclopropyl.

In certain embodiments, $Q^1$ is selected from hydrogen, halogen, cyano, hydroxyl, amino, and substituted or unsubstituted alkyl.

In certain embodiments, $Q^1$ is selected from hydrogen, halogen, and substituted or unsubstituted $C_{1-3}$ alkyl.

In certain embodiments, $Q^1$ is selected from hydrogen and halogen.

In certain embodiments, $Q^1$ is selected from hydrogen.

In certain embodiments, $R^b$ is selected from hydrogen, substituted or unsubstituted alkyl, and cycloalkyl.

In certain embodiments, $R^b$ is selected from substituted or unsubstituted $C_{1-6}$ alkyl and $C_{3-7}$ cycloalkyl.

In certain embodiments, $R^b$ is selected from substituted or unsubstituted $C_{1-3}$ alkyl and $C_{3-6}$ cycloalkyl.

In certain embodiments, $R^b$ is selected from substituted or unsubstituted methyl, cyclopropyl, and cyclohexyl.

In certain embodiments, $Q^5$ is selected from hydrogen, halogen, cyano, hydroxyl, amino, and substituted or unsubstituted heteroaryl.

In certain embodiments, $Q^5$ is selected from hydrogen, halogen, and substituted or unsubstituted 5-10 membered heteroaryl.

In certain embodiments, $Q^5$ is selected from hydrogen, halogen, and substituted or unsubstituted 5-6 membered heteroaryl, where the heteroaryl contains 1-4 heteroatoms selected from N, O, and S.

In certain embodiments, $Q^5$ is selected from hydrogen and substituted or unsubstituted pyridyl.

In certain embodiments, $R^c$ is selected from hydrogen, substituted or unsubstituted alkyl, and cycloalkyl.

In certain embodiments, $R^c$ is selected from substituted or unsubstituted $C_{1-6}$ alkyl and $C_{3-7}$ cycloalkyl.

In certain embodiments, $R^c$ is selected from substituted or unsubstituted $C_{1-3}$ alkyl and $C_{3-6}$ cycloalkyl.

In certain embodiments, $R^c$ is selected from substituted or unsubstituted methyl, cyclopropyl, and cyclohexyl.

In certain embodiments, $Q^6$ is selected from hydrogen, halogen, cyano, hydroxyl, amino, and substituted or unsubstituted heteroaryl.

In certain embodiments, $Q^6$ is selected from hydrogen, halogen, and substituted or unsubstituted 5-10

membered heteroaryl.

In certain embodiments, $Q^6$ is selected from hydrogen, halogen, and substituted or unsubstituted 5-6 membered heteroaryl, where the heteroaryl contains 1-4 heteroatoms selected from N, O, and S.

In certain embodiments, $Q^6$ is selected from hydrogen and substituted or unsubstituted pyridyl.

In certain embodiments, $R^i$ is selected from hydrogen, $-S(O)_2-R^d$, substituted or unsubstituted alkyl, cycloalkyl, aryl, and heteroaryl.

In certain embodiments, $R^i$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^i$ is selected from hydrogen, $-S(O)_2-R^d$, substituted or unsubstituted methyl, ethyl, cyclopentyl, phenyl, pyridyl, furanyl, and imidazolyl.

In certain embodiments, $R^j$ is selected from hydrogen, $-S(O)_2-R^d$, substituted or unsubstituted alkyl, cycloalkyl, aryl, and heteroaryl.

In certain embodiments, $R^j$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^j$ is selected from hydrogen, $-S(O)_2-R^d$, substituted or unsubstituted methyl, ethyl, cyclopentyl, phenyl, pyridyl, furanyl, and imidazolyl.

In certain embodiments, $Q^7$ is selected from hydrogen, halogen, cyano, hydroxyl, amino, and substituted or unsubstituted heteroaryl.

In certain embodiments, $Q^7$ is selected from hydrogen, halogen, and substituted or unsubstituted 5-10 membered heteroaryl.

In certain embodiments, $Q^7$ is selected from hydrogen, halogen, and substituted or unsubstituted 5-6 membered heteroaryl, where the heteroaryl contains 1-4 heteroatoms selected from N, O, and S.

In certain embodiments, $Q^7$ is selected from hydrogen and pyridyl.

In certain embodiments, $R^d$ is selected from hydrogen, substituted or unsubstituted alkyl, cycloalkyl, aryl, and heteroaryl.

In certain embodiments, $R^d$ is selected from hydrogen, substituted or unsubstituted $C_{1-6}$ alkyl, $C_{3-7}$ cycloalkyl, $C_{6-10}$ aryl, and 5-10 membered heteroaryl.

In certain embodiments, $R^d$ is selected from hydrogen, substituted or unsubstituted $C_{1-3}$ alkyl, $C_{3-6}$ cycloalkyl, and $C_{6-10}$ aryl.

In certain embodiments, $R^d$ is selected from methyl, cyclopropyl, and phenyl.

In certain embodiments, $Q^8$ is selected from hydrogen, halogen, cyano, hydroxyl, mercapto, and amino.

In certain embodiments, $Q^8$ is selected from hydrogen, halogen, and amino.

In certain embodiments, $Q^8$ is selected from hydrogen, fluorine, and chlorine.

In certain embodiments, $Q^8$ is selected from hydrogen.

In certain embodiments, $R^2$ is selected from hydrogen, halogen, $-S(O)_2-R^d$, $-C(O)-R^e$, $-NR^iR^j$, substituted or unsubstituted alkyl, cycloalkyl, aryl, and heteroaryl.

In certain embodiments, $R^2$ is selected from hydrogen, halogen, $-S(O)_2-R^d$, $-C(O)-R^e$, substituted or unsubstituted $C_{1-6}$ alkyl, and $C_{3-7}$ cycloalkyl.

In certain embodiments, $R^2$ is selected from hydrogen, halogen, $-S(O)_2-R^d$, $-C(O)-R^e$, $C_{1-3}$ alkyl, and $C_{3-6}$ cycloalkyl.

In certain embodiments, $R^2$ is selected from hydrogen, fluorine, $-S(O)_2-R^d$, $-C(O)-R^e$, methyl, isopropyl, and cyclopropyl.

In certain embodiments, $Q^2$ is selected from hydrogen, halogen, cyano, hydroxyl, mercapto, and amino.

In certain embodiments, $Q^2$ is selected from hydrogen, halogen, and amino.

In certain embodiments, $Q^2$ is selected from hydrogen, fluorine, and chlorine.

In certain embodiments, $Q^2$ is selected from hydrogen.

In certain embodiments, $R^e$ is selected from hydrogen, substituted or unsubstituted alkyl, aryl, and heterocyclyl.

In certain embodiments, $R^e$ is selected from hydrogen, substituted or unsubstituted $C_{1-6}$ alkyl, $C_{6-10}$ aryl, and 3-10 membered heterocyclyl.

In certain embodiments, $R^e$ is selected from hydrogen, substituted or unsubstituted $C_{1-3}$ alkyl, $C_{6-10}$ aryl, and 3-6 membered heterocyclyl.

In certain embodiments, $R^e$ is selected from hydrogen, substituted or unsubstituted methyl, ethyl, phenyl, and

piperidinyl.

In certain embodiments, $Q^9$ is selected from hydrogen, halogen, substituted or unsubstituted alkyl, aryl, and heteroaryl.

In certain embodiments, $Q^9$ is selected from hydrogen, halogen, substituted or unsubstituted $C_{1-6}$ alkyl, $C_{6-10}$ aryl, and 5-10 membered heteroaryl.

In certain embodiments, $Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S.

In certain embodiments, $Q^9$ is selected from hydrogen, fluorine, methyl, and pyrrolyl.

In certain embodiments, $Q^{10}$ is selected from hydrogen, halogen, and substituted or unsubstituted alkyl.

In certain embodiments, $Q^{10}$ is selected from hydrogen and halogen.

In certain embodiments, $Q^{10}$ is selected from hydrogen and fluorine.

In certain embodiments, $R^3$ is selected from hydrogen, hydroxyl, $-NR^iR^j$, substituted or unsubstituted alkyl, and alkoxy.

In certain embodiments, $R^3$ is selected from hydrogen, hydroxyl, and $-NR^iR^j$.

In certain embodiments, $R^3$ is selected from $-NR^iR^j$.

In certain embodiments, $Q^3$ is selected from hydrogen, halogen, cyano, hydroxyl, mercapto, and amino.

In certain embodiments, $Q^3$ is selected from hydrogen, halogen, and amino.

In certain embodiments, $Q^3$ is selected from hydrogen, fluorine, and chlorine.

In certain embodiments, $Q^3$ is selected from hydrogen.

In certain embodiments, $R^4$ is selected from hydrogen, halogen, $-OR^b$, and $-NR^iR^j$.

In certain embodiments, $R^4$ is selected from hydrogen and halogen.

In certain embodiments, $R^4$ is selected from hydrogen, fluorine, chlorine, and bromine.

In certain embodiments, $R^4$ is selected from hydrogen and fluorine.

In certain embodiments, $Q^4$ is selected from hydrogen, halogen, cyano, hydroxyl, amino, and substituted or unsubstituted alkyl.

In certain embodiments, $Q^4$ is selected from hydrogen, halogen, and substituted or unsubstituted $C_{1-3}$ alkyl.

In certain embodiments, $Q^4$ is selected from hydrogen and halogen.

In certain embodiments, $Q^4$ is selected from hydrogen.

In certain embodiments, p is 1, 2, or 3.

In certain embodiments, q is 0, 1, or 2.

In certain embodiments, X is selected from $NR^a$;

$R^a$ is selected from hydrogen and $C_{1-3}$ alkyl;

$R^1$ is selected from hydrogen, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl, where the substituted means substituted with one or more $Q^1$;

$Q^1$ is selected from hydrogen and halogen;

$R^b$ is selected from substituted or unsubstituted $C_{1-3}$ alkyl and $C_{3-6}$ cycloalkyl, where the substituted means substituted with one or more $Q^5$;

$Q^5$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$Q^7$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^d$ is selected from hydrogen, substituted or unsubstituted $C_{1-3}$ alkyl, $C_{3-5}$ cycloalkyl, and $C_{6-10}$ aryl, where the substituted means substituted with one or more $Q^8$;

$Q^8$ is selected from hydrogen, fluorine, and chlorine;

$R^2$ is selected from hydrogen, halogen, $-S(O)_2-R^d$, $-C(O)-R^e$, $C_{1-3}$ alkyl, and $C_{3-6}$ cycloalkyl;

$R^e$ is selected from hydrogen, substituted or unsubstituted $C_{1-3}$ alkyl, $C_{6-10}$ aryl, and 3-6 membered heterocyclyl, where the substituted means substituted with one or more $Q^9$;

$Q^9$ is selected from hydrogen, halogen, substituted or unsubstituted $C_{1-3}$ alkyl, and 5-6 membered heteroaryl, where

the heteroaryl contains 1-4 heteroatoms selected from N, O, and S, and the substituted means substituted with one or more $Q^{10}$;
$Q^{10}$ is selected from hydrogen and halogen;
$R^3$ is selected from $-NR^iR^j$;
$R^4$ is selected from hydrogen, fluorine, chlorine, and bromine;
p is 1, 2, or 3; and
q is 0, 1, or 2.

In certain embodiments, X is selected from $NR^a$;

$R^a$ is selected from hydrogen and methyl;
$R^1$ is selected from hydrogen, fluorine, chlorine, $-OR^b$, $-NR^iR^j$, methyl, and cyclopropyl;
$R^b$ is selected from substituted or unsubstituted methyl, cyclopropyl, and cyclohexyl, where the substituted means substituted with one or more $Q^5$;
$Q^5$ is selected from hydrogen and substituted or unsubstituted pyridyl, where the substituted means substituted with one or more $Q^{10}$;
$R^i$ is selected from hydrogen, $-S(O)_2-R^d$, substituted or unsubstituted methyl, ethyl, cyclopentyl, phenyl, pyridyl, furanyl, and imidazolyl, where the substituted means substituted with one or more $Q^7$;
$R^j$ is selected from hydrogen, $-S(O)_2-R^d$, substituted or unsubstituted methyl, ethyl, cyclopentyl, phenyl, pyridyl, furanyl, and imidazolyl, where the substituted means substituted with one or more $Q^7$;
$Q^7$ is selected from hydrogen and pyridyl;
$R^d$ is selected from methyl, cyclopropyl, and phenyl;
$R^2$ is selected from hydrogen, fluorine, $-S(O)_2-R^d$, $-C(O)-R^e$, methyl, isopropyl, and cyclopropyl;
$R^e$ is selected from hydrogen, substituted or unsubstituted methyl, ethyl, phenyl, and piperidinyl, where the substituted means substituted with one or more $Q^9$;
$Q^9$ is selected from hydrogen, fluorine, methyl, and pyrrolyl;
$Q^{10}$ is selected from hydrogen and fluorine;
$R^3$ is selected from $-NR^iR^j$;
$R^4$ is selected from hydrogen and fluorine;
p is 1, 2, or 3; and
q is 0, 1, or 2.

In another aspect, the present disclosure provides a compound of formula I-3, or a stereoisomer, a tautomer, a solvate, a hydrate, a prodrug, a stable isotope derivative, and a pharmaceutically acceptable salt thereof:

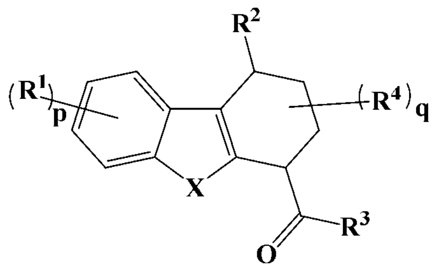

I-3

where,

X is selected from $NR^a$, O, and S;
$R^1$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, where the substituted means substituted with one or more $Q^1$;
$R^2$ is selected from hydrogen, deuterium, halogen, $-S(O)_2-R^d$, $-C(O)-R^e$, $-NR^iR^j$, substituted or unsubstituted alkyl, alkoxy, haloalkyl, cycloalkyl, aryl, heteroaryl, and heterocyclyl, where the substituted means substituted with one or more $Q^2$;
$R^3$ is selected from hydrogen, deuterium, hydroxyl, $-NR^iR^j$, substituted or unsubstituted alkyl, alkoxy, haloalkyl, cycloalkyl, aryl, heteroaryl, and heterocyclyl, where the substituted means substituted with one or more $Q^3$;
$R^4$ is selected from hydrogen, deuterium, halogen, hydroxyl, cyano, $-OR^b$, $-SR^c$, $-NR^iR^j$, substituted or unsubstituted

alkyl, alkoxy, haloalkyl, cycloalkyl, aryl, heteroaryl, and heterocyclyl, where the substituted means substituted with one or more $Q^4$;

$R^a$ is selected from hydrogen, deuterium, alkyl, and haloalkyl;

each $R^b$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^5$;

each $R^c$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^6$;

each of $R^i$ and $R^j$ is respectively and independently selected from hydrogen, deuterium, $-S(O)_2-R^d$, substituted or unsubstituted alkyl, alkoxy, haloalkyl, cycloalkyl, aryl, heteroaryl, and heterocyclyl, where the substituted means substituted with one or more $Q^7$;

each $R^d$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^8$;

each $R^e$ is respectively and independently selected from hydrogen, deuterium, substituted or unsubstituted alkyl, alkoxy, haloalkyl, cycloalkyl, aryl, heteroaryl, and heterocyclyl, where the substituted means substituted with one or more $Q^9$;

each of $Q^1$, $Q^2$, $Q^3$, $Q^4$, $Q^5$, $Q^6$, $Q^7$, $Q^8$, and $Q^9$ is independently selected from hydrogen, deuterium, halogen, cyano, hydroxyl, mercapto, amino, substituted or unsubstituted alkyl, alkoxy, haloalkyl, cycloalkyl, aryl, heteroaryl, and heterocyclyl, where the substituted means substituted with one or more $Q^{10}$;

$Q^{10}$ is selected from hydrogen, deuterium, halogen, alkyl, alkoxy, haloalkyl, cycloalkyl, aryl, heteroaryl, and heterocyclyl; and

p and q are each independently 0, 1, 2, 3, or 4.

In certain embodiments, X is selected from $NR^a$ and O.

In certain embodiments, X is selected from $NR^a$.

In certain embodiments, $R^a$ is selected from hydrogen, deuterium, and alkyl.

In certain embodiments, $R^a$ is selected from hydrogen and $C_{1-3}$ alkyl.

In certain embodiments, $R^a$ is selected from hydrogen and methyl.

In certain embodiments, $R^1$ is selected from hydrogen, deuterium, halogen, $-OR^b$, $-NR^iR^j$, substituted or unsubstituted alkyl, cycloalkyl, aryl, heteroaryl, and heterocyclyl.

In certain embodiments, $R^1$ is selected from hydrogen, deuterium, halogen, $-OR^b$, $-NR^iR^j$, substituted or unsubstituted $C_{1-6}$ alkyl, $C_{3-7}$ cycloalkyl, $C_{6-10}$ aryl, 5-10 membered heteroaryl, and 3-10 membered heterocyclyl.

In certain embodiments, $R^1$ is selected from hydrogen, deuterium, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl.

In certain embodiments, $R^1$ is selected from hydrogen, fluorine, chlorine, $-OR^b$, $-NR^iR^j$, substituted or unsubstituted methyl, and cyclopropyl.

In certain embodiments, $Q^1$ is selected from hydrogen, halogen, cyano, hydroxyl, amino, and substituted or unsubstituted alkyl.

In certain embodiments, $Q^1$ is selected from hydrogen, halogen, and substituted or unsubstituted $C_{1-3}$ alkyl.

In certain embodiments, $Q^1$ is selected from hydrogen and halogen.

In certain embodiments, $Q^1$ is selected from hydrogen.

In certain embodiments, $R^b$ is selected from hydrogen, substituted or unsubstituted alkyl, and cycloalkyl.

In certain embodiments, $R^b$ is selected from substituted or unsubstituted $C_{1-6}$ alkyl and $C_{3-7}$ cycloalkyl.

In certain embodiments, $R^b$ is selected from substituted or unsubstituted $C_{1-3}$ alkyl and $C_{3-6}$ cycloalkyl.

In certain embodiments, $R^b$ is selected from substituted or unsubstituted methyl, cyclopropyl, and cyclohexyl.

In certain embodiments, $Q^5$ is selected from hydrogen, halogen, cyano, hydroxyl, amino, and substituted or unsubstituted heteroaryl.

In certain embodiments, $Q^5$ is selected from hydrogen, halogen, and substituted or unsubstituted 5-10 membered heteroaryl.

In certain embodiments, $Q^5$ is selected from hydrogen, halogen, and substituted or unsubstituted 5-6 membered heteroaryl, where the heteroaryl contains 1-4 heteroatoms selected from N, O, and S.

In certain embodiments, $Q^5$ is selected from hydrogen and substituted or unsubstituted pyridyl.

In certain embodiments, $R^c$ is selected from hydrogen, substituted or unsubstituted alkyl, and cycloalkyl.

In certain embodiments, $R^c$ is selected from substituted or unsubstituted $C_{1-6}$ alkyl and $C_{3-7}$ cycloalkyl.

In certain embodiments, $R^c$ is selected from substituted or unsubstituted $C_{1-3}$ alkyl and $C_{3-6}$ cycloalkyl.

In certain embodiments, $R^c$ is selected from substituted or unsubstituted methyl, cyclopropyl, and cyclohexyl.

In certain embodiments, $Q^6$ is selected from hydrogen, halogen, cyano, hydroxyl, amino, and substituted or unsubstituted heteroaryl.

In certain embodiments, $Q^6$ is selected from hydrogen, halogen, and substituted or unsubstituted 5-10 membered heteroaryl.

In certain embodiments, $Q^6$ is selected from hydrogen, halogen, and substituted or unsubstituted 5-6 membered heteroaryl, where the heteroaryl contains 1-4 heteroatoms selected from N, O, and S.

In certain embodiments, $Q^6$ is selected from hydrogen and substituted or unsubstituted pyridyl.

In certain embodiments, $R^i$ is selected from hydrogen, $-S(O)_2-R^d$, substituted or unsubstituted alkyl, cycloalkyl, aryl, and heteroaryl.

In certain embodiments, $R^i$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^i$ is selected from hydrogen, $-S(O)_2-R^d$, substituted or unsubstituted methyl, ethyl, cyclopentyl, phenyl, pyridyl, furanyl, and imidazolyl.

In certain embodiments, $R^j$ is selected from hydrogen, $-S(O)_2-R^d$, substituted or unsubstituted alkyl, cycloalkyl, aryl, and heteroaryl.

In certain embodiments, $R^j$ is selected from hydrogen, $-S(O)_2-R^d$, substituted or unsubstituted $C_{1-6}$ alkyl, $C_{3-7}$ cycloalkyl, $C_{6-10}$ aryl, and 5-10 membered heteroaryl.

In certain embodiments, $R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S.

In certain embodiments, $R^j$ is selected from hydrogen, $-S(O)_2-R^d$, substituted or unsubstituted methyl, ethyl, cyclopentyl, phenyl, pyridyl, furanyl, and imidazolyl.

In certain embodiments, $Q^7$ is selected from hydrogen, halogen, cyano, hydroxyl, amino, and substituted or unsubstituted heteroaryl.

In certain embodiments, $Q^7$ is selected from hydrogen, halogen, and substituted or unsubstituted 5-10 membered heteroaryl.

In certain embodiments, $Q^7$ is selected from hydrogen, halogen, and substituted or unsubstituted 5-6 membered heteroaryl, where the heteroaryl contains 1-4 heteroatoms selected from N, O, and S.

In certain embodiments, $Q^7$ is selected from hydrogen and pyridyl.

In certain embodiments, $R^d$ is selected from hydrogen, substituted or unsubstituted alkyl, cycloalkyl, aryl, and heteroaryl.

In certain embodiments, $R^d$ is selected from hydrogen, substituted or unsubstituted $C_{1-6}$ alkyl, $C_{3-7}$ cycloalkyl, $C_{6-10}$ aryl, and 5-10 membered heteroaryl.

In certain embodiments, $R^d$ is selected from hydrogen, substituted or unsubstituted $C_{1-3}$ alkyl, $C_{3-6}$ cycloalkyl, and $C_{6-10}$ aryl.

In certain embodiments, $R^d$ is selected from methyl, cyclopropyl, and phenyl.

In certain embodiments, $Q^8$ is selected from hydrogen, halogen, cyano, hydroxyl, mercapto, and amino.

In certain embodiments, $Q^8$ is selected from hydrogen, halogen, and amino.

In certain embodiments, $Q^8$ is selected from hydrogen, fluorine, and chlorine.

In certain embodiments, $Q^8$ is selected from hydrogen.

In certain embodiments, $R^2$ is selected from hydrogen, halogen, $-S(O)_2-R^d$, $-C(O)-R^e$, $-NR^iR^j$, substituted or unsubstituted alkyl, cycloalkyl, aryl, and heteroaryl.

In certain embodiments, $R^2$ is selected from hydrogen, halogen, $-S(O)_2-R^d$, $-C(O)-R^e$, substituted or unsubstituted $C_{1-6}$ alkyl, and $C_{3-7}$ cycloalkyl.

In certain embodiments, $R^2$ is selected from hydrogen, halogen, $-S(O)_2-R^d$, $-C(O)-R^e$, $C_{1-3}$ alkyl, and $C_{3-6}$ cycloalkyl.

In certain embodiments, $R^2$ is selected from hydrogen, fluorine, $-S(O)_2-R^d$, $-C(O)-R^e$, methyl, isopropyl, and cyclopropyl.

In certain embodiments, $Q^2$ is selected from hydrogen, halogen, cyano, hydroxyl, mercapto, and amino.

In certain embodiments, $Q^2$ is selected from hydrogen, halogen, and amino.

In certain embodiments, $Q^2$ is selected from hydrogen, fluorine, and chlorine.

In certain embodiments, $Q^2$ is selected from hydrogen.

In certain embodiments, $R^e$ is selected from hydrogen, substituted or unsubstituted alkyl, aryl, and heterocyclyl.

In certain embodiments, $R^e$ is selected from hydrogen, substituted or unsubstituted $C_{1-6}$ alkyl, $C_{6-10}$ aryl, and

3-10 membered heterocyclyl.

In certain embodiments, $R^e$ is selected from hydrogen, substituted or unsubstituted $C_{1-3}$ alkyl, $C_{6-10}$ aryl, and 3-6 membered heterocyclyl.

In certain embodiments, $R^e$ is selected from hydrogen, substituted or unsubstituted methyl, ethyl, phenyl, and piperidinyl.

In certain embodiments, $Q^9$ is selected from hydrogen, halogen, substituted or unsubstituted alkyl, aryl, and heteroaryl.

In certain embodiments, $Q^9$ is selected from hydrogen, halogen, substituted or unsubstituted $C_{1-6}$ alkyl, $C_{6-10}$ aryl, and 5-10 membered heteroaryl.

In certain embodiments, $Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S.

In certain embodiments, $Q^9$ is selected from hydrogen, fluorine, methyl, and pyrrolyl.

In certain embodiments, $Q^{10}$ is selected from hydrogen, halogen, and substituted or unsubstituted alkyl.

In certain embodiments, $Q^{10}$ is selected from hydrogen and halogen.

In certain embodiments, $Q^{10}$ is selected from hydrogen and fluorine.

In certain embodiments, $R^3$ is selected from hydrogen, hydroxyl, $-NR^iR^j$, substituted or unsubstituted alkyl, and alkoxy.

In certain embodiments, $R^3$ is selected from hydrogen, hydroxyl, and $-NR^iR^j$.

In certain embodiments, $R^3$ is selected from $-NR^iR^j$.

In certain embodiments, $Q^3$ is selected from hydrogen, halogen, cyano, hydroxyl, mercapto, and amino.

In certain embodiments, $Q^3$ is selected from hydrogen, halogen, and amino.

In certain embodiments, $Q^3$ is selected from hydrogen, fluorine, and chlorine.

In certain embodiments, $Q^3$ is selected from hydrogen.

In certain embodiments, $R^4$ is selected from hydrogen, halogen, $-OR^b$, and $-NR^iR^j$.

In certain embodiments, $R^4$ is selected from hydrogen and halogen.

In certain embodiments, $R^4$ is selected from hydrogen, fluorine, chlorine, and bromine.

In certain embodiments, $R^4$ is selected from hydrogen and fluorine.

In certain embodiments, $Q^4$ is selected from hydrogen, halogen, cyano, hydroxyl, amino, and substituted or unsubstituted alkyl.

In certain embodiments, $Q^4$ is selected from hydrogen, halogen, and substituted or unsubstituted $C_{1-3}$ alkyl.

In certain embodiments, $Q^4$ is selected from hydrogen and halogen.

In certain embodiments, $Q^4$ is selected from hydrogen.

In certain embodiments, p is 1, 2, or 3.

In certain embodiments, q is 0, 1, or 2.

In certain embodiments, X is selected from $NR^a$;

$R^a$ is selected from hydrogen and $C_{1-3}$ alkyl;

$R^1$ is selected from hydrogen, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl, where the substituted means substituted with one or more $Q^1$;

$Q^1$ is selected from hydrogen and halogen;

$R^b$ is selected from substituted or unsubstituted $C_{1-3}$ alkyl and $C_{3-6}$ cycloalkyl, where the substituted means substituted with one or more $Q^5$;

$Q^5$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, where the heteroaryl contains 1-4 heteroatoms selected from N, O, and S, and the substituted means substituted with one or more $Q^7$;

$Q^7$ is selected from hydrogen, halogen, and substituted or unsubstituted 5-6 membered heteroaryl, where the heteroaryl contains 1-4 heteroatoms selected from N, O, and S, and the substituted means substituted with one or more $Q^{10}$;

$R^d$ is selected from hydrogen, substituted or unsubstituted $C_{1-3}$ alkyl, $C_{3-5}$ cycloalkyl, and $C_{6-10}$ aryl, where the substituted means substituted with one or more $Q^8$;

$Q^8$ is selected from hydrogen, fluorine, and chlorine;

$R^2$ is selected from hydrogen, halogen, -S(O)$_2$-$R^d$, -C(O)-$R^e$, $C_{1-3}$ alkyl, and $C_{3-6}$ cycloalkyl;
$R^e$ is selected from hydrogen, substituted or unsubstituted $C_{1-3}$ alkyl, $C_{6-10}$ aryl, and 3-6 membered heterocyclyl, where the substituted means substituted with one or more $Q^9$;
$Q^9$ is selected from hydrogen, halogen, substituted or unsubstituted $C_{1-3}$ alkyl, and 5-6 membered heteroaryl, where the heteroaryl contains 1-4 heteroatoms selected from N, O, and S, and the substituted means substituted with one or more $Q^{10}$;
$Q^{10}$ is selected from hydrogen and halogen;
$R^3$ is selected from -$NR^iR^j$;
$R^4$ is selected from hydrogen, fluorine, chlorine, and bromine;
p is 1, 2, or 3; and
q is 0, 1, or 2.

In certain embodiments, X is selected from $NR^a$;

$R^a$ is selected from hydrogen and methyl;
$R^1$ is selected from hydrogen, fluorine, chlorine, -$OR^b$, -$NR^iR^j$, methyl, and cyclopropyl;
$R^b$ is selected from substituted or unsubstituted methyl, cyclopropyl, and cyclohexyl, where the substituted means substituted with one or more $Q^5$;
$Q^5$ is selected from hydrogen and substituted or unsubstituted pyridyl, where the substituted means substituted with one or more $Q^{10}$;
$R^i$ is selected from hydrogen, -S(O)$_2$-$R^d$, substituted or unsubstituted methyl, ethyl, cyclopentyl, phenyl, pyridyl, furanyl, and imidazolyl, where the substituted means substituted with one or more $Q^7$;
$R^j$ is selected from hydrogen, -S(O)$_2$-$R^d$, substituted or unsubstituted methyl, ethyl, cyclopentyl, phenyl, pyridyl, furanyl, and imidazolyl, where the substituted means substituted with one or more $Q^7$;
$Q^7$ is selected from hydrogen and pyridyl;
$R^d$ is selected from methyl, cyclopropyl, and phenyl;
$R^2$ is selected from hydrogen, fluorine, -S(O)$_2$-$R^d$, -C(O)-$R^e$, methyl, isopropyl, and cyclopropyl;
$R^e$ is selected from hydrogen, substituted or unsubstituted methyl, ethyl, phenyl, and piperidinyl, where the substituted means substituted with one or more $Q^9$;
$Q^9$ is selected from hydrogen, fluorine, methyl, and pyrrolyl;
$Q^{10}$ is selected from hydrogen and fluorine;
$R^3$ is selected from -$NR^iR^j$;
$R^4$ is selected from hydrogen and fluorine;
p is 1, 2, or 3; and
q is 0, 1, or 2.

In another aspect, the present disclosure provides a compound, or a stereoisomer, a tautomer, a solvate, a hydrate, a prodrug, a stable isotope derivative, and a pharmaceutically acceptable salt thereof, where the compound is any one of the following:

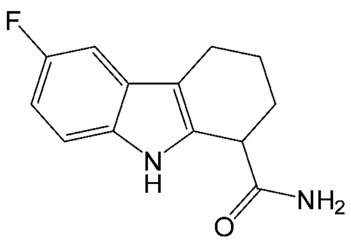 , 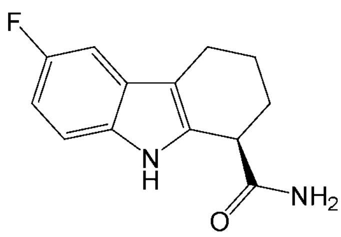 , 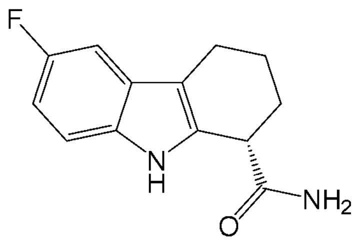 ,

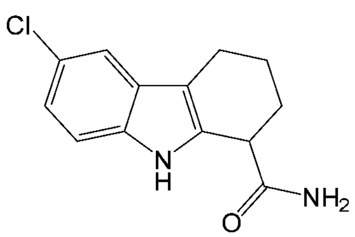 , 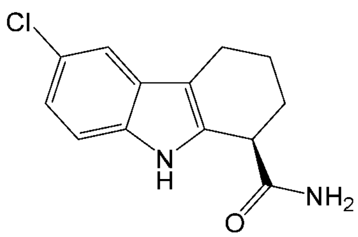 , 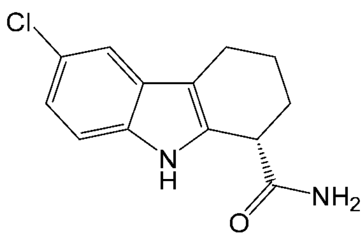 ,

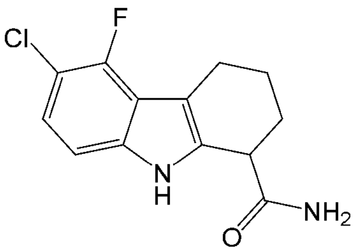
,
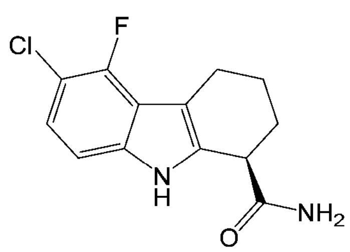
,
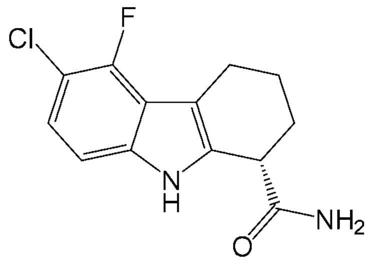
,
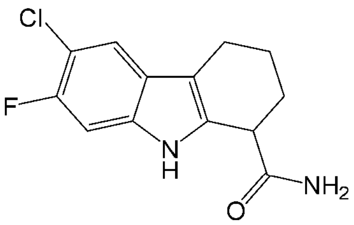
,
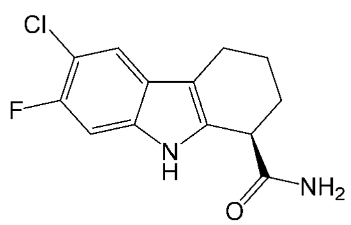
,
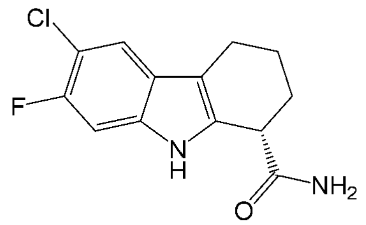
,
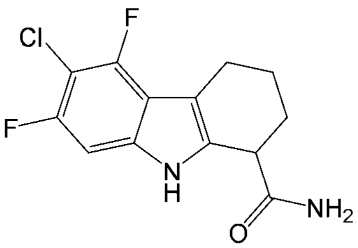
,
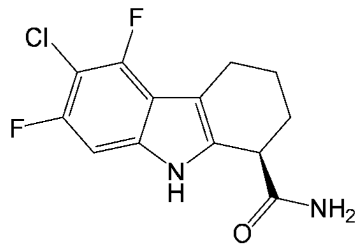
,
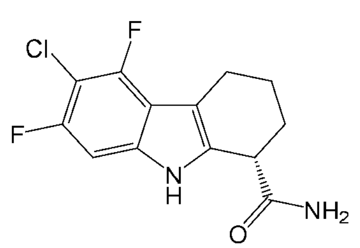
,
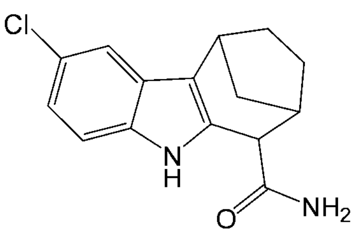
,
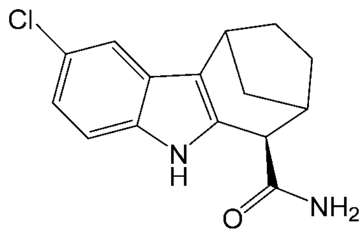
,
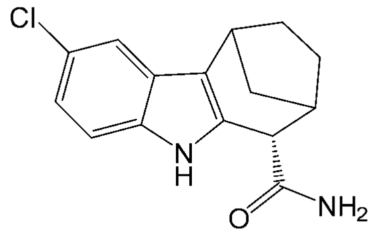
,
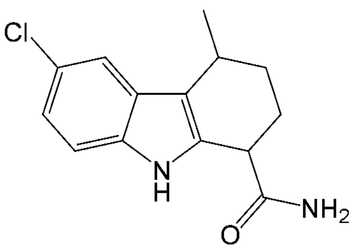
,
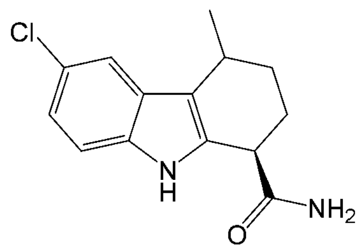
,
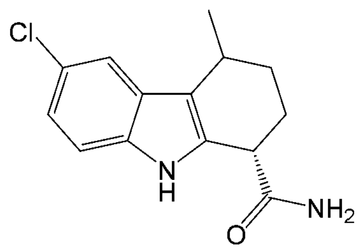
,
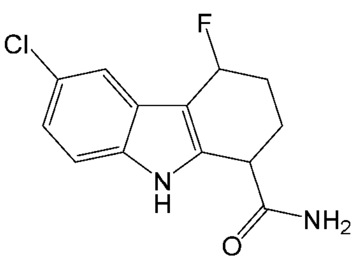
,
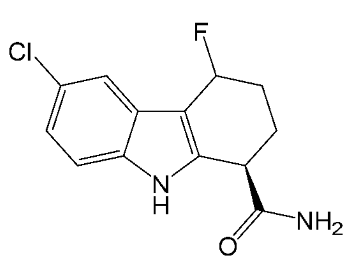
,
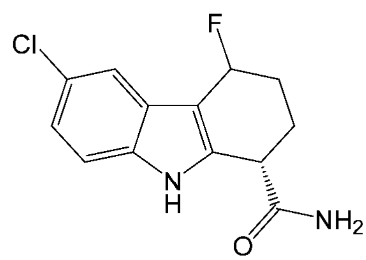
,

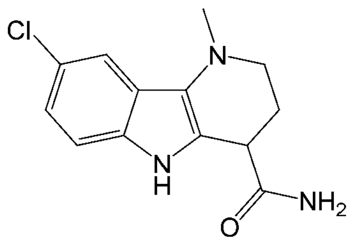 , 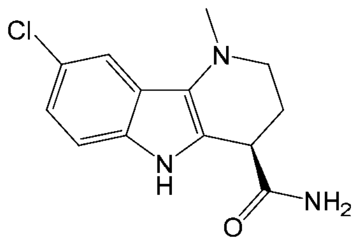 , 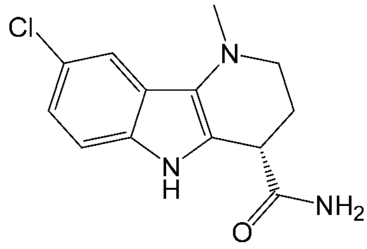 ,

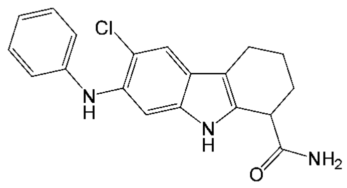 , 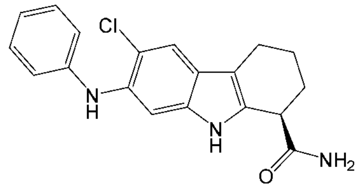 , 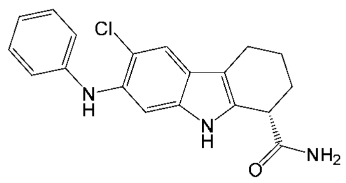 ,

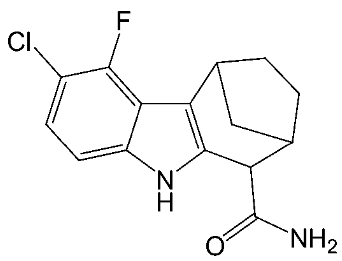 , 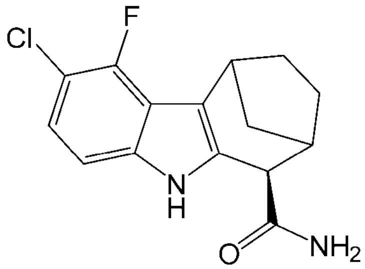 , 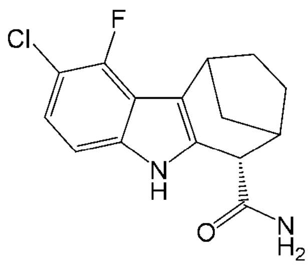 ,

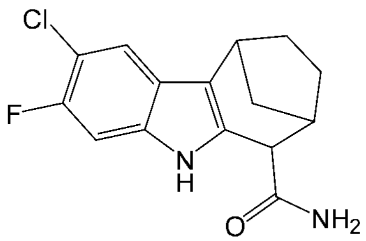 , 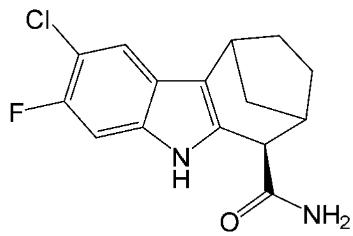 , 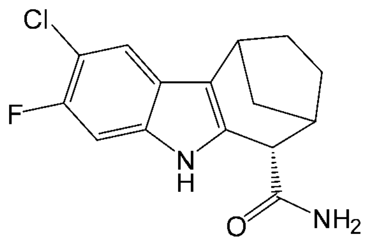 ,

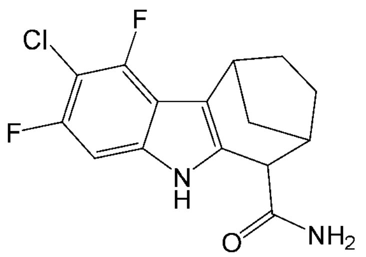 , 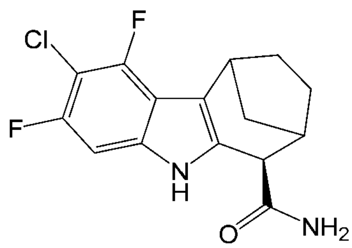 , 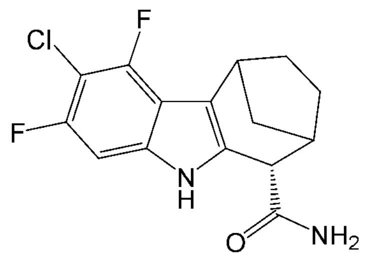 ,

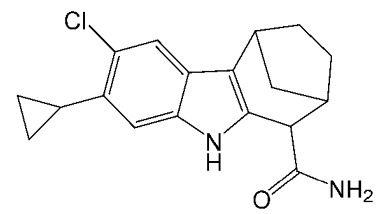 , 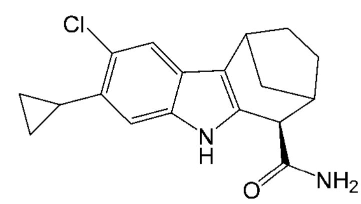 , 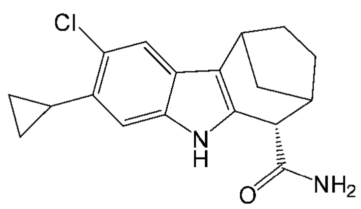 ,

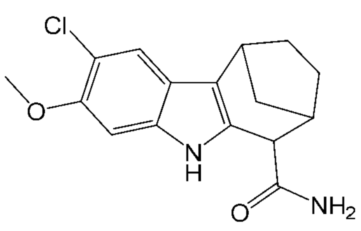 , 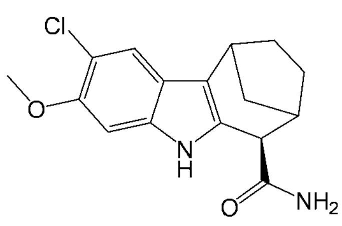 , 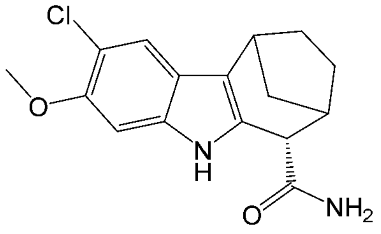 ,

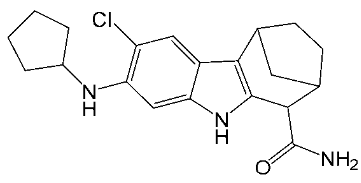 , 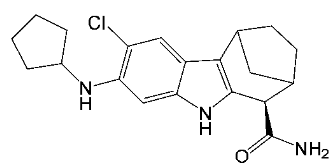 , 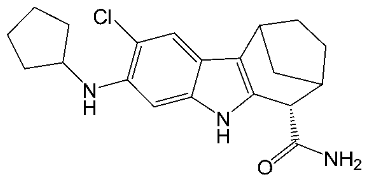 ,

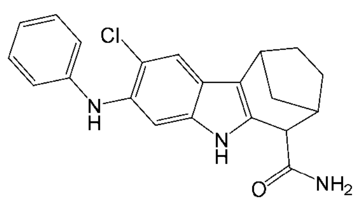 , 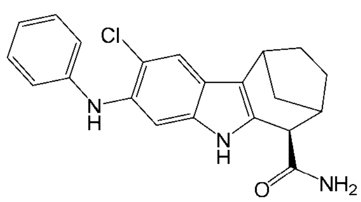 , 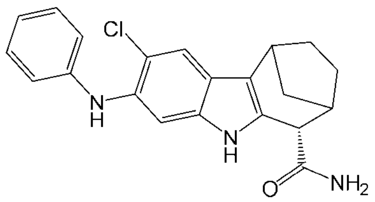 ,

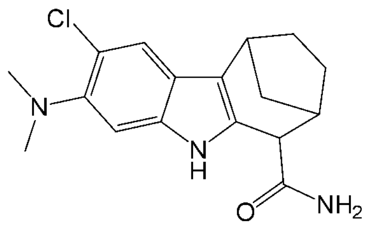 , 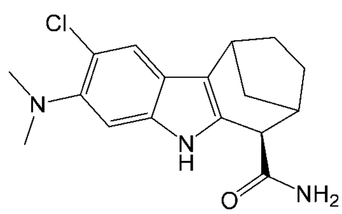 , 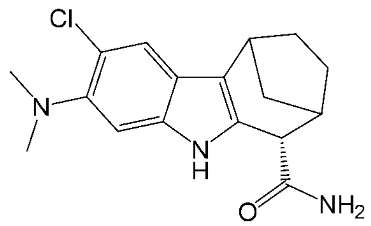 ,

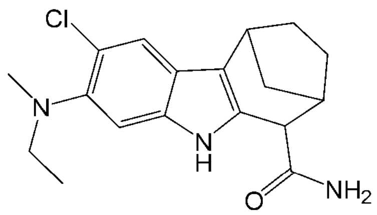 , 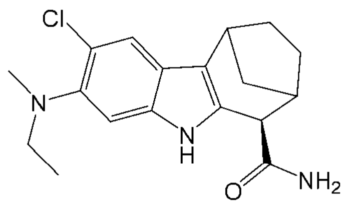 , 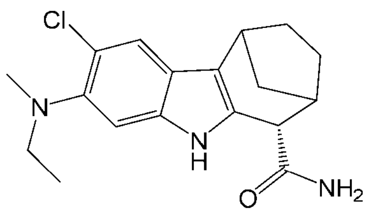 ,

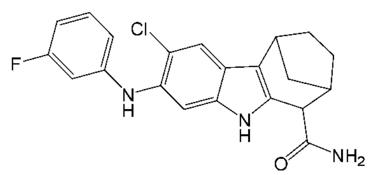 , 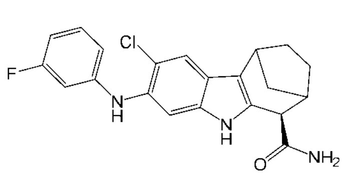 , 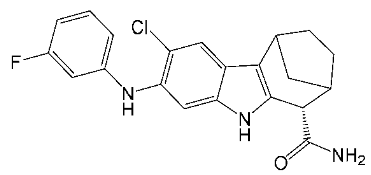 ,

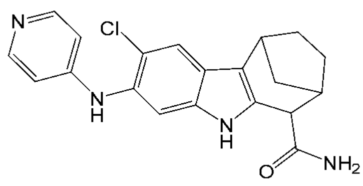 , 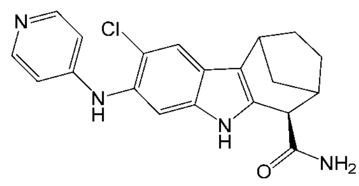 , 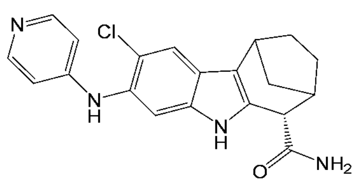 ,

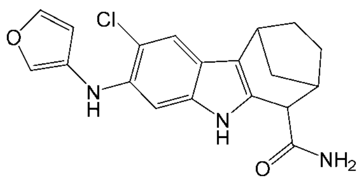 , 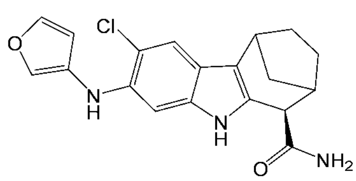 , 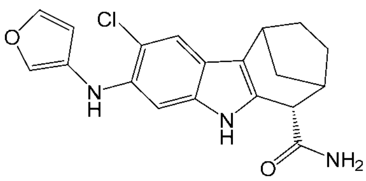 ,

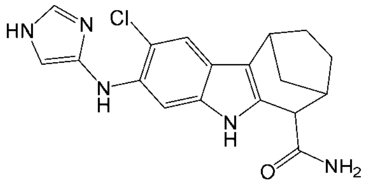 , 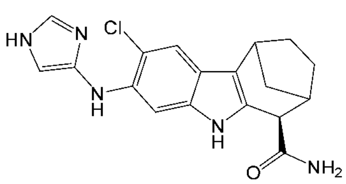 , 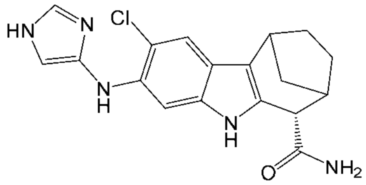 ,

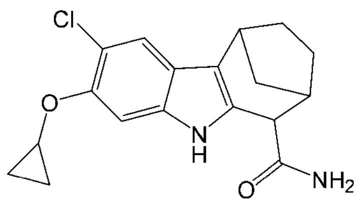 , 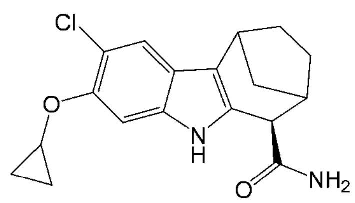 , 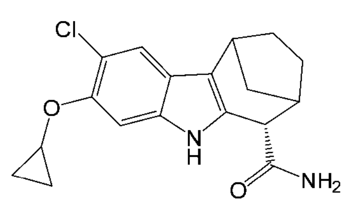 ,

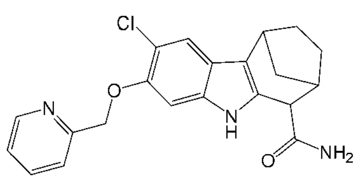 , 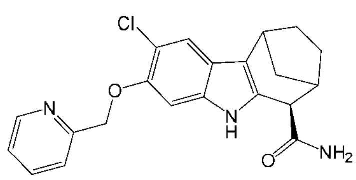 , 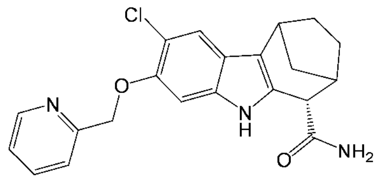 ,

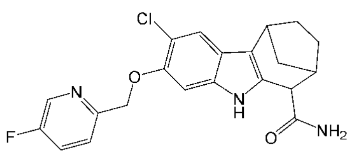 , 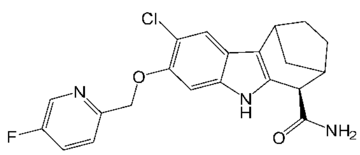 , 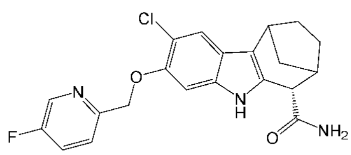 ,

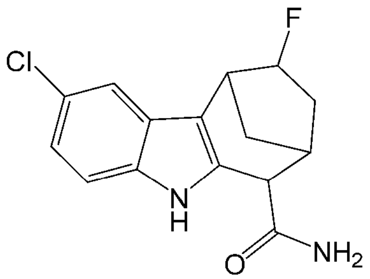 , 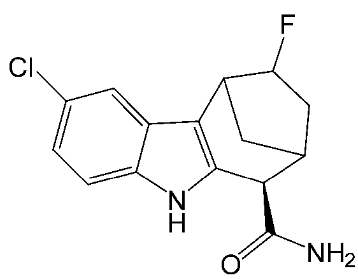 , 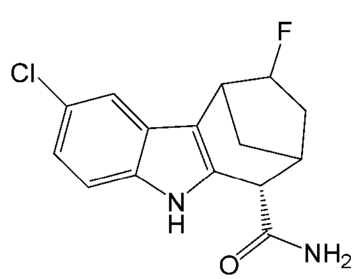 ,

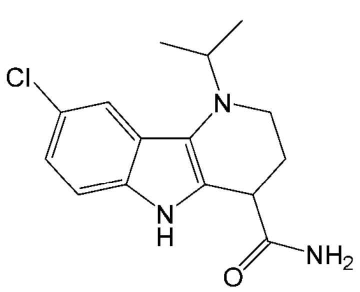 , 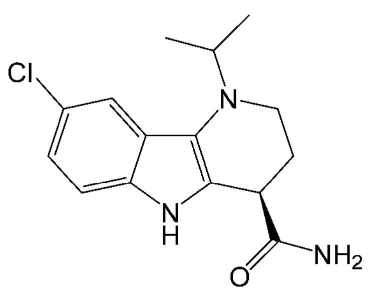 , 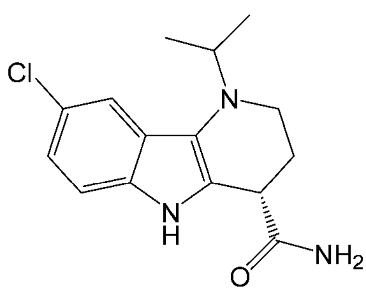 ,

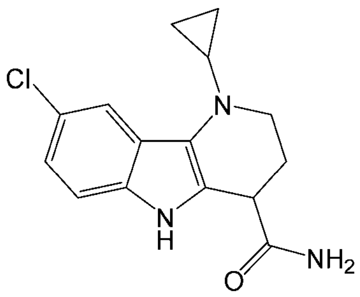 , 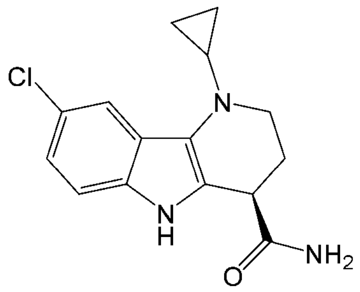 , 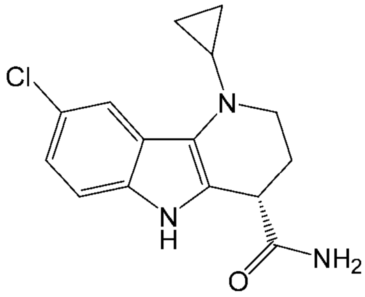 ,

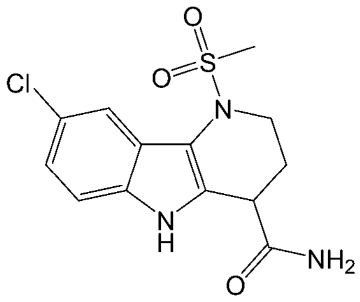 , 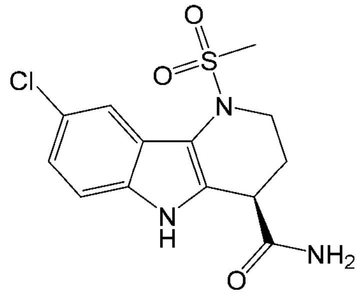 , 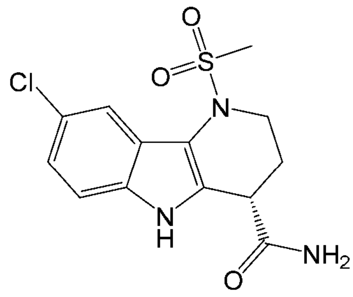 ,

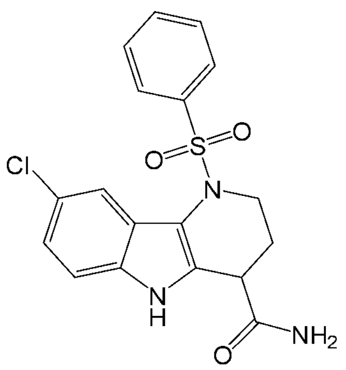 , 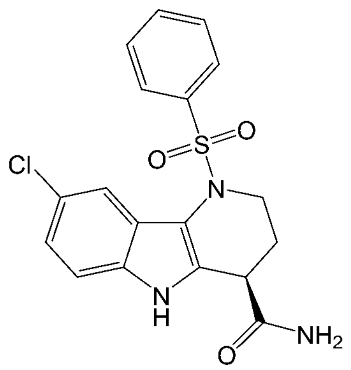 , 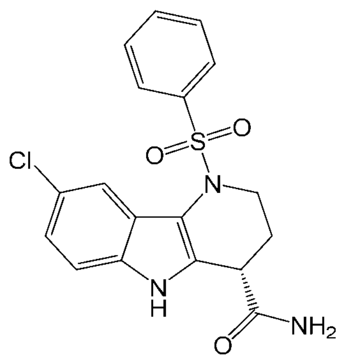 ,

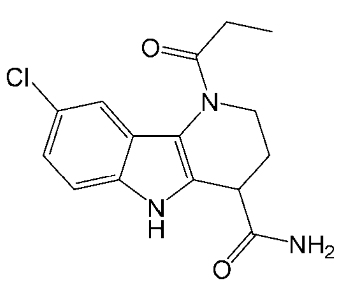 , 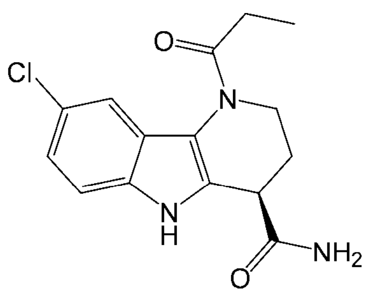 , 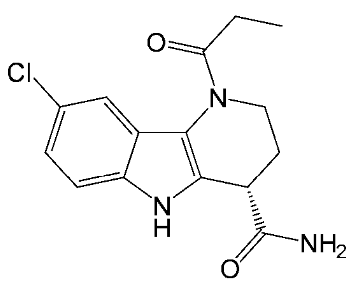 ,

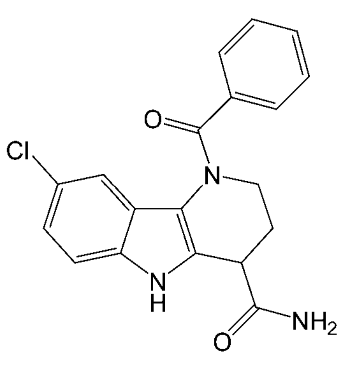 , 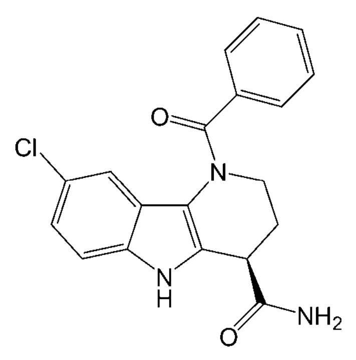 , 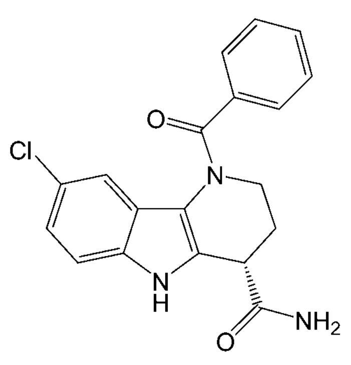 ,

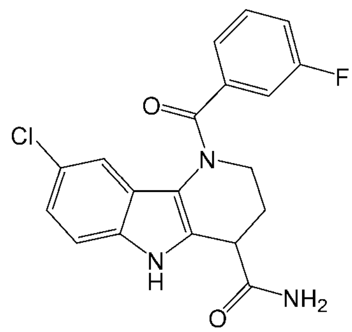
,
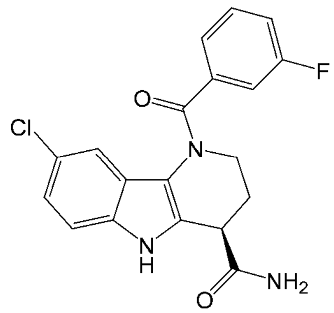
,
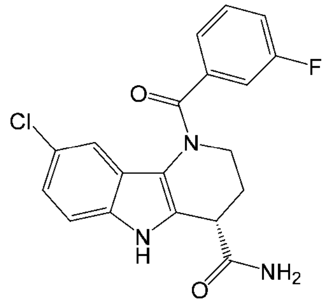
,

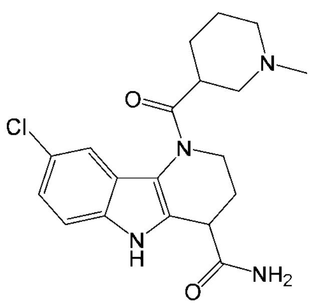
,
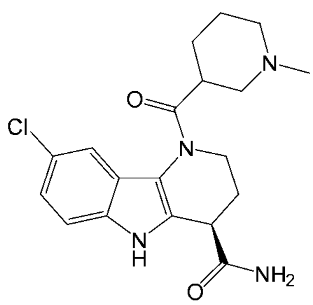
,
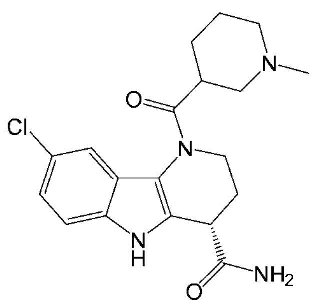
,

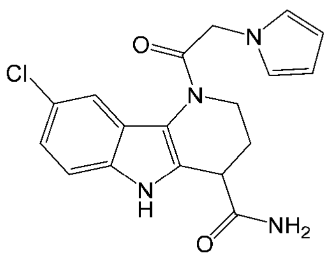
,
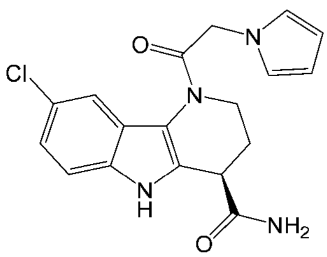
,
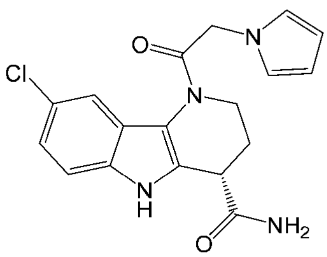
,

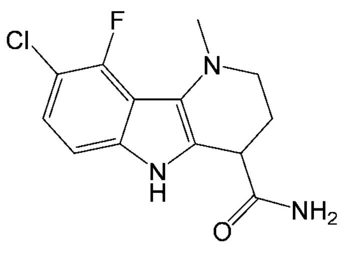
,
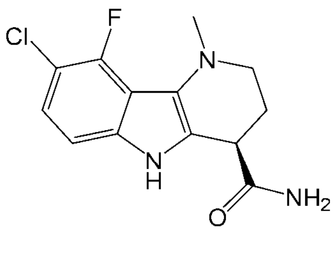
,
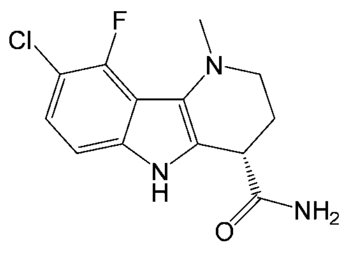
,

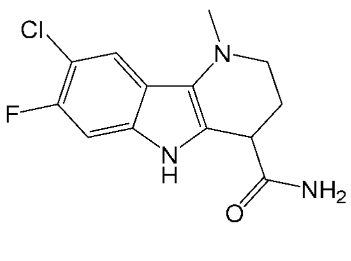
,
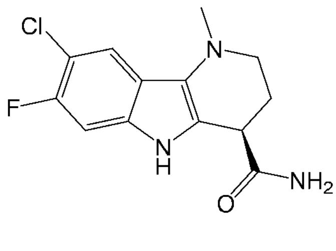
,
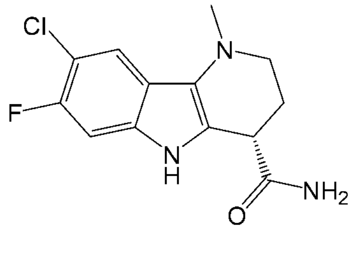
,

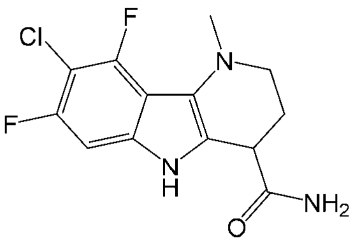 , 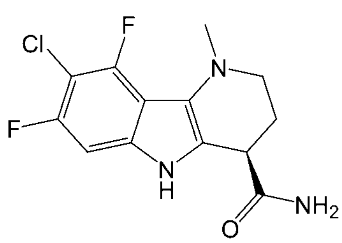 , 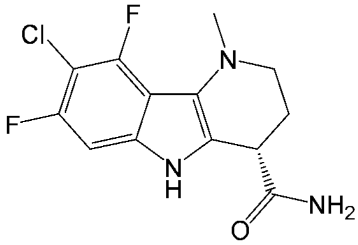 ,

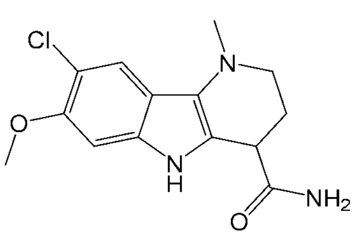 , 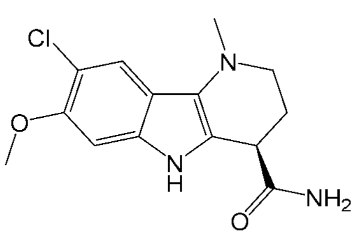 , 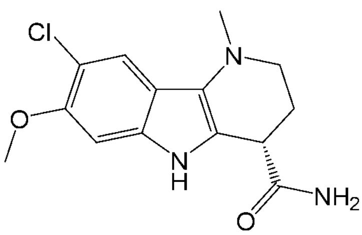 ,

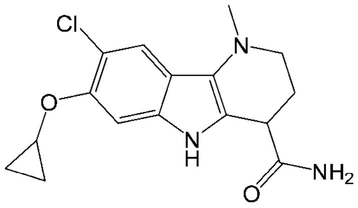 , 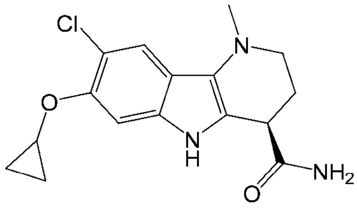 , 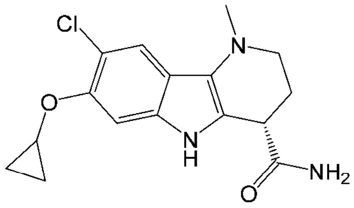 ,

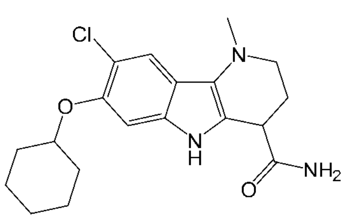 , 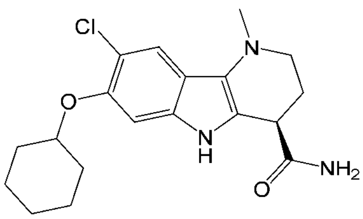 , 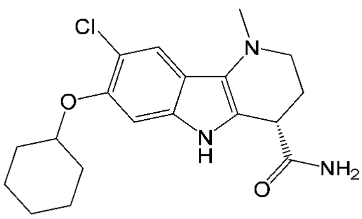 ,

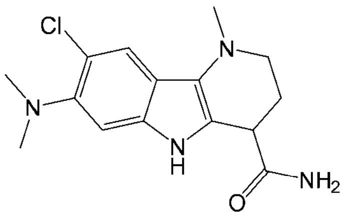 , 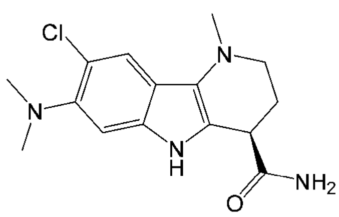 , 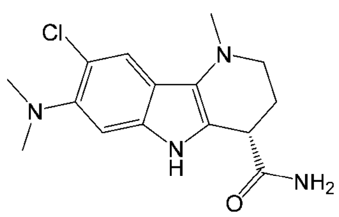 ,

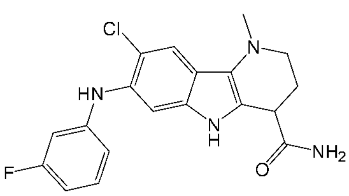 , 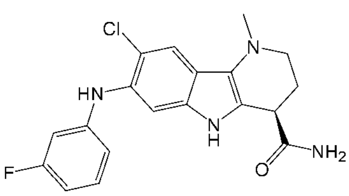 , 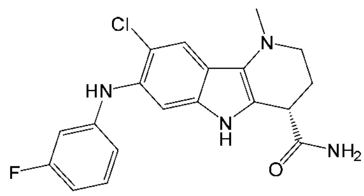 ,

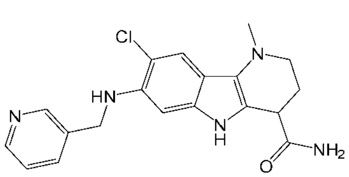 , 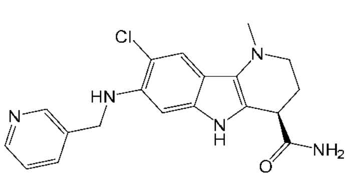 , 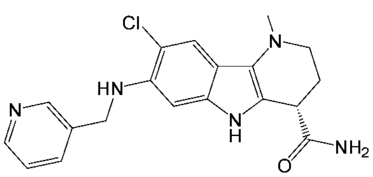 ,

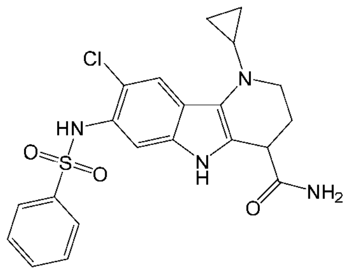 , 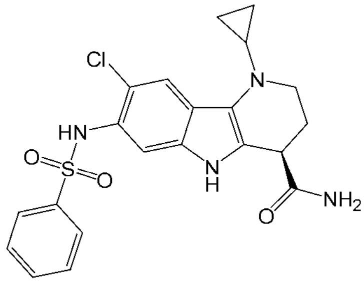 , 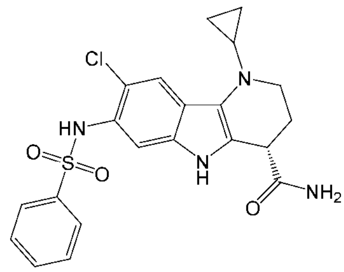 ,

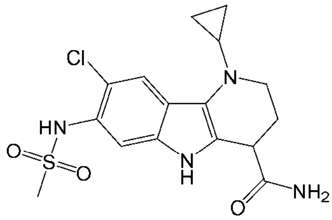 , 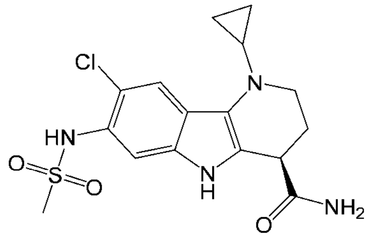 , 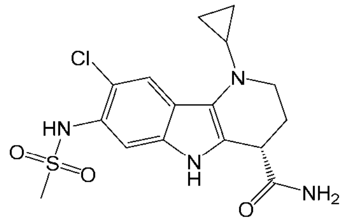 ,

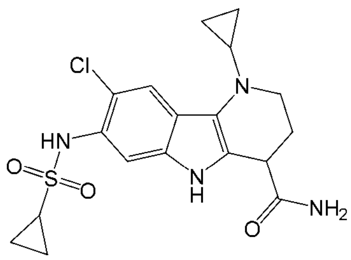 , 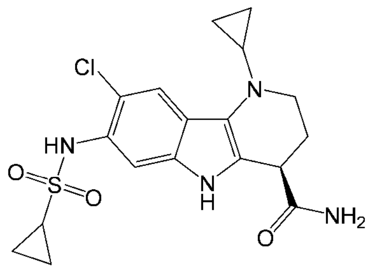 , 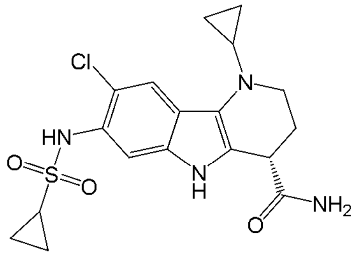 ,

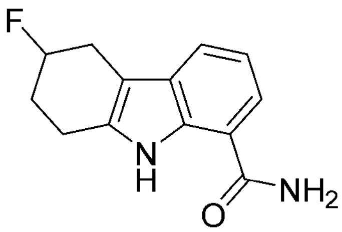 , 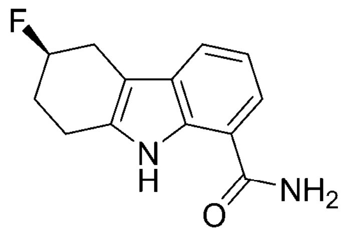 , 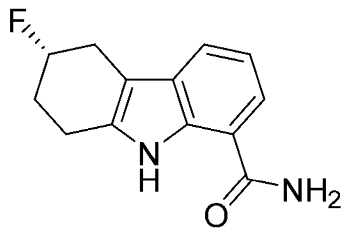 ,

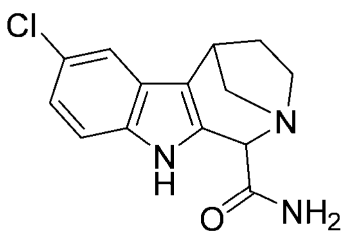 , 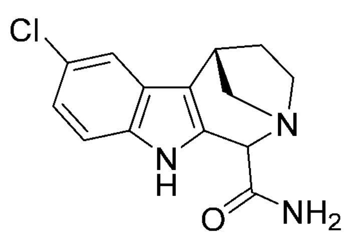 , 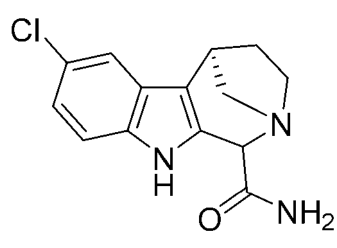 ,

and

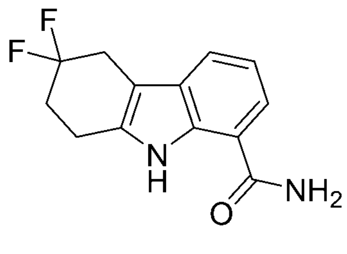 .

The present disclosure further provides a method for preparing the compound of formula I, which is specifically as follows:

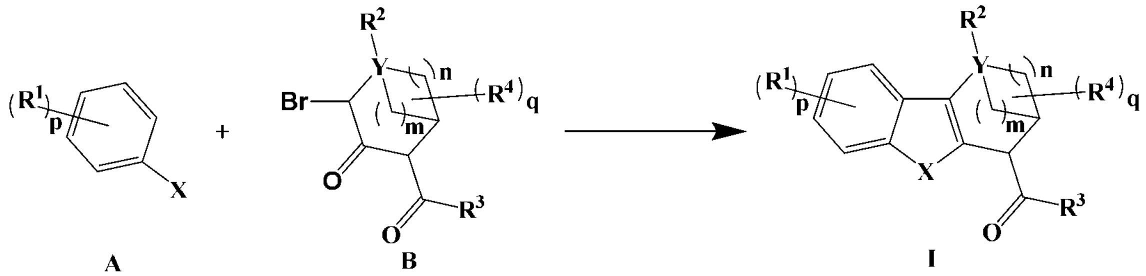

A B I

mixing raw material A and raw material B, heating up to 100-200°C for a reaction for 3-8 hours, cooling to room temperature, performing extraction with an appropriate solvent (such as dichloromethane) and concentration, adding an LiOH solution, stirring at room temperature for 1-3 hours, then performing extraction and concentration again, adding a reaction reagent (such as ammonia water), heating up to 50-100°C for a reaction for 12-36 hours, and then performing separation and purification to obtain the compound of formula I.

The present disclosure further provides a pharmaceutical composition, which includes the aforementioned compound, or the stereoisomer, the tautomer, the solvate, the hydrate, the prodrug, the stable isotope derivative, and the pharmaceutically acceptable salt thereof.

In certain embodiments, a unit dose of the pharmaceutical composition is 0.001 mg - 1,000 mg.

In certain embodiments, based on a total weight of the composition, the pharmaceutical composition includes 0.01%-99.99% of the aforementioned compound. In certain embodiments, the pharmaceutical composition includes 0.1%-99.9% of the aforementioned compound. In certain embodiments, the pharmaceutical composition includes 0.5%-99.5% of the aforementioned compound. In certain embodiments, the pharmaceutical composition includes 1%-99% of the aforementioned compound. In certain embodiments, the pharmaceutical composition includes 2%-98% of the aforementioned compound.

In certain embodiments, based on a total weight of the composition, the pharmaceutical composition includes 0.01%-99.99% of a pharmaceutically acceptable carrier, a diluent, or an excipient. In certain embodiments, the pharmaceutical composition includes 0.1%-99.9% of a pharmaceutically acceptable carrier, a diluent, or an excipient. In certain embodiments, the pharmaceutical composition includes 0.5%-99.5% of a pharmaceutically acceptable carrier, a diluent, or an excipient. In certain embodiments, the pharmaceutical composition includes 1%-99% of a pharmaceutically acceptable carrier, a diluent, or an excipient. In certain embodiments, the pharmaceutical composition includes 2%-98% of a pharmaceutically acceptable carrier, a diluent, or an excipient.

All the compounds involved in the present disclosure, as well as mixtures and compositions containing the compounds of the present disclosure may be administered to an organism via any route of administration. The route of administration may be oral administration, intravenous injection, intramuscular injection, subcutaneous injection, rectal administration, vaginal administration, sublingual administration, nasal inhalation, oral inhalation, eye dropping, and may also be local or systemic transdermal administration.

All the compounds involved in the present disclosure, as well as mixtures and compositions containing the compounds of the present disclosure may be formulated at a single dose, which includes the active compounds of the present disclosure, as well as a carrier, an excipient, etc. A dosage form for administration may be a tablet, a capsule, an injection, a granule, a powder, a suppository, a pill, a cream, a paste, a gel, a dispersible powder, an oral solution, an inhalant, a suspension, a dry suspension, a patch, a lotion, etc. These dosage forms may include ingredients commonly used in pharmaceutical preparations, such as a diluent, an absorbent, a wetting agent, a binder, a disintegrant, a colorant, a pH adjuster, an antioxidant, a bacteriostat, an isotonic regulator, an anti-adherent, etc.

Suitable formulations of the above dosage forms may be obtained from public channels, such as Remington: The Science and Practice of Pharmacy, 21st Edition, published by Lippincott Williams & Wilkins in 2006, and Rowe, Raymond C. Handbook of Pharmaceutical Excipients, Chicago, Pharmaceutical Press, published in 2005, and thus can be easily prepared by those skilled in the art.

According to the nature and severity of diseases in different individuals, the age, gender, and body weight of patients, routes of administration, and other factors, different administration doses may be selected. The administration dose of the compound of the present disclosure may be 0.01 to 500 mg/kg per day, preferably 1-100 mg/kg per day, with administration performed once or several times.

The present disclosure further provides the compound of the present disclosure, or the stereoisomer, the tautomer, the solvate, the hydrate, the prodrug, the stable isotope derivative, and the pharmaceutically acceptable salt thereof or the aforementioned pharmaceutical composition for use in prevention and/or treatment of a skin-related

disease.

In certain embodiments, the skin-related disease is selected from a skin hyperkeratosis-related disease.

In certain embodiments, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

In certain embodiments, the hand-foot syndrome is caused by administration of a group consisting of one or more selected from docetaxel, capecitabine, fluorouracil, paclitaxel, cytarabine, adriamycin, oxaliplatin, and doxorubicin.

In certain embodiments, the hand-foot syndrome is caused by administration of a group consisting of one or more selected from docetaxel and capecitabine.

In certain embodiments, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor.

In certain embodiments, the hand-foot skin reaction is caused by administration of a group consisting of one or more selected from an EGFR inhibitor and a VEGFR inhibitor.

In certain embodiments, the hand-foot skin reaction is caused by administration of a group consisting of one or more selected from sorafenib, lenvatinib, axitinib, donafenib, regorafenib, apatinib, fruquintinib, anlotinib, erlotinib, afatinib, and osimertinib.

The present disclosure further provides use of the compound of the present disclosure, or the stereoisomer, the tautomer, the solvate, the hydrate, the prodrug, the stable isotope derivative, and the pharmaceutically acceptable salt thereof or the aforementioned pharmaceutical composition in prevention and/or treatment of a skin-related disease.

In certain embodiments, the skin-related disease is selected from a skin hyperkeratosis-related disease.

In certain embodiments, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

In certain embodiments, the hand-foot syndrome is caused by administration of a group consisting of one or more selected from docetaxel, capecitabine, fluorouracil, paclitaxel, cytarabine, adriamycin, oxaliplatin, and doxorubicin.

In certain embodiments, the hand-foot syndrome is caused by administration of a group consisting of one or more selected from docetaxel and capecitabine.

In certain embodiments, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor.

In certain embodiments, the hand-foot skin reaction is caused by administration of a group consisting of one or more selected from an EGFR inhibitor and a VEGFR inhibitor.

In certain embodiments, the hand-foot skin reaction is caused by administration of a group consisting of one or more selected from sorafenib, lenvatinib, axitinib, donafenib, regorafenib, apatinib, fruquintinib, anlotinib, erlotinib, afatinib, and osimertinib.

The present disclosure further provides use of the compound of the present disclosure, or the stereoisomer, the tautomer, the solvate, the hydrate, the prodrug, the stable isotope derivative, and the pharmaceutically acceptable salt thereof or the aforementioned pharmaceutical composition in preparation of a drug for preventing and/or treating a skin-related disease.

In certain embodiments, the skin-related disease is selected from a skin hyperkeratosis-related disease.

In certain embodiments, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

In certain embodiments, the hand-foot syndrome is caused by administration of a group consisting of one or more selected from docetaxel, capecitabine, fluorouracil, paclitaxel, cytarabine, adriamycin, oxaliplatin, and doxorubicin.

In certain embodiments, the hand-foot syndrome is caused by administration of a group consisting of one or more selected from docetaxel and capecitabine.

In certain embodiments, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor.

In certain embodiments, the hand-foot skin reaction is caused by administration of a group consisting of one or more selected from an EGFR inhibitor and a VEGFR inhibitor.

In certain embodiments, the hand-foot skin reaction is caused by administration of a group consisting of one or more selected from sorafenib, lenvatinib, axitinib, donafenib, regorafenib, apatinib, fruquintinib, anlotinib, erlotinib,

afatinib, and osimertinib.

The present disclosure further provides the compound of the present disclosure, or the stereoisomer, the tautomer, the solvate, the hydrate, the prodrug, the stable isotope derivative, and the pharmaceutically acceptable salt thereof or the aforementioned pharmaceutical composition for use in prevention and/or treatment of an abnormal RNA m6A methylation-related disease.

In certain embodiments, the abnormal RNA m6A methylation-related disease is selected from a skin-related disease.

In certain embodiments, the skin-related disease is selected from a skin hyperkeratosis-related disease.

In certain embodiments, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

In certain embodiments, the hand-foot syndrome is caused by administration of a group consisting of one or more selected from docetaxel, capecitabine, fluorouracil, paclitaxel, cytarabine, adriamycin, oxaliplatin, and doxorubicin.

In certain embodiments, the hand-foot syndrome is caused by administration of a group consisting of one or more selected from docetaxel and capecitabine.

In certain embodiments, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor.

In certain embodiments, the hand-foot skin reaction is caused by administration of a group consisting of one or more selected from an EGFR inhibitor and a VEGFR inhibitor.

In certain embodiments, the hand-foot skin reaction is caused by administration of a group consisting of one or more selected from sorafenib, lenvatinib, axitinib, donafenib, regorafenib, apatinib, fruquintinib, anlotinib, erlotinib, afatinib, and osimertinib.

The present disclosure further provides use of the compound of the present disclosure, or the stereoisomer, the tautomer, the solvate, the hydrate, the prodrug, the stable isotope derivative, and the pharmaceutically acceptable salt thereof or the aforementioned pharmaceutical composition in prevention and/or treatment of an abnormal RNA m6A methylation-related disease.

In certain embodiments, the abnormal RNA m6A methylation-related disease is selected from a skin-related disease.

In certain embodiments, the skin-related disease is selected from a skin hyperkeratosis-related disease.

In certain embodiments, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

In certain embodiments, the hand-foot syndrome is caused by administration of a group consisting of one or more selected from docetaxel, capecitabine, fluorouracil, paclitaxel, cytarabine, adriamycin, oxaliplatin, and doxorubicin.

In certain embodiments, the hand-foot syndrome is caused by administration of a group consisting of one or more selected from docetaxel and capecitabine.

In certain embodiments, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor.

In certain embodiments, the hand-foot skin reaction is caused by administration of a group consisting of one or more selected from an EGFR inhibitor and a VEGFR inhibitor.

In certain embodiments, the hand-foot skin reaction is caused by administration of a group consisting of one or more selected from sorafenib, lenvatinib, axitinib, donafenib, regorafenib, apatinib, fruquintinib, anlotinib, erlotinib, afatinib, and osimertinib.

The present disclosure further provides use of the compound of the present disclosure, or the stereoisomer, the tautomer, the solvate, the hydrate, the prodrug, the stable isotope derivative, and the pharmaceutically acceptable salt thereof or the aforementioned pharmaceutical composition in preparation of a drug for preventing and/or treating an abnormal RNA m6A methylation-related disease.

In certain embodiments, the abnormal RNA m6A methylation-related disease is selected from a skin-related disease.

In certain embodiments, the skin-related disease is selected from a skin hyperkeratosis-related disease.

In certain embodiments, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis.

In certain embodiments, the hand-foot syndrome is caused by administration of a group consisting of one or more selected from docetaxel, capecitabine, fluorouracil, adriamycin, oxaliplatin, and daunorubicin.

In certain embodiments, the hand-foot syndrome is caused by administration of a group consisting of one or more selected from docetaxel and capecitabine.

In certain embodiments, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor.

In certain embodiments, the hand-foot skin reaction is caused by administration of a group consisting of one or more selected from an EGFR inhibitor and a VEGFR inhibitor.

In certain embodiments, the hand-foot skin reaction is caused by administration of a group consisting of one or more selected from sorafenib, lenvatinib, axitinib, donafenib, regorafenib, apatinib, fruquintinib, anlotinib, erlotinib, afatinib, and osimertinib.

Definition of terms:

Unless otherwise stated, the terms used in the specification and claims have the following meanings.

The term "alkyl" refers to a saturated aliphatic hydrocarbon group, which is a linear or branched group containing 1 to 20 carbon atoms, preferably alkyl containing 1 to 12 (e.g., 1, 2, 3, 4, 5, 6, 7, 8, 9, 10, 11, and 12) carbon atoms, and more preferably alkyl containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like. More preferred is a lower alkyl group containing 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted. When substituted, it may be substituted at any accessible connection site, where the substituent is preferably one or more substituents independently and optionally selected from a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The residue derived by removing one hydrogen atom from the above alkyl group is "alkylene group".

The term "alkylene" refers to an alkyl compound containing at least one carbon-carbon double bond in the molecule, where the alkyl is defined as above. The alkylene can be substituted or unsubstituted, when substituted, the substituents are preferably one or more of the following groups, independently selected from one or more of a alkoxyl, halogen, haloalkyl, haloalkoxyl, cycloalkyloxyl, heterocyclic oxyl, hydroxyl, hydroxyalkyl, cyano, amino, nitro, cycloalkyl, heterocyclyl, aryl group, and heteroaryl.

The term "alkenyl" refers to an alkyl compound containing at least one carbon-carbon triple bond in the molecule, where the alkyl is defined as above. The alkenyl can be substituted or unsubstituted, when substituted, the substituents are preferably one or more of the following groups, independently selected from one or more of a alkoxyl, halogen, haloalkyl, haloalkoxyl, cycloalkyloxyl, heterocyclic oxyl, hydroxyl, hydroxyalkyl, cyano, amino, nitro, cycloalkyl, heterocyclyl, aryl group, and heteroaryl.

The term "cycloalkyl" refers to a saturated or partially unsaturated monocyclic or polycyclic cyclic hydrocarbon substituent. The cycloalkyl ring contains 3 to 20 carbon atoms, preferably 3 to 12 carbon atoms, preferably 3 to 8 (e.g., 3, 4, 5, 6, 7, and 8) carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic cycloalkyl include spirocyclic, fused, and bridged cycloalkyl.

The term "spirocycloalkyl" refers to a 5- to 20-membered polycyclic group where single rings share one carbon atom (called a spiro atom), which may contain one or more double bonds. It is preferably 6- to 14-membered, more preferably 7- to 10-membered (e.g., 7, 8, 9, or 10-membered). Spirocycloalkyl is classified into mono-spirocycloalkyl, bis-spirocycloalkyl, or poly-spirocycloalkyl according to the number of spiro atoms shared between rings, preferably mono-spirocycloalkyl and bis-spirocycloalkyl. More preferred is 3-membered/5-membered, 3-membered/6-membered, 4-membered/4-membered, 4-membered/5-membered, 4-membered/6-membered, 5-membered/5-membered, or 5-membered/6-membered mono-spirocycloalkyl. Non-limiting examples of spirocycloalkyl include:

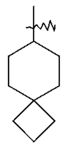 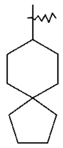 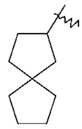 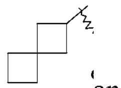 and 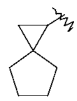 .

The term "fused cycloalkyl" refers to a 5- to 20-membered all-carbon polycyclic group where each ring in the system shares an adjacent pair of carbon atoms with other rings in the system, and one or more rings may contain one or more double bonds. It is preferably 6- to 14-membered, more preferably 7- to 10-membered (e.g., 7, 8, 9, or 10-membered). Fused cycloalkyl can be classified into bicyclic, tricyclic, tetracyclic, or polycyclic fused cycloalkyl according to the number of constituent rings, preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic alkyl groups. Non-limiting examples of fused cycloalkyl include:

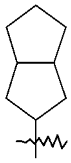 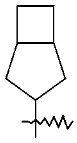 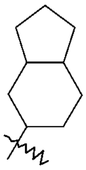 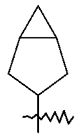 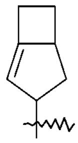 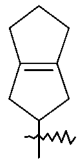 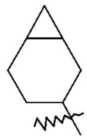 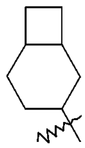 and 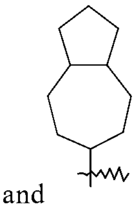 .

The term "bridged cycloalkyl" refers to a 5- to 20-membered all-carbon polycyclic group where any two rings share two non-directly connected carbon atoms, which may contain one or more double bonds. It is preferably 6- to 14-membered, more preferably 7- to 10-membered (e.g., 7, 8, 9, or 10-membered). Bridged cycloalkyl can be classified into bicyclic, tricyclic, tetracyclic, or polycyclic bridged cycloalkyl groups according to the number of constituent rings, preferably bicyclic, tricyclic, or tetracyclic, and more preferably bicyclic or tricyclic. Non-limiting examples of bridged cycloalkyl groups include:

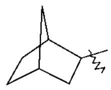 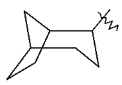 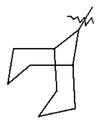 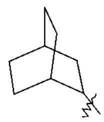 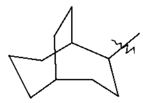

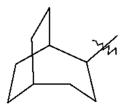 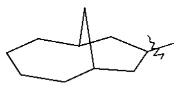 and 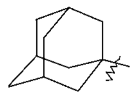 .

The cycloalkyl include the aforementioned cycloalkyl (including monocyclic, spirocyclic, fused-ring, and bridged-ring) fused to aryl, heteroaryl, or heterocyclic rings, where the ring connected to the parent structure is a cycloalkyl group. Non-limiting examples include:

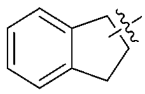 , 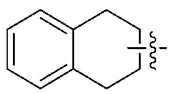 , 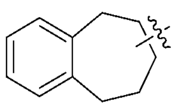 ,

etc.; preferably

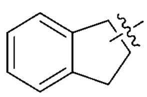 and 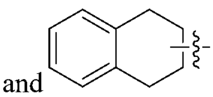 .

Cycloalkyl may be substituted or unsubstituted; when substituted, the cycloalkyl may be substituted at any

available attachment point, and the substituents are preferably independently and optionally selected from one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclic, aryl, and heteroaryl.

The term "alkoxy" refers to -O-(alkyl) and -O-(cycloalkyl), where the alkyl and cycloalkyl are defined as above. Non-limiting examples of alkoxy include methoxy, ethoxy, propoxy, and butoxy. Alkoxy may be optionally substituted or unsubstituted; when substituted, the alkoxy is preferably one or more of the following groups, independently selected from deuterium atom, halogen, alkoxy, haloalkyl, haloalkoxy, cycloalkyloxy, heterocyclyloxy, hydroxyl, hydroxyalkyl, cyano, amino, nitro, cycloalkyl, heterocyclic, aryl, and heteroaryl.

The term "heterocyclyl" refers to a saturated or partially unsaturated non-aromatic monocyclic or polycyclic cyclic substituent containing 3 to 20 ring atoms, where one or more ring atoms are heteroatoms selected from nitrogen, oxygen, and sulfur. The sulfur can be optionally oxidized (i.e., forming a sulfoxide or sulfone), but excludes -O-O-, -O-S-, or -S-S-, and the remaining ring atoms are carbon. It preferably contains 3 to 12 (e.g., 3, 4, 5, 6, 7, 8, 9, 10, 11, and 12) ring atoms, of which 1 to 4 (e.g., 1, 2, 3, and 4) are heteroatoms; more preferably contains 3 to 8 ring atoms (e.g., 3, 4, 5, 6, 7, and 8), of which 1 to 3 (e.g., 1, 2, and 3) are heteroatoms; more preferably contains 3 to 6 ring atoms, of which 1 to 3 are heteroatoms; most preferably contains 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, etc. Polycyclic heterocyclyl include spirocyclic, fused, and bridged heterocyclyl.

The term "spiroheterocyclyl" refers to a 5- to 20-membered non-aromatic polycyclic heterocyclic group where single rings share one atom (called a spiro atom), where one or more ring atoms are heteroatoms selected from nitrogen, oxygen, and sulfur. The sulfur can be optionally oxidized (i.e., forming a sulfoxide or sulfone), but excludes -O-O-, -O-S-, or -S-S-, and the remaining ring atoms are carbon. It may contain one or more double bonds. It is preferably 6- to 14-membered, more preferably 7- to 10-membered (e.g., 7, 8, 9, or 10-membered). Spiroheterocyclyl is classified into mono-spiroheterocyclyl, bis-spiroheterocyclyl, or poly-spiroheterocyclyl according to the number of spiro atoms shared between rings, preferably mono-spiroheterocyclyl and bis-spiroheterocyclyl. More preferred is 3-membered/5-membered, 3-membered/6-membered, 4-membered/4-membered, 4-membered/5-membered, 4-membered/6-membered, 5-membered/5-membered, or 5-membered/6-membered mono-spiroheterocyclyl. Non-limiting examples of spiroheterocyclyl groups include:

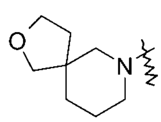 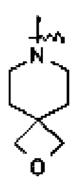 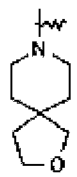 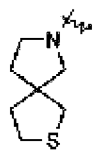 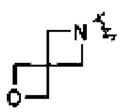 and 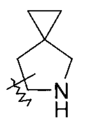 .

The term "fused heterocyclyl" refers to a 5- to 20-membered non-aromatic polycyclic heterocyclic group where each ring in the system shares an adjacent pair of atoms with other rings in the system. One or more rings may contain one or more double bonds, where one or more ring atoms are heteroatoms selected from nitrogen, oxygen, and sulfur. The sulfur can be optionally oxidized (i.e., forming a sulfoxide or sulfone), but excludes - O-O-, -O-S-, or -S-S-, and the remaining ring atoms are carbon. It is preferably 6- to 14-membered, more preferably 7- to 10-membered (e.g., 7, 8, 9, or 10-membered). Fused heterocyclyl can be classified into bicyclic, tricyclic, tetracyclic, or polycyclic fused heterocyclyl according to the number of constituent rings, preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl. Non-limiting examples of fused heterocyclyl include:

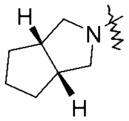 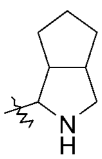 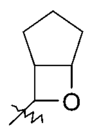 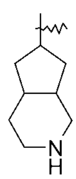 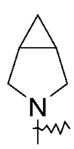 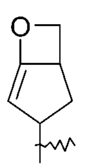 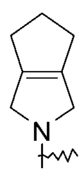 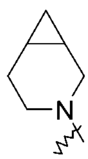

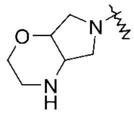 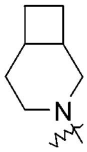 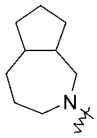 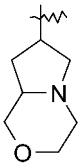 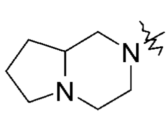 and 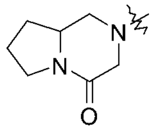 .

The term "bridged heterocyclyl" refers to a 5- to 14-membered non-aromatic polycyclic heterocyclic group where any two rings share two non-directly connected atoms, which may contain one or more double bonds. One or more ring atoms are heteroatoms selected from nitrogen, oxygen, and sulfur. The sulfur can be optionally oxidized (i.e., forming a sulfoxide or sulfone), but excludes -O-O-, -O-S-, or -S-S-, and the remaining ring atoms are carbon. It is preferably 6- to 14-membered, more preferably 7- to 10-membered (e.g., 7, 8, 9, or 10-membered). Bridged heterocyclyl can be classified into bicyclic, tricyclic, tetracyclic, or polycyclic bridged heterocyclyl according to the number of constituent rings, preferably bicyclic, tricyclic, or tetracyclic, and more preferably bicyclic or tricyclic. Non-limiting examples of bridged heterocyclyl include:

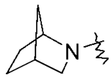 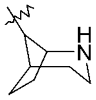 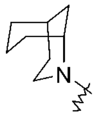 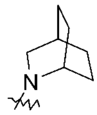

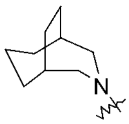 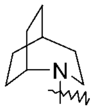 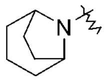 and 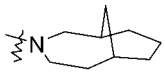 .

The heterocyclyl rings include the aforementioned heterocyclyl (including monocyclic, spiroheterocyclic, fused heterocyclic, and bridged heterocyclic) fused to aryl, heteroaryl, or cycloalkyl rings, where the ring connected to the parent structure is a heterocyclyl group. Non-limiting examples include:

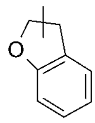 ,

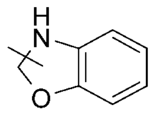 , 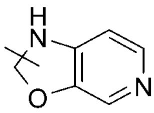 and 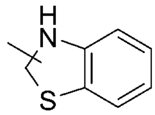 , etc.

Heterocyclyl can be substituted or unsubstituted. When substituted, it can be substituted at any available point of attachment, and the substituents are preferably selected from one or more of halogen, alkyl, alkoxy, haloalkyl, haloalkoxy, cycloalkyloxyl, heterocyclyloxyl, hydroxyl, hydroxyalkyl, oxo, cyano, amino, nitro, cycloalkyl, heterocyclyl, aryl and heteroaryl.

The term "aryl" refers to a 6- to 14-membered all-carbon monocyclic or fused polycyclic (fused polycyclic refers to rings sharing an adjacent pair of carbon atoms) group with a conjugated $\pi$-electron system, preferably 6- to 10-membered, such as phenyl and naphthyl. The aryl includes the aforementioned aryl rings fused to heteroaryl, heterocyclyl, or cycloalkyl rings, where the ring connected to the parent structure is an aryl ring. Non-limiting examples include:

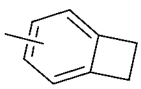 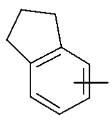 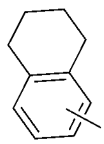 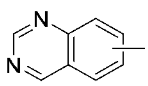 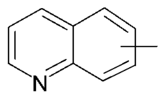 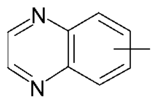 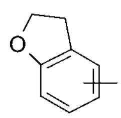 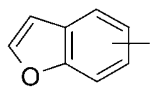

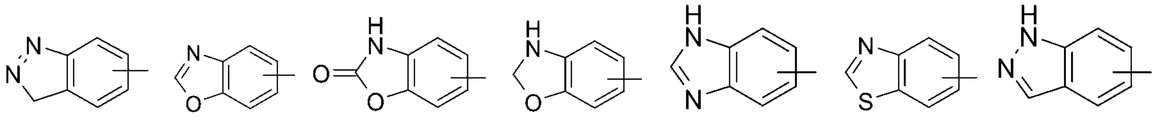

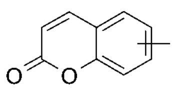 and 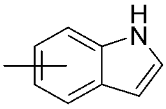 .

Aryl can be substituted or unsubstituted. When substituted, it can be substituted at any available attachment point, and the substituents are preferably selected from one or more of halogen, alkyl, alkoxyl, haloalkyl, haloalkoxyl, cycloalkyloxyl, heterocyclic oxyl, hydroxyll, hydroxyalkyl 1, oxosl, cyanosl, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, where the heteroatoms are selected from oxygen, sulfur, and nitrogen. Heteroaryl is preferably 5- to 10-membered (e.g., 5, 6, 7, 8, 9, or 10-membered), more preferably 5-membered or 6-membered, such as furyl, thienyl, pyridyl, pyrrolyl, N-alkylpyrrolyl, pyrimidinyl, pyrazinyl, pyridazinyl, imidazolyl, pyrazolyl, triazolyl, tetrazolyl, etc. The heteroaryl groups include the aforementioned heteroaryl fused to aryl, heterocyclyl, or cycloalkyl rings, where the ring connected to the parent structure is a heteroaryl ring. Non-limiting examples of heteroaryl groups include:

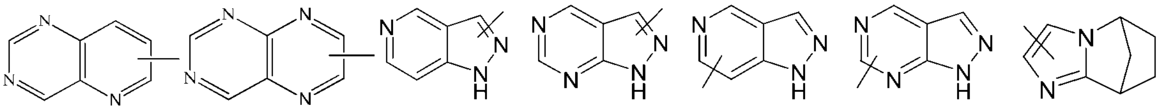

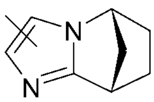 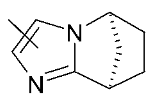 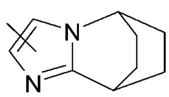 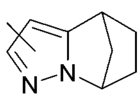 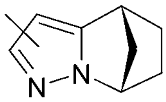 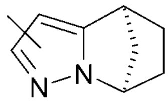

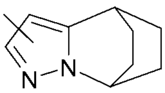 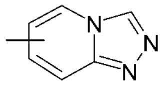 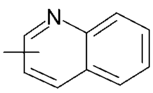 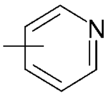 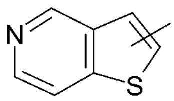 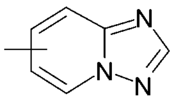 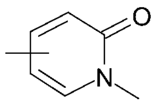

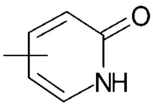 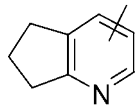 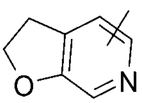 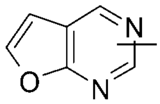 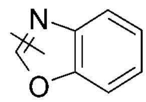 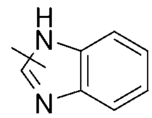 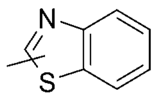

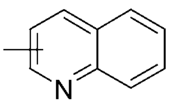 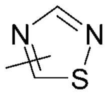 and 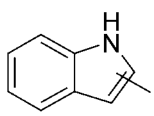 .

Heteroaryl can be substituted or unsubstituted. When substituted, it can be substituted at any available point of attachment, and the substituents are preferably selected from one or more of halogen, alkyl, alkoxy, haloalkyl, haloalkoxy, cycloalkyloxy, heterocyclyloxyl, hydroxyl, hydroxyalkyl, cyano, amino, nitro, cycloalkyl, heterocyclyl, aryl and heteroaryl.

The term "halogen" refers to fluorine, chlorine, bromine or iodine.

The term "hydroxyl" refers to -OH.

The term "amino" refers to $-NH_2$.

The term "cyano" refers to -CN.

The term "nitro" refers to $-NO_2$.

The term "stereoisomer" refers to compounds with the same chemical structure but different spatial arrangements of atoms or groups. Stereoisomers include enantiomers, diastereomers, conformational isomers (rotamers), geometric (cis/trans) isomers, atropisomers, etc.

The term "tautomer" refers to structural isomers of different energies that can interconvert via a low energy

barrier. For example, proton tautomers (also called proton transfer tautomers) include interconversion via proton migration, such as keto-enol and imine-enamine, lactam-lactim isomerization, pyrazolyl isomerization, etc.

The compounds of the present disclosure include their isotopic derivatives. The term "isotopic derivative" refers to compounds that differ in structure only in the presence of one or more isotope-enriched atoms. For example, compounds having the structure of the present disclosure, where hydrogen is replaced with deuterium or tritium, or fluorine is replaced with $^{18}F$-fluorine label ($^{18}F$ isotope), or carbon atoms are replaced with $^{11}C$- , $^{13}C$-, or $^{14}C$-enriched carbon ($^{11}C$, $^{13}C$, or $^{14}C$-carbon label; $^{11}C$, $^{13}C$, or $^{14}C$-isotope) are within the scope of the present disclosure. Such compounds can be used, for example, as analytical tools or probes in biological assays, or as in vivo diagnostic imaging tracers for diseases, or as tracers for pharmacodynamic, pharmacokinetic, or receptor studies. Various deuterated forms of the compounds of the present disclosure mean that each available hydrogen atom attached to a carbon atom can be independently replaced with a deuterium atom. A person skilled in the art can synthesize deuterated forms of the compounds with reference to relevant literature. Commercially available deuterated starting materials can be used in the preparation of deuterated forms of the compounds, or they can be synthesized using deuterated reagents using conventional techniques, including but not limited to deuterated borane, borane-d3 tetrahydrofuran solution, lithium aluminum deuteride, ethyl-d5 iodide, methyl-d3 iodide, etc. Deuterated compounds can generally retain activity comparable to that of non-deuterated compounds, and when deuterated at certain specific positions, can achieve better metabolic stability, thereby obtaining certain therapeutic advantages.

"Substituted" means that one or more hydrogen atoms in a group, preferably 1 to 5, more preferably 1 to 3 hydrogen atoms, are independently replaced by the corresponding number of substituents. Those skilled in the art can determine possible or impossible substitutions without undue effort (through experiments or theory). For example, an amino or a hydroxyl group with a free hydrogen may be unstable when combined with a carbon atom having an unsaturated bond (such as an alkene).

"Pharmaceutical composition" means a mixture containing one or more of the compounds described herein or the pharmaceutically acceptable salts thereof and other chemical components, and other components such as pharmaceutically acceptable carriers and excipients. The purpose of the pharmaceutical composition is to facilitate the administration to organisms, facilitating the absorption of the active ingredient to exert its biological activity.

"Pharmaceutically acceptable salt" refers to a salt of the compounds of the present disclosure. Such salts are safe and effective when used in mammals and have the desired biological activity. They can be prepared separately during the final separation and purification of the compound, or by reacting a suitable group with a suitable base or acid. Bases commonly used to form pharmaceutically acceptable salts include inorganic and organic bases. Acids commonly used to form pharmaceutically acceptable salts include both inorganic and organic acids.

In the context of a drug or a pharmacological active agent, the term "therapeutically effective amount" refers to a non-toxic but sufficient amount of the drug or agent to achieve the desired effect. The determination of an effective amount varies from person to person, depending on the age and general condition of the recipient, as well as the specific active substance. A suitable effective amount in individual cases can be determined by a person skilled in the art through routine experiments.

As used herein, the term "Hydrate" refers to a substance formed by the binding of the compounds of the present disclosure or the pharmaceutically acceptable salt thereof to water through non-covalent intermolecular forces. Common hydrates include, but are not limited to, hemihydrate, monohydrate, dihydrate, trihydrate, etc.

"Prodrug" refers to a substance that can be converted *in vivo* under physiological conditions, e.g., by hydrolysis in blood, to generate the active parent drug compound.

"Pharmaceutically acceptable" means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use.

As used herein, the singular forms "a", "an", and "the" include plural references and vice versa, unless the context clearly indicates otherwise.

The terms involved in the present disclosure are defined above, the above terms may also be understood by those skilled in the art in conjunction with the prior art, and the following description is further provided based on the contents of the present disclosure and the definition of the terms.

The following examples are merely used for illustrating the present disclosure and are not intended to limit the scope of the present disclosure. Experimental methods without specific conditions in the following examples are generally used according to conventional conditions or according to conditions recommended by manufacturers. Unless otherwise specified, parts and percentages are parts by weight and percentages by weight. In a preparation process, each reaction is carried out in an inert solvent at a temperature ranging from room temperature to a reflux temperature. A reaction time is generally from 0.25 hour to 48 hours.

Further, ACQUITY Arc of Waters or equivalent equipment is used as a liquid chromatograph-mass spectrometer (LC-MS). Mass spectrometry (MS) is performed using an electrospray ionization (ESI) source, which indicates only the

molecular weight M of a parent molecule, and is generally reported as $[M+H]^+$. Further, a nuclear magnetic resonance (NMR) spectrogram adopts a Varian 400 MHz nuclear magnetic resonance spectrometer or equivalent equipment to obtain data, which usually adopts $CDCl_3$ or DMSO-$d_6$ as a solvent and reports a chemical shift in ppm. Various peaks are described as follows: s (singlet), d (doublet), t (triplet), q (quartet), m (multiplet), dd (double doublet). A coupling constant is expressed in Hz.

A list of abbreviations used in an experimental section is as follows:

| | |
|---|---|
| AA | Ammonium acetate |
| AcOH | Acetic acid |
| Boc | Tert-butoxycarbonyl |
| DAST | Diethylaminosulfur trifluoride |
| DIEA | N,N-diisopropylethylamine |
| DCM | Dichloromethane |
| DMSO | Dimethyl sulfoxide |
| DMF | Dimethylformamide |
| THF | Tetrahydrofuran |
| TFA | Trifluoroacetic acid 2-(7-azabenzotriazole)-N,N,N',N'-tetramethyluronium |
| HATU | hexafluorophosphate |

**Example 1:** Preparation of 6-fluoro-2,3,4,9-tetrahydro-1H-carbazole-1-carboxamide **(compound 1)**

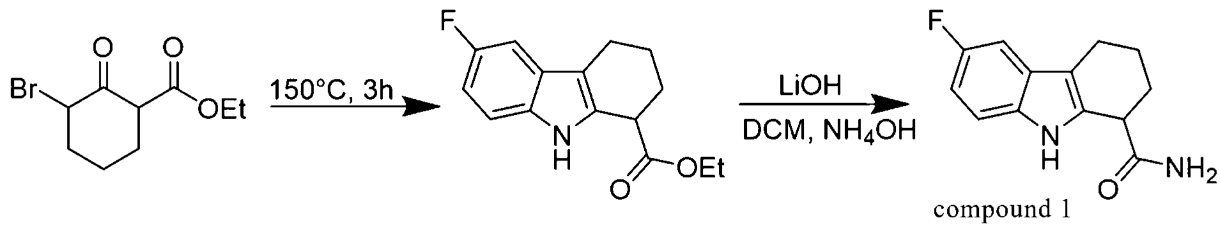


compound 1

Ethyl 3-bromo-2-oxocyclohexane-1-formate (20 mg, 0.08 mmol) and 4-fluoroaniline (22 mg, 0.2 mmol) were mixed and heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-fluoro-2,3,4,9-tetrahydro-1H-carbazole-1-formate (15.6 mg). 10 mg of the ethyl 6-fluoro-2,3,4,9-tetrahydro-1H-carbazole-1-formate (0.038 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.038 mmol), and acetonitrile (5 mL) were added for a reaction at room temperature for 24 hours, and the mixture was separated and purified by silica gel column chromatography to obtain 6-fluoro-2,3,4,9-tetrahydro-1H-carbazole-1-carboxamide as a white solid (compound 1, 6.2 mg). LCMS $[M+H]^+$: 233. $^1$H-NMR (400 MHz, $CDCl_3$): $\delta$ 8.52 (s, 1H), 7.32-7.41 (m, 1H), 7.24-7.30 (m, 1H), 6.76-6.82 (m, 1H), 5.76-5.81 (m, 2H), 3.68 (m, 1H), 2.67-2.72 (m, 2H), 1.64-1.90 (m, 4H).

**Example 2:** Preparation of 6-chloro-2,3,4,9-tetrahydro-1H-carbazole-1-carboxamide **(compound 2),** (*R*)-6-chloro-2,3,4,9-tetrahydro-1H-carbazole-1-carboxamide **(compound 2a),** and (S)-6-chloro-2,3,4,9-tetrahydro-1H-carbazole-1-carboxamide **(compound 2b)**

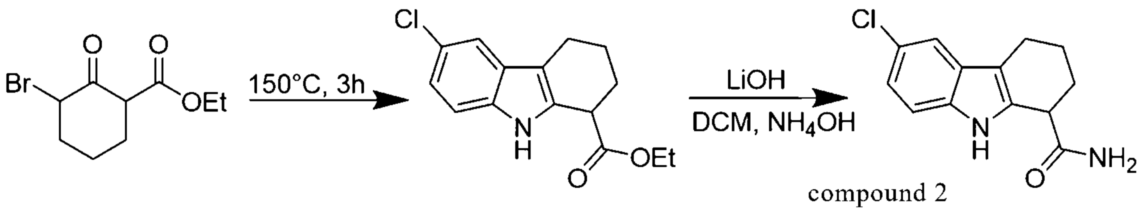


compound 2

Ethyl 3-bromo-2-oxocyclohexane-1-formate (20 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-2,3,4,9-tetrahydro-1H-carbazole-1-formate (16 mg). 10 mg of the ethyl 6-chloro-2,3,4,9-tetrahydro-1H-carbazole-1-formate (0.036 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by silica gel column chromatography to obtain 6-chloro-2,3,4,9-tetrahydro-1H-carbazole-1-carboxamide as a white solid (compound 2, 7.6 mg), which was then separated by using a Chiralpak AD chiral column to obtain (R)-6-chloro-2,3,4,9-tetrahydro-1H-carbazole-1-carboxamide (compound 2a) and (S)-6-chloro-2,3,4,9-tetrahydro-1H-carbazole-1-carboxamide (compound 2b). LCMS $[M+H]^+$ for the compounds 2a and 2b: 249. $^1$H-NMR (400 MHz, DMSO-$d_6$): δ 10.79 (s, 1H), 7.37-7.39 (m, 2H), 7.28 (d, $J$ = 8.0 Hz, 1H), 7.08 (s, 1H), 6.98-7.01 (m, 1H), 3.64-3.67 (m, 1H), 2.58-2.61 (m, 2H), 1.66-2.04 (m, 4H).

**Example 3:** Preparation of 6-chloro-5-fluoro-2,3,4,9-tetrahydro-1H-carbazole-1-carboxamide (**compound 3a**)

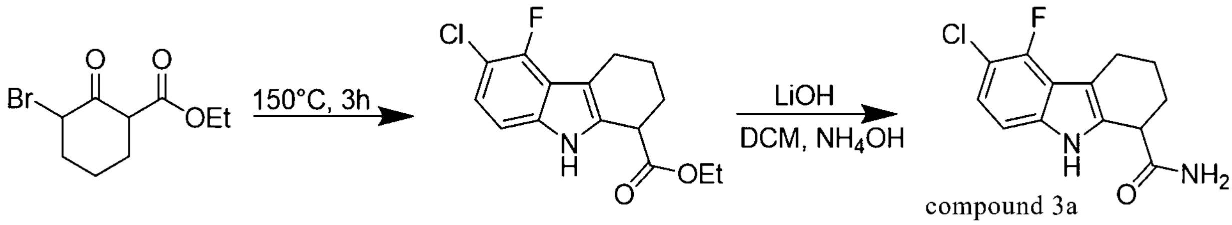


compound 3a

Ethyl 3-bromo-2-oxocyclohexane-1-formate (20 mg, 0.08 mmol) and 4-chloro-3-fluoroaniline (29 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-5-fluoro-2,3,4,9-tetrahydro-1H-carbazole-1-formate (10 mg). 10 mg of the ethyl 6-chloro-5-fluoro-2,3,4,9-tetrahydro-1H-carbazole-1-formate (0.034 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was purified by supercritical fluid chromatography (SFC)-preparative liquid chromatography to obtain 6-chloro-5-fluoro-2,3,4,9-tetrahydro-1H-carbazole-1-carboxamide as a white solid (compound 3a, 7 mg). LCMS $[M+H]^+$: 267. $^1$H-NMR (400 MHz, $CDCl_3$): δ 8.63 (s, 1H), 7.45 (d, $J$ = 8 Hz 1H), 7.10 (d, $J$ = 8.0 Hz, 1H), 5.52-5.68 (m, 2H), 3.75 (t, $J$ = 6.2 Hz, 1H), 2.71 (t, $J$ = 5.5 Hz, 2H), 1.66-2.24 (m, 4H).

**Example 4:** Preparation of 6-chloro-7-fluoro-2,3,4,9-tetrahydro-1H-carbazole-1-carboxamide (**compound 3b**)

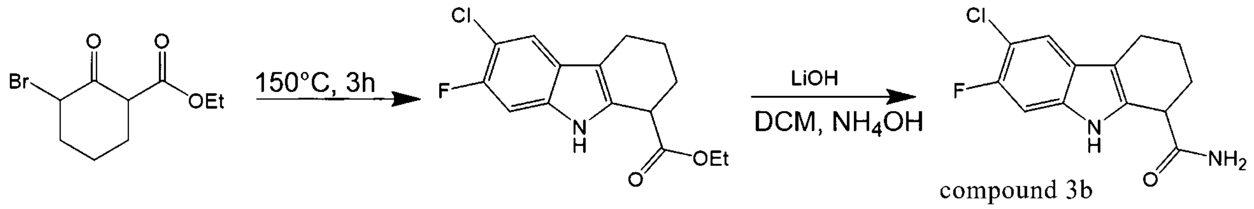

compound 3b

Ethyl 3-bromo-2-oxocyclohexane-1-formate (20 mg, 0.08 mmol) and 4-chloro-3-fluoroaniline (29 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-7-fluoro-2,3,4,9-tetrahydro-1H-carbazole-1-formate (9 mg). 9 mg of the ethyl 6-chloro-7-fluoro-2,3,4,9-tetrahydro-1H-carbazole-1-formate (0.03 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by SFC-preparative liquid chromatography to obtain 6-chloro-7-fluoro-2,3,4,9-tetrahydro-1H-carbazole-1-carboxamide as a white solid (compound 3b, 6 mg). LCMS $[M+H]^+$: 267. $^1H$-NMR (400 MHz, $CDCl_3$): δ 8.71 (s, 1H), 7.31-7.43 (m, 1H), 7.23 (s, 1H), 5.51-5.71 (m, 2H), 3.62-3.65 (m, 1H), 2.59-2.63 (m, 2H), 1.66-2.01 (m, 4H).

**Example 5:** Preparation of 6-chloro-5,7-difluoro-2,3,4,9-tetrahydro-1H-carbazole-1-carboxamide **(compound 4)**

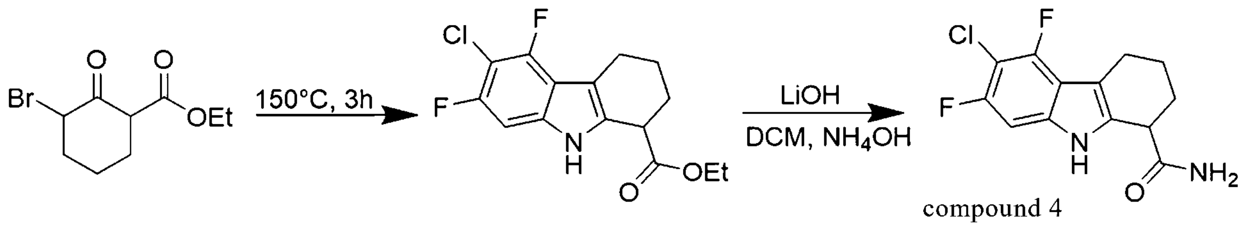

compound 4

Ethyl 3-bromo-2-oxocyclohexane-1-formate (20 mg, 0.08 mmol) and 4-chloro-3,5-difluoroaniline (32.7 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-5,7-difluoro-2,3,4,9-tetrahydro-1H-carbazole-1-formate (16 mg). 10 mg of the ethyl 6-chloro-5,7-difluoro-2,3,4,9-tetrahydro-1H-carbazole-1-formate (0.032 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by silica gel column chromatography to obtain 6-chloro-5,7-difluoro-2,3,4,9-tetrahydro-1H-carbazole-1-carboxamide as a white solid (compound 4, 6.8 mg). LCMS $[M+H]^+$: 285. $^1H$-NMR (400 MHz, $CDCl_3$): δ 8.72 (s, 1H), 7.28-7.35 (m, 1H), 5.35-5.41 (m, 2H), 3.58-3.65 (m, 1H), 2.58-2.65 (m, 2H), 1.67-2.04 (m, 4H).

**Example 6:** Preparation of 2-chloro-5,6,7,8,9,10-hexahydro-7,10-methanocyclohepta[b]indole-6-carboxamide **(compound 5),** (*S*)-2-chloro-5,6,7,8,9,10-hexahydro-7,10-methanocyclohepta[b]indole-6-carboxamide **(compound 5a),** and (R)-2-chloro-5,6,7,8,9,10-hexahydro-7,10-methanocyclohepta[b]indole-6-carboxamide **(compound 5b)**

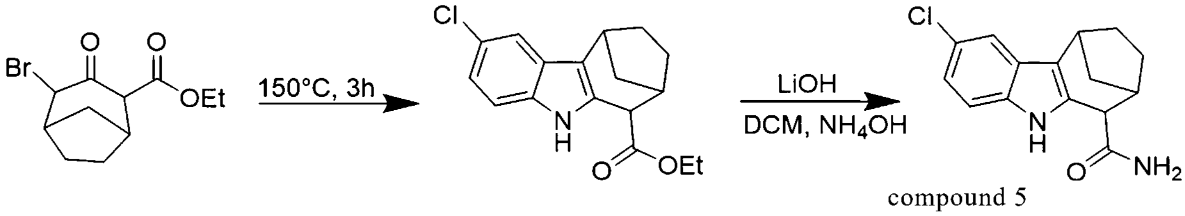

compound 5

Ethyl 4-bromo-3-oxobicyclo[3.2.1]octane-2-carboxylate (22 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 2-chloro-5,6,7,8,9-hexahydro-7,10-methanocyclohepta[b]indole-6-formate (18.5 mg). 10 mg of the ethyl 2-chloro-5,6,7,8,9-hexahydro-7,10-methanocyclohepta[b]indole-6-formate (0.033 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by silica gel column chromatography to obtain 2-chloro-5,6,7,8,9,10-hexahydro-7,10-methanocyclohepta[b]indole-6-carboxamide as a white solid (compound 5, 7.3 mg), which was then separated by using a Chiralpak AD chiral column to obtain (S)-2-chloro-5,6,7,8,9,10-hexahydro-7,10-methanocyclohepta[b]indole-6-carboxamide (compound 5a) and (R)-2-chloro-5,6,7,8,9,10-hexahydro-7,10-methanocyclohepta[b]indole-6-carboxamide (compound 5b). LCMS $[M+H]^+$ for the compounds 5a and 5b: 275. $^1$H-NMR (400 MHz, DMSO-$d_6$): δ 10.74 (s, 1H), 7.30-7.33 (m, 2H), 7.24 (d, $J$ = 8.0 Hz, 1H), 7.18 (s, 1H), 6.93 (d, $J$ = 8.0 Hz, 1H), 3.52-3.58 (m, 1H), 2.77-2.80 (m, 1H), 2.17-2.20 (m, 1H), 1.56-1.92 (m, 6H).

**Example 7:** Preparation of 6-chloro-4-methyl-2,3,4,9-tetrahydro-1H-carbazole-1-carboxamide **(compound 6)**

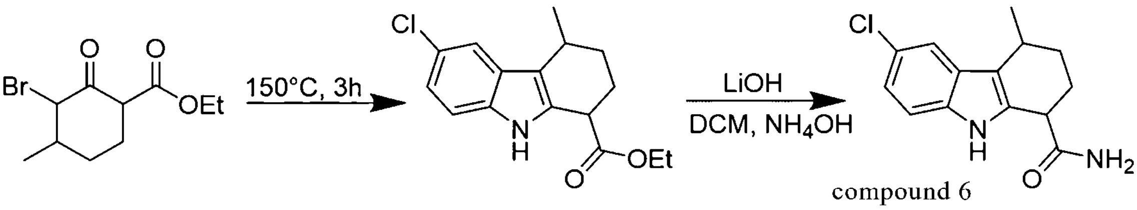

compound 6

Ethyl 3-bromo-4-methyl-2-oxocyclohexane-1-formate (21 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-4-methyl-2,3,4,9-tetrahydro-1H-carbazole-1-formate (18 mg). 10 mg of the ethyl 6-chloro-4-methyl-2,3,4,9-tetrahydro-1H-carbazole-1-formate (0.034 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by silica gel column chromatography to obtain 6-chloro-4-methyl-2,3,4,9-tetrahydro-1H-carbazole-1-carboxamide as a white solid (compound 6, 7.1 mg). LCMS $[M+H]^+$: 263. $^1$H-NMR (400 MHz, $CDCl_3$): δ 8.72-8.77 (m, 1H), 8.44 (s, 1H), 7.54-7.56 (m, 1H), 7.45 (s, 1H), 7.21-7.23 (m, 2H), 7.09-7.12 (m, 2H), 5.62-5.75 (m, 4H), 3.71 (s, 1H), 3.37 (m, 1H), 3.10-3.12 (m, 1H), 2.72-2.73 (m, 1H), 2.03-2.27 (m, 4H), 1.19-1.37 (m, 6H).

**Example 8:** Preparation of 6-chloro-4-fluoro-2,3,4,9-tetrahydro-1H-carbazole-1-carboxamide (**compound** 7)

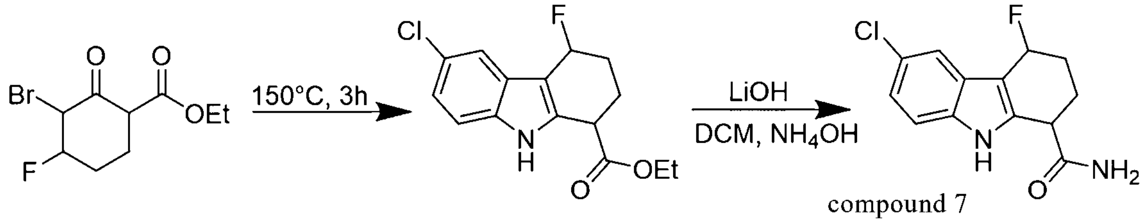

compound 7

Ethyl 3-bromo-4-fluoro-2-oxocyclohexane-1-formate (21.4 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-4-fluoro-2,3,4,9-tetrahydro-1H-carbazole-1-formate (19 mg). 10 mg of the ethyl 6-chloro-4-fluoro-2,3,4,9-tetrahydro-1H-carbazole-1-formate (0.034 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by silica gel column chromatography to obtain 6-chloro-4-fluoro-2,3,4,9-tetrahydro-1H-carbazole-1-carboxamide as a white solid (compound 7, 7.3 mg). LCMS $[M+H]^+$: 267. $^1$H-NMR (400 MHz, $CDCl_3$): δ 8.53 (s, 1H), 7.36-7.39 (m, 1H), 7.28-7.31 (m, 1H), 7.10-7.15 (m, 1H), 5.64-5.78 (m, 2H), 5.51 (td, *J* = 51.6, 4.1 Hz, 1H), 3.63-3.66 (m, 1H), 1.63-1.95 (m, 4H).

**Example 9:** Preparation of 8-chloro-1-methyl-2,3,4,5-tetrahydropyrido[3,2-b]indole-4-carboxamide **(compound 8),** (*R*)-8-chloro-1-methyl-2,3,4,5-tetrahydropyrido[3,2-b]indole-4-carboxamide **(compound 8a),** and (*S*)-8-chloro-1-methyl-2,3,4,5-tetrahydropyrido[3,2-b]indole-4-carboxamide (**compound 8b**)

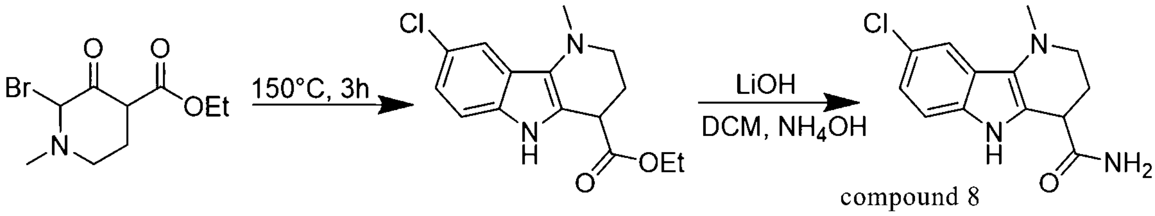

compound 8

Ethyl 2-bromo-1-methyl-3-oxopiperidine-4-carboxylate (21.1 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 M HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 8-chloro-1-methyl-2,3,4,5-tetrahydropyrido [3,2-b]indole-4-carboxylate (16.8 mg). 10 mg of the ethyl 8-chloro-1-methyl-2,3,4,5-tetrahydropyrido[3,2-b]indole-4-carboxylate (0.036 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, the pH was adjusted to 2, and then the mixture was extracted 3 times with 50 mL of dichloromethane. The organic phases were combined, dried, and concentrated, then ammonia water, HATU (0.036 mmol), and acetonitrile (5 mL) were added for a reaction at room temperature for 24 hours, and the mixture was separated and purified by silica gel column chromatography to obtain 8-chloro-1-methyl-2,3,4,5-tetrahydropyrido[3,2-b]indole-4-carboxamide as a white solid (compound 8, 6.8 mg), which was then separated by using a Chiralpak AD chiral column to obtain (*R*)-8-chloro-1-methyl-2,3,4,5-tetrahydropyrido[3,2-b]indole-4-carboxamide (compound 8a) and (S)-8-chloro-1-methyl-2,3,4,5-tetrahydropyrido[3,2-b]indole-4-carboxamide (compound 8b).LCMS $[M+H]^+$ for the compounds 8a and 8b: 264. $^1$H-NMR (400 MHz, DMSO-$d_6$): δ 10.90 (s, 1H), 7.59 (s, 1H), 7.33-7.37(m, 3H), 6.92 (d, *J* = 8.0 Hz, 1H), 3.60-3.63 (m, 1H), 2.96-3.03 (m, 2H), 2.79 (s, 3H), 1.92-2.10 (m, 2H).

**Example 10:** Preparation of 6-chloro-7-phenylamino-2,3,4,9-tetrahydro-1H-carbazole-1-carboxamide **(compound 9)**

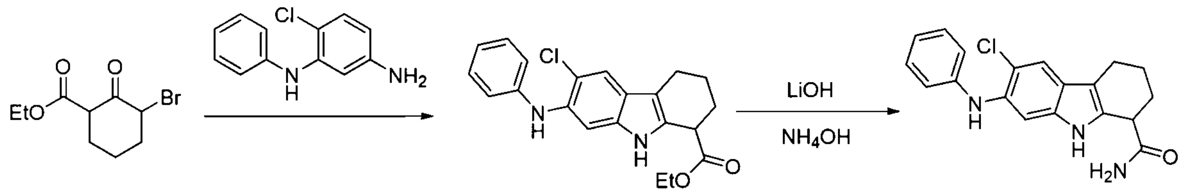


9-1

compound 9

Ethyl 3-bromo-2-oxocyclohexane-1-formate (1.3 g, 6.07 mmol), 6-chloro-1N-phenyl-1,3-diamine (0.5 g, 2.02 mmol), and ethylene glycol (1 mL) were heated to 110°C for a reaction for 2 hours, the reaction was quenched by adding water, and the mixture was extracted with ethyl acetate for liquid separation. The organic phase was desolventized under reduced pressure and subjected to column chromatography to obtain 0.22 g of crude product 9-1. The 9-1 (0.15 g, 0.406 mmol, 1.0 eq), 0.6 mL of a THF/MeOH/$H_2O$ (4:4:1) mixed solution (4 V), and LiOH (97.5 mg, 4.07 mmol, 10.0 eq) were weighed, stirred overnight at room temperature, and then concentrated under reduced pressure. Ethyl acetate (2 mL) and water (2 mL) were added and stirred for liquid separation. The organic phase was washed with a saturated salt solution, dried, and concentrated under reduced pressure. Then, ammonia water, HATU (0.44 mmol), and acetonitrile (5 mL) were added to the residue for a reaction at room temperature for 24 hours, and the mixture was separated and purified by silica gel column chromatography to obtain 6-chloro-7-phenylamino-2,3,4,9-tetrahydro-1H-carbazole-1-carboxamide as a white solid (compound 9, 6.9 mg). LCMS $[M+H]^+$: 340. $^1$H-NMR (400 MHz, $CD_3OD$): δ 7.38 (s, 1H), 7.16-7.23 (m, 3H), 6.94-6.96 (m, 2H), 6.78-6.82 (m, 1H), 3.64-3.72 (m, 1H), 2.73-2.77 (m, 2H), 1.68-2.10 (m, 4H).

**Example 11:** Preparation of 3-fluoro-2,3,4,9-tetrahydro-1H-carbazole-8-carboxamide **(compound 10)**

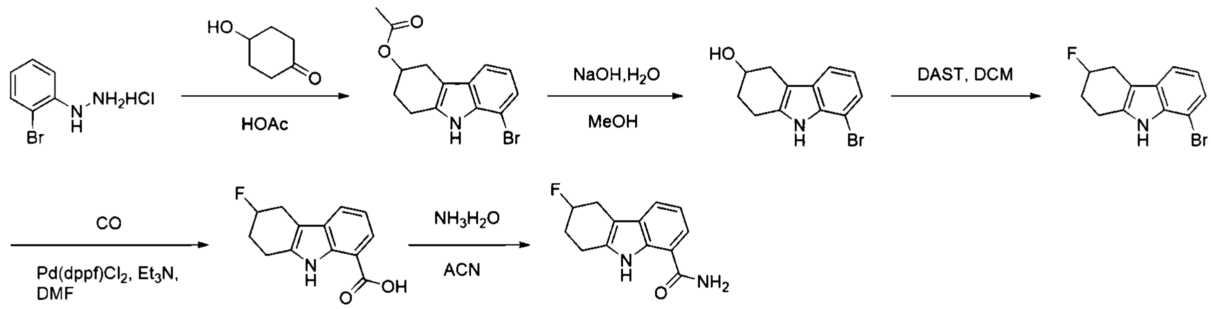


compound 10

(2-bromophenyl)hydrazine hydrochloride (7.83 g, 35.03 mmol), 4-hydroxycyclohexane-1-one (4 g, 35.03 mmol), and acetic acid (80 mL) were added into a reaction flask, subjected to nitrogen replacement, and allowed to react at 110°C for 2 hours. The mixture was concentrated, then diluted with dichloromethane, and extracted with a saturated sodium bicarbonate solution. The organic phase was washed with a saturated salt solution, concentrated, and separated and purified by silica gel column chromatography to obtain 8-bromo-2,3,4,9-tetrahydro-1H-carbazoe-3-ylacetate (7.04 g). The 8-bromo-2,3,4,9-tetrahydro-1H-carbazole-3-ylacetate (7.04 g), methanol (30 mL), and an aqueous solution of sodium hydroxide (30%, 10 mL) were weighed, added into a reaction flask, and stirred at 50°C for 1 hour. After completion of the reaction monitored by thin layer chromatography (TLC), rotary evaporation was performed under reduced pressure to remove the methanol, ethyl acetate was added for extraction three times, and the organic phase was concentrated. Purification was performed by silica gel column chromatography to obtain 8-bromo-2,3,4,9-tetrahydro-1H-carbazole-3-ol (5.09 g). The 8-bromo-2,3,4,9-tetrahydro-1H-carbazole-3-ol (5.09 g) and dichloromethane (50 mL) were weighed, added into a reaction flask, stirred until dissolved and clarified, subjected to nitrogen replacement, and cooled to -10°C, and then DAST (1.21 g) was added for a reaction at -10°C for 1.5 hours. The reaction solution was added dropwise into an ice-cold saturated sodium bicarbonate solution for quenching, and then extracted with dichloromethane. The organic phase was concentrated and purified by silica gel column chromatography to obtain 8-bromo-3-fluoro-2,3,4,9-tetrahydro-1H-carbazole (758 mg). The 8-bromo-3-fluoro-2,3,4,9-tetrahydro-1H-carbazole (758 mg), DMF (10 mL), Pd(dppf)$Cl_2$ (414 mg, 0.566 mmol), and triethylamine (859 mg, 8.49 mmol) were weighed, added into a reaction flask, subjected to CO replacement, and allowed to react at 80°C for 16 hours. After completion of the reaction monitored by TLC, the reaction solution was cooled to room temperature, diluted with ethyl acetate (30 mL), and washed three times with a saturated salt solution. The organic phase was concentrated and purified by silica gel column chromatography to obtain 3-fluoro-2,3,4,9-tetrahydro-1H-carbazole-8-formic acid (78 mg). The 3-fluoro-2,3,4,9-tetrahydro-1H-carbazole-8-formic acid (78 mg), ammonia water (24 mg), TCFH (140 mg), NMI (55 mg), and acetonitrile (5 mL) were weighed, added into a reaction flask, subjected to nitrogen replacement, and stirred at room temperature for 2 hours. The reaction solution was extracted with ethyl acetate, dried over anhydrous sodium sulfate, and purified by preparative liquid chromatography to obtain 0.915 mg of 3-fluoro-2,3,4,9-tetrahydro-1H-carbazole-8-carboxamide (compound 10).LCMS $[M+H]^+$: 233. $^1H$-NMR (400 MHz, $CDCl_3$): δ 7.97 (s, 1H), 7.32-7.41 (m, 1H), 7.24-7.30 (m, 1H), 6.96-7.00 (m, 1H), 5.76-5.81 (m, 2H), 5.09-5.26 (m, 1H), 2.95-3.16 (m, 3H), 2.81-2.88 (m, 1H), 2.26-2.34 (m, 1H), 2.06-2.21(m, 1H).

**Example 12:** Synthesis of 3,3-difluoro-2,3,4,9-tetrahydro-1H-carbazole-8-carboxamide (compound 11)

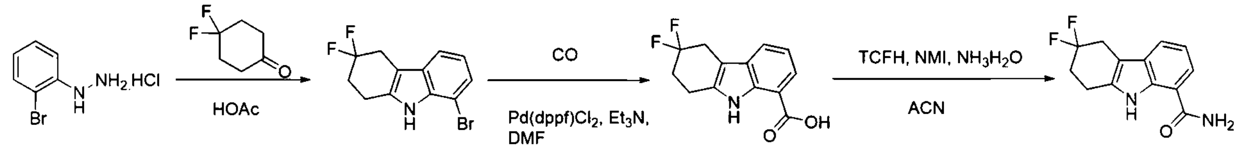


compound 11

(2-bromophenyl)hydrazine hydrochloride (1 g, 4.474 mmol), 4,4-difluorocyclohexane-1-one (0.6 g, 4.474 mmol), and acetic acid (10 mL) were added into a reaction flask, subjected to nitrogen replacement, and stirred at 110°C for 2 hours. The reaction solution was concentrated, diluted with dichloromethane, and then extracted with a saturated aqueous solution of sodium bicarbonate. The organic phase was washed with a saturated salt solution, concentrated, and purified by silica gel column chromatography to obtain 8-bromo-3,3-difluoro-2,3,4,9-tetrahydro-1H-carbazole (991 mg). The 8-bromo-3,3-difluoro-2,3,4,9-tetrahydro-1H-carbazole (891 mg), DMF (10 mL), $Pd(dppf)Cl_2$ (456 mg, 0.623 mmol), and triethylamine (946 mg, 9.34 mmol) were weighed, added into a reaction flask, subjected to CO replacement, and allowed to react at 100°C for 4 hours. After completion of the reaction monitored by TLC, the reaction solution was cooled to room temperature, diluted with ethyl acetate (30 mL), and washed three times with a saturated salt solution. The organic phase was concentrated and purified by silica gel column chromatography to obtain 3,3-difluoro-2,3,4,9-tetrahydro-1H-carbazole-8-formic acid (57 mg). The 3,3-difluoro-2,3,4,9-tetrahydro-1H-carbazole-8-formic acid (57 mg), ammonia water (16 mg), TCFH (96 mg), NMI (38 mg), and acetonitrile (5 mL) were weighed, added into a reaction flask, subjected to nitrogen replacement, and stirred at room temperature for 2 hours. The reaction solution was extracted with ethyl acetate, dried over anhydrous sodium sulfate, and purified by preparative liquid chromatography to obtain 3,3-difluoro-2,3,4,9-tetrahydro-1H-carbazole-8-carboxamide (10 mg). LCMS $[M+H]^+$: 251. $^1H$ NMR (400 MHz, DMSO-$d_6$) δ 11.31 (s, 1H), 8.09 (br. s., 1H), 7.53 (br. s., 1H), 7.38-7.40 (m, 1H), 7.28-7.29 (m, 1H), 6.91-7.00 (m, 1H), 3.51 (t, *J*=14.4 Hz, 2H), 2.89 (t, *J*=6.6 Hz, 2H), 2.23 (tt, *J*=13.9, 6.7 Hz, 2H).

**Example 13:** Synthesis of 5-chloro-9,12-diazatetracyclo(10.2.1.$0^{2,10}$.$0^{3,8}$)pentadeca-2(10),3,5,7-tetraene-11-carboxamide **(compound 12)**

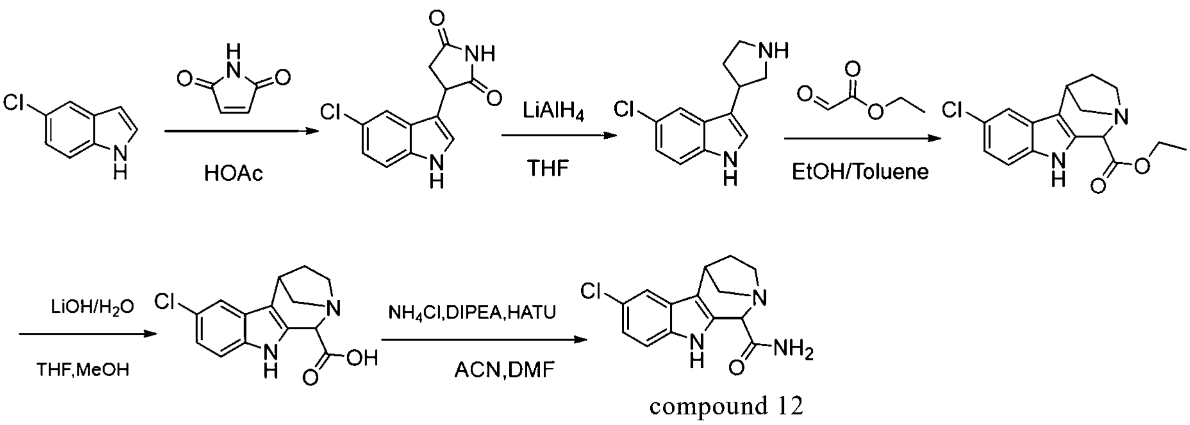


compound 12

5-chloro-1H-indole (25 g, 164.9 mmol), 1H-pyrrole-2,5-dione (24 g, 247.35 mmol), and acetic acid (150 mL) were

added into a reaction flask, subjected to nitrogen replacement, and allowed to react at 120°C for 48 hours. The reaction solution was concentrated and purified by silica gel column chromatography to obtain 3-(5-chloro-1H-indol-3-yl)pyrrolidine-2,5-dione (6 g). Lithium aluminum hydride (4.554 g, 120 mmol) and tetrahydrofuran (120 mL) were weighed, added into a reaction flask, subjected to nitrogen replacement, and cooled to 0°C. The 3-(5-chloro-1H-indol-3-yl)pyrrolidine-2,5-dione (6 g, 24 mmol) was added in portions. After stirring for 10 minutes, the mixture was heated to reflux, allowed to react for 16 hours, and then cooled to below 0°C. Water and an aqueous solution of NaOH (10%) were added in sequence to quench the reaction. The mixture was stirred at room temperature for 4 hours, filtered, and concentrated under reduced pressure to obtain a crude product, which was then purified by silica gel column chromatography to obtain 5-chloro-3-(pyrrolidin-3-yl)-1H-indole (4.6 g).The 5-chloro-3-(pyrrolidin-3-yl)-1H-indole (4.6 g, 20.84 mmol), ethanol (46 mL), and a solution of ethyl glyoxylate in toluene (50%) (5.1 g, 25 mmol) were added into a microwave reaction tube, subjected to nitrogen replacement, and allowed to carry out a microwave reaction at 95°C for 30 minutes. The reaction solution was concentrated and purified by silica gel column chromatography to obtain ethyl 5-chloro-9,12-diazatetracyclo(10.2.1.$0^{2,10}$.$0^{3,8}$)pentadeca-2(10),3,5,7-tetraene-11-formate (500 mg). The ethyl 5-chloro-9,12-diazatetracyclo(10.2.1.$0^{2,10}$.$0^{3,8}$)pentadeca-2(10),3,5,7-tetraene-11-formate (450 mg, 1.477 mmol), tetrahydrofuran (10 mL), methanol (10 mL), lithium hydroxide (353 mg), and water (5 mL) were added into a reaction flask for a reaction at room temperature for 12 hours. After completion of the reaction monitored by TLC, the reaction solution was concentrated, the pH was adjusted to 4-5 with 3M hydrochloric acid, and the mixture was extracted with ethyl acetate. The organic phase was concentrated and purified by silica gel column chromatography to obtain 5-chloro-9,12-diazatetracyclo(10.2.1.$0^{2,10}$.$0^{3,8}$)pentadeca-2(10),3,5,7-tetraene-11-formic acid (56 mg). The 5-chloro-9,12-diazatetracyclo(10.2.1.$0^{2,10}$.$0^{3,8}$)pentadeca-2(10),3,5,7-tetraene-11-formic acid (56 mg, 0.202 mmol), acetonitrile (2 mL), DMF (1 mL), $NH_4Cl$ (21.6 mg, 0.404 mmol), and DIPEA (78.3 mg, 0.606 mmol) were added into a reaction flask, subjected to nitrogen replacement, and cooled to 0°C. Then, HATU (153.6 mg, 0.404 mmol) was added and stirred for 4 hours. The reaction solution was concentrated and purified by silica gel column chromatography to obtain a crude product, which was then purified by preparative high performance liquid chromatography (HPLC) to obtain 5-chloro-9,12-diazatetracyclo(10.2.1.$0^{2,10}$.$0^{3,8}$)pentadeca-2(10),3,5,7-tetraene-11-carboxamide (1.6 mg). LCMS $[M+H]^+$: 275. $^1$H-NMR (400 MHz, $CD_3OD$): $\delta$ 8.39 (br, 1H), 7.48-7.50 (m, 1H), 7.26-7.32 (m, 1H), 7.03-7.06 (m, 1H), 4.63 (s, 1H), 3.49-3.52 (m, 2H), 3.34-3.36 (m, 1H), 2.98-3.08 (m, 2H), 2.17-2.24 (m, 1H), 2.03-2.09 (m, 1H).

**Example 14:** Changes in an mRNA methylation level of drug-induced keratinocytes

1*$10^8$ HaCat cells were seeded in culture dishes, into which 50 nM drugs (including sorafenib, capecitabine, docetaxel, and osimertinib) were added, respectively. The cells were cultured in an incubator containing 5% $CO_2$ at 37°C for 24 hours and washed with PBS after culture media were removed. Centrifugation was performed, the supernatant was discarded, and then, 400 μL of an RLT cell lysis buffer (QIAGEN, Cat.#79216) was added. A portion of samples were selected, into which 400 μL of 70% ethanol was added. The mixture was evenly stirred and then transferred to an RNeasy mini column, and RNA was extracted using an RNeasy mini kit (QIAGEN, Cat.#74104). 2 μL of an RNA sample was selected and dissolved in 98 μL of a 10 mM Tris-HCl buffer solution, and the absorbance at 260 nm was measured using a Nanodrop (Thermo Fisher Scientific) to determine the RNA concentration.

40 μg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit™ dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

Results are shown in FIG. 1. Compared with a blank control group, the m6A methylation levels of HaCat cells are increased by 5.05 times, 6.01 times, 3.97 times, and 5.13 times after the addition of the sorafenib, the capecitabine, the docetaxel, and the osimertinib, respectively. It is indicated that antitumor drugs (including the sorafenib, the capecitabine, the docetaxel, and the osimertinib) can induce the abnormal m6A methylation of mRNA of human keratinocytes. It is further found through bioinformatics analysis that the m6A methylation level of NAP1L2 has a significant difference, mainly showing in the m6A methylation of a UGAGGACUCA fragment.

**Example 15:** Changes in an mRNA methylation level in drug-induced mouse models with adverse skin reactions

After adaptive feeding for 7 days, male ICR mice (5-6 weeks old, with a body weight of approximately 35 g) were randomly divided into groups, with 6 mice in each group, including a blank control group, a sorafenib group, a capecitabine group, a docetaxel group, and an osimertinib group. The groups were respectively administered corresponding modeling drugs by gavage (sorafenib at 100 mg/kg, capecitabine at 200 mg/kg, docetaxel at 25 mg/kg, and osimertinib at 10 mg/kg) once daily. After continuous administration for 30 days, the mice were sacrificed, and skin tissues of hind limb toes of the mice were collected. A small amount of a tissue sample was selected, placed in a mortar containing liquid nitrogen, and ground into powder. A single-phase lysis buffer was added. After being placed at room temperature for 5 minutes, the sample was centrifuged (12,000 rpm) for 5 minutes. 1 mL of the supernatant was collected, 200 μL of chloroform was added, and the mixture was evenly mixed by shaking, placed at room temperature for 15 minutes, and centrifuged (12,000 rpm, 4°C) for 15 minutes. An aqueous phase on an upper layer was sucked, added into an equal volume of isopropanol, placed at -20°C for 1 hour, and then centrifuged (12,000 rpm, 4°C) to discard the supernatant. 400 μL of 70% ethanol was added to a precipitate, the mixture was evenly stirred and then transferred to an RNeasy mini column, and RNA was extracted using an RNeasy mini kit (QIAGEN, Cat.#74104). 2 μL of an RNA sample was selected and dissolved in 98 μL of a 10 mM Tris-HCl buffer solution, and the absorbance at 260 nm was measured using a Nanodrop (Thermo Fisher Scientific) to determine the RNA concentration.

40 μg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit™ dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

Results are shown in FIG. 2. Compared with a positive control group, the m6A methylation levels of skin tissues of hind limb toes of the mice in the drug modeling groups are significantly increased. The m6A methylation level of NAP1L2 in the drug addition groups has a significant difference from a blank control group.

**Example 16:** Regulation of an mRNA expression level of NAP1L2 by m6A

$1*10^8$ HaCat cells were seeded in culture dishes, into which a recombinant protein of m6A methylase METTL3 (Abcam, Cat.#ab271611) and a recombinant protein of m6A demethylase FTO (Abcam, Cat.#ab271525) were added, or METTL3 siRNA (sh-METTL3, GeneChem) and FTO siRNA (sh-FTO, GeneChem) were transfected, respectively. The cells were placed in an incubator containing 5% carbon dioxide for culture at 37°C for 24 hours, washed with PBS, and then collected by high-speed centrifugation. Total RNA was extracted using a Trizol reagent (Sigma Aldrich). 1 μg of RNA was reverse-transcribed into cDNA using a cDNA reverse transcription kit (Transgene Biotech, Cat.#AT311-03). 1.25 μL of a primer (Beyotime, Cat.#QH18721S), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression amount of NAP1L2.

Results are shown in FIG. 3. When the m6A methylase METTL3 is added to the HaCat keratinocytes or a function (sh-FTO) of the m6A demethylase FTO is inhibited, the mRNA expression amount of NAP1L2 is significantly increased; and when the m6A demethylase FTO is added to the HaCat keratinocytes or a function (sh-METTL3) of the m6A methylase METTL3 is inhibited, the mRNA expression amount of NAP1L2 is significantly decreased. It is indicated that m6A methylation can regulate the mRNA expression amount of NAP1L2.

**Example 17:** Regulation of the expression of matrix metalloproteins in human HaCat keratinocytes by NAP1L2

$1*10^8$ HaCat cells were seeded in culture dishes, into which a human recombinant NAP1L2 protein (Abcam, Cat.#ab117213), sorafenib, capecitabine, docetaxel, and osimertinib were added, or NAP1L2 siRNA (sh-NAP1L2, GeneChem) was transfected, respectively. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the supernatant, and the expression of matrix metalloproteins was measured by Western blot.

Total RNA was extracted from the above collected cells using a Trizol reagent (Sigma Aldrich). 1 μg of RNA was reverse-transcribed into cDNA using a cDNA reverse transcription kit (Transgene Biotech, Cat.#AT311-03). 1.25 μL of a primer (SinoBiological, Cat.#HP100168&HP100367), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression amounts of the matrix metalloproteins.

Results are shown in FIG. 4. After the recombinant NAP1L2 protein is added to the HaCat keratinocytes, the mRNA and protein expression amounts of matrix metalloproteinases MMP2 and MMP9 are significantly increased; and when NAP1L2 siRNA is added to the HaCat keratinocytes to inhibit the expression of NAP1L2, the mRNA and protein expression amounts of the matrix metalloproteinases MMP2 and MMP9 are significantly decreased. Numerous studies have confirmed that abnormal expression of matrix metalloproteinases is closely associated with various skin-related diseases (Kumper M. et al., Am. J. Physiol. Cell. Physiol. 2022, 323(4): 1290-1303).

**Example 18:** Regulation of the expression of human keratinocyte differentiation markers by m6A

$1*10^8$ HaCat cells were seeded in culture dishes, into which a recombinant HBEGF protein (Thermo Fisher Scientific, Cat.#100-47-1mg) was added. The cells were placed in an incubator containing 5% carbon dioxide for culture at 37°C for 24 hours, into which an HBEGF antibody (Invitrogen, Cat.#406316) was added, or METTL3 siRNA (sh-METTL3, GeneChem) and NAP1L2 siRNA (sh-NAP1L2, GeneChem) were transfected. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the supernatant, and the protein expression amounts of keratinocyte differentiation markers KRT1, KRT10, Loricrin, and Involucrin were measured by Western blot.

Total RNA was extracted using a Trizol reagent (Sigma Aldrich). 1 μg of RNA was reverse-transcribed into cDNA using a cDNA reverse transcription kit (Transgene Biotech, Cat.#AT311-03). 1.25 μL of a primer (with a primer sequence shown in Table 2), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression amounts of the keratinocyte differentiation markers KRT1, KRT10, Loricrin, and Involucrin.

Results are shown in FIG. 5. After the recombinant HBEGF protein is added to the HaCat keratinocytes, the mRNA and protein expression amounts of the keratinocyte differentiation markers KRT1, KRT10, Loricrin, and Involucrin are significantly increased, indicating that the HaCat keratinocytes are in a highly differentiated state; however, when the HBEGF antibody or METTL3 or NAP1L2 siRNA is added to inhibit an m6A methylation function in the cells, the mRNA and protein expression amounts of the keratinocyte differentiation markers KRT1, KRT10, Loricrin, and Involucrin are significantly decreased. It is indicated that m6A can regulate the expression of the keratinocyte differentiation markers, thereby regulating the differentiation of the keratinocytes.

**Example 19:** Impacts of compounds on an m6A methylation level of THP-1 cells

$1*10^8$ THP-1 cells were seeded in culture dishes, into which 400 nM compound 1, 4 nM and 400 nM compounds 2b, 5a, and 8b, 4 nM and 4 μM nicotinamide, and 5 μM m6A methylase inhibitor UZH2 were added, respectively. The cells were cultured in an incubator containing 5% $CO_2$ at 37°C for 24 hours. After culture media were discarded, the cells were washed with PBS and centrifuged to discard the supernatant. 400 μL of an RLT cell lysis buffer was added, and the cells were stored at -80°C. A portion of samples were selected, into which 400 μL of 70% ethanol was added and evenly stirred. 700 μL of a sample was transferred to an RNeasy centrifugation column and centrifuged for 15 seconds (8,000 g, 25°C). Then, 700 μL of an RW1 buffer solution was added and centrifuged for 15 seconds (8,000 g, 25°C), followed by the addition of 500 μL of an RPE buffer solution and centrifugation for 16 seconds (8,000 g, 25°C). The above steps were repeated twice, followed by centrifugation for 2 minutes to completely remove the eluent. 30 μL of nuclease-free water was added to a chromatographic column, and the cells were incubated for 5 minutes and then centrifuged for 2 minutes (12,000 g, 25°C). The steps were repeated 3 times. 2 μL of an RNA sample was selected and dissolved in 98 μL of a 10 mM Tris buffer solution, and the absorbance at 260 nm was measured using a Nanodrop to determine the RNA concentration.

50 μg of total RNA was selected and dissolved in 100 μL of nuclease-free water. Oligo (dT) magnetic beads were resuspended, 50 μL of the magnetic beads were transferred to a 1.5 mL test tube, 500 μL of a binding buffer solution was added, the mixture was allowed to stand, and the supernatant was removed. 100 μL of an RNA sample was added to 200 μL of a magnetic bead suspension for mixing, the mixture was stirred and incubated in a Thermomixer at 25°C for 5 minutes, the supernatant was discarded, and then 200 μL of a washing buffer solution was added. The steps were repeated 2 times, and then centrifugation was performed for 10 seconds (2,000 g, 25°C). 50 μL of an elution buffer solution was added and evenly mixed, the mixture was heated to 75°C, the supernatant was transferred to a new 1.5 mL test tube

and purified using an RNA Clean & Concentrator kit, and the RNA concentration was determined.

20 μL (approximately 200 ng) of an mRNA sample and 20 μL of a Nuclease P1 digestion premix (including 0.5 μL of 2 unit/μL Nuclease P1, 0.4 μL of 5 M NaCl, 2 μL of 0.1 M $ZnCl_2$, and 17.1 μL of PCR-grade water) were added into each test tube, and the mixture was stirred and incubated in a Thermomixer at 37°C for 2 hours. 2 μL of a 2 M $NH_4HCO_3$ solution and 1 unit of alkaline phosphatase were added, and the mixture was stirred and incubated in a Thermomixer at 37°C for 2 hours. 1 μL of a 1.2 M HCl neutralization solution was added, and centrifugation was performed for 30 minutes (16,000 g, 4°C). 20 μL of the supernatant was collected, an m6A methylation fragment was analyzed by LC-MS, and the m6A inhibition rate was calculated.

m6A inhibition rate (%)=(measured ion peak area-blank control)/(positive ion peak area-blank control)*100.

Results are shown in FIG. 6. The compounds 2b, 5a, and 8b can significantly inhibit the m6A methylation level of mRNA of the cells in a concentration-dependent manner. At a concentration of 400 nM, the compound 2b exhibits m6A inhibition activity comparable to that of the m6A methylase inhibitor UZH2 at a concentration of 5 μM. However, the nicotinamide exhibits no inhibitory effect on the m6A methylation level of mRNA of the cells at either a low concentration (4 nM) or a high concentration (4 μM).

**Example 20:** Impacts of compounds on an m6A methylation level of human keratinocytes

HaCat cells were cultured in a DMEM culture medium containing 10% fetal bovine serum, 100 U/mL penicillin, and 100 μg/mL streptomycin. Culture dishes were placed in an incubator containing 5% $CO_2$ for culture at 37°C for 24 hours. An equal volume of DMSO was added in control group 1, 50 nM sorafenib, capecitabine, docetaxel, and osimertinib were respectively added in control groups A, B, C, and D, and 400 nM test compounds or nicotinamide were respectively added in sample groups A1-17, B1-17, C1-17, and D1-17 after the addition of 50 nM corresponding compounds. The cells were continuously cultured for 24 hours, washed with PBS, and then collected by high-speed centrifugation. Total RNA was extracted using a Trizol reagent (Sigma Aldrich), and RNA m6A methylation of a sample was quantitatively detected using an EpiQuik m6A RNA methylation quantification kit. The concentrations of positive control samples for a standard curve were 0.01 ng/μl, 0.02 ng/μl, 0.05 ng/μl, 0.1 ng/μl, 0.2 ng/μl, and 0.5 ng/μl. The absorbance at 450 nm was read using a microplate reader (Tecan GENios). A quantitative calculation formula for m6A is as follows:

m6A (ng)=(absorbance of sample well–absorbance of background well)/slope of standard curve

m6A (%)=m6A (ng)/RNA amount of sample (ng)*100%.

Results are shown in Table 1. The treatment with the sorafenib, the capecitabine, the docetaxel, and the osimertinib can significantly increase the m6A methylation level of human HaCat keratinocytes (as shown in Table 1). The compounds 2, 2b, 5a, and 8b can effectively inhibit an abnormal increase in the RNA m6A level of human keratinocytes caused by chemotherapeutic drugs and multikinase inhibitors.

Table 1. Fold changes in the m6A methylation level relative to control group 1. A specific calculation method is: fold change=measured m6A amount/measured m6A amount of control 1.

| Group | Drug/compound | m6A fold change | Group | Drug/compound | m6A fold change |
|---|---|---|---|---|---|
| A | Sorafenib | 4.5 | B | Capecitabine | 6.7 |
| A1 | Compound 1 | 2.9 | B1 | Compound 1 | 3.0 |
| A2 | Compound 2 | 2.4 | B2 | Compound 2 | 2.3 |
| A3 | Compound 2a | 3.4 | B3 | Compound 2a | 3.5 |
| A4 | compound 2b | 1.4 | B4 | compound 2b | 1.1 |
| A5 | Compound 3a | 3.3 | B5 | Compound 3a | 2.8 |
| A6 | compound 3b | 3.3 | B6 | compound 3b | 2.9 |
| A7 | Compound 4 | 3.6 | B7 | Compound 4 | 3.2 |
| A8 | Compound 5 | 2.4 | B8 | Compound 5 | 3.0 |

(continued)

| Group | Drug/compound | m6A fold change | Group | Drug/compound | m6A fold change |
|---|---|---|---|---|---|
| A9 | Compound 5a | 1.0 | B9 | Compound 5a | 1.2 |
| A10 | compound 5b | 3.8 | B10 | compound 5b | 4.8 |
| A11 | Compound 6 | 3.4 | B11 | Compound 6 | 3.2 |
| A12 | Compound 7 | 3.3 | B12 | Compound 7 | 3.9 |
| A13 | Compound 8 | 2.5 | B13 | Compound 8 | 1.7 |
| A14 | Compound 8a | 4.0 | B14 | Compound 8a | 2.4 |
| A15 | compound 8b | 1.0 | B15 | compound 8b | 1.0 |
| A16 | Compound 9 | 3.5 | B16 | Compound 9 | 3.9 |
| A17 | Nicotinamide | 4.7 | B17 | Nicotinamide | 4.5 |
| C | Docetaxel | 5.6 | D | Osimertinib | 4.8 |
| C1 | Compound 1 | 3.2 | D1 | Compound 1 | 3.4 |
| C2 | Compound 2 | 3.3 | D2 | Compound 2 | 2.2 |
| C3 | Compound 2a | 5.6 | D3 | Compound 2a | 3.2 |
| C4 | compound 2b | 1.0 | D4 | compound 2b | 1.2 |
| C5 | Compound 3a | 4.2 | D5 | Compound 3a | 2.9 |
| C6 | compound 3b | 3.2 | D6 | compound 3b | 3.1 |
| C7 | Compound 4 | 5.1 | D7 | Compound 4 | 4.2 |
| C8 | Compound 5 | 2.9 | D8 | Compound 5 | 3.1 |
| C9 | Compound 5a | 1.0 | D9 | Compound 5a | 1.2 |
| C10 | compound 5b | 4.8 | D10 | compound 5b | 5.0 |
| C11 | Compound 6 | 5.1 | D11 | Compound 6 | 3.5 |
| C12 | Compound 7 | 3.4 | D12 | Compound 7 | 2.9 |
| C13 | Compound 8 | 3.0 | D13 | Compound 8 | 2.1 |
| C14 | Compound 8a | 4.8 | D14 | Compound 8a | 3.2 |
| C15 | compound 8b | 1.2 | D15 | compound 8b | 1.0 |
| C16 | Compound 9 | 3.9 | D16 | Compound 9 | 4.0 |
| C17 | Nicotinamide | 5.4 | D17 | Nicotinamide | 5.0 |

**Example 21:** Inhibitory effects of compounds on the differentiation of HaCaT keratinocytes

Human HaCaT keratinocytes were cultured in a DMEM culture medium containing 10% fetal bovine serum, 100 U/mL penicillin, and 100 μg/mL streptomycin. The cells were seeded into a 96-well plate at a density of $2*10^6$ cells/$cm^2$ and placed in an incubator containing 5% carbon dioxide for culture at 37°C for 24 hours. An equal volume of DMSO was added into control well 1, a 2.5 ng/mL human recombinant HBEGF protein (Abcam, ab205523) was added into control well 2, and a 2.5 ng/mL human recombinant HBEGF protein and corresponding concentrations of test compounds were added into sample wells. The cells were continuously cultured for 24 hours, washed with PBS, and then collected by high-speed centrifugation. Total RNA was extracted using a Trizol reagent (Sigma Aldrich). 1 μg of RNA was reverse-transcribed into cDNA using a cDNA reverse transcription kit (Transgene Biotech, AT311-03). 1.25 μL of a primer (with a primer sequence shown in Table 2), 10 μL of an iTag Universal SYBR Green supermix (Bio-Rad, 172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression amounts of keratinocyte differentiation markers KRT1, KRT10, Loricrin, and Involucrin.

Table 2. Sequence information of some primers

| | | |
|---|---|---|
| KRT1 | Forward primer | 5'-GTCAAGTCCTCTGGTGGCAG-3' |
| | Reverse primer | 5'-AAGGCTGGGACAAATCGACC-3' |
| KRT10 | Forward primer | 5'-CCCGGGTGTTGATCTGACTC-3' |
| | Reverse primer | 5'-CCAGGCTTCAGCATCTTTGC-3' |
| Loricrin | Forward primer | 5'-TCATGATGCTACCCGAGGTTTG-3' |
| | Reverse primer | 5'-CAGAACTAGATGCAGCCGGAGA-3' |
| Involucrin | Forward primer | 5'-TCGCTCCTCAAGACTGTTCCTCC-3' |
| | Reverse primer | 5'-CAGGCAGTCCCTTTACAGCA-3' |

As shown in FIG. 7, after the human recombinant HBEGF protein is added for induction, the mRNA expression amounts of the HaCaT differentiation markers KRT1, KRT10, Loricrin, and Involucrin are significantly increased. However, after some compounds (such as compounds 2, 2b, 3a, 3b, 5a, and 8b, etc.) are added, the mRNA expression amounts of the HaCaT differentiation markers KRT1, KRT10, Loricrin, and Involucrin are significantly inhibited.

**Example 22:** Inhibitory effects of compounds on the differentiation of keratinocytes in rat models with hand-foot skin reaction

After adaptive feeding for one week, SD rats (with a body weight of approximately 200 g) were randomly divided into groups according to the body weight, with 12 rats in each group. Modeling drugs (including sorafenib, erlotinib, afatinib, and osimertinib) were respectively dissolved in a solution containing 5% of DMSO, 45% of PEG400, and 50% of $H_2O$, and the modeling drugs were diluted to required concentrations and then administered once daily by gavage at doses shown in Table 3. After administration by gavage for 1 hour, 0.05 g of ointments containing different mass proportions of test compounds were evenly applied to left paws of the rats, a blank matrix ointment was applied to right paws of the rats to serve as a self-control, and no ointment was applied to the rats in a blank control group. After drug application, four limbs were continuously immobilized for 2 hours, then wiped with clean water to remove the residual drugs, removed from immobilization, and restored to free movement. The modeling drugs, the blank matrix ointment, and the ointments containing the test compounds were all administered once daily. After continuous administration for 30 days, the rats were euthanized, and paw plantar skin tissues were collected, fixed in 10% neutral formalin, cut into 5 μm sections, dehydrated, and then embedded in paraffin. The ointments for the test compounds were prepared by mixing the compounds, white beeswax, white petrolatum, and light liquid paraffin in specific ratios (weight ratios of 3 ointments were respectively 1:18:58:23, 3:18:57:22, and 10:20:40:30). The blank matrix ointment was prepared by mixing white beeswax, white petrolatum, and light liquid paraffin in a specific ratio (weight ratio of 18:60:22).

Tissue staining: After dewaxing and rehydration, the paw plantar skin tissue sections of the above rats were stained in a hematoxylin solution for several minutes, cleaned, and placed in 1% acid alcohol for soaking until the sections faded to light blue-red. After cleaning with running water for 5 minutes, the sections were stained with eosin for 2-3 minutes, cleaned to remove excess dyes, dehydrated, then permeabilized by adding xylene for several minutes, and sealed and fixed with a neutral resin. The stratum corneum was observed under an optical microscope, and the thickness of the epidermal stratum corneum was measured using Dmetrix software.

Immunohistochemical staining: After dewaxing and hydration, the paw plantar skin tissue sections of the above rats were incubated with 3% $H_2O_2$ at room temperature for 30 minutes and blocked with 10% goat serum for 30 minutes after antigen retrieval. A KRT1 antibody (Abcam, ab93652) and a Loricrin antibody (Abcam, ab183646) were added dropwise, the sections were incubated overnight at 4°C, and an HRP secondary antibody (ZSGB-BIO, PV-6001) and a DAB kit (ZSG-BIO, ZL19017) were added for color development. The sections were re-stained with hematoxylin, cleaned, and sealed and fixed with a neutral resin, and the expression amounts of KRT1 and Loricrin were observed under an optical microscope.

Criteria for determining successful modeling of the above rat models are as follows: (i) symptoms, such as erythema, swelling, desquamation, ulcers, or blisters, occur at paw sites; and/or (ii) the stratum corneum thickness in tissue staining is significantly higher than that of normal rats; and/or (iii) markers, such as KRT1, KRT5, and Loricrin, are significant increased. An incidence rate calculation method involves a proportion of animals in each group meeting the above criteria for determining successful modeling, that is, incidence rate = (number of successfully modeled rats/total number of rats in the group)*100%.

Pathological scoring criteria for tissue staining of the above rats are as follows: no blisters, recorded as 0 point; the number of blisters being 1-3, recorded as 1 point; the number of blisters being 4-6, recorded as 2 points; the number of

blisters being 7-9, recorded as 3 points; and the number of blisters being more than 10, recorded as 4 points; an inflammation area accounting for less than 10% of a total section area, recorded as 0 point; the inflammation area accounting for 10-25% of the total section area, recorded as 1 point; the inflammation area accounting for 25-50% of the total section area, recorded as 2 points; the inflammation area accounting for 50-75% of the total section area, recorded as 3 points; and the inflammation area accounting for more than 75% of the total section area, recorded as 4 points; an congestion area accounting for less than 10% of a total section area, recorded as 0 point; the congestion area accounting for 10-25% of the total section area, recorded as 1 point; the congestion area accounting for 25-50% of the total section area, recorded as 2 points; the congestion area accounting for 50-75% of the total section area, recorded as 3 points; and the congestion area accounting for more than 75% of the total section area, recorded as 4 points. Scoring was performed using a double-blind scoring system. After completion of statistics, data for each group was presented in the form of mean + SEM (N=12).

Table 3. Doses of drugs used in rat models and experimental results

| **Serial number** | **Modeling drug** | **Dose** | **Compound and its mass proportion in an ointment** | | **Incidence rate** | |
|---|---|---|---|---|---|---|
| | | | Left paw | Right paw | 左侧足爪 | 右侧足爪 |
| 1 | Sorafenib | 100 mg/kg | Compound 2, 1% | None | 3/12 (25%) | 8/12 (67%) |
| 2 | Erlotinib | 70 mg/kg | Compound 2, 1% | None | 3/12 (25%) | 10/12 (83%) |
| 3 | Afatinib | 50 mg/kg | Compound 2, 1% | None | 2/12 (17%) | 7/12 (58%) |
| 4 | Osimertinib | 60 mg/kg | Compound 2, 1% | None | 2/12 (17%) | 9/12 (75%) |
| 5 | Sorafenib | 100 mg/kg | Compound 2b, 1% | None | 2/12 (17%) | 9/12 (75%) |
| 6 | Erlotinib | 70 mg/kg | Compound 2b, 1% | None | 3/12 (25%) | 10/12 (83%) |
| 7 | Afatinib | 50 mg/kg | Compound 2b, 1% | None | 3/12 (25%) | 9/12 (75%) |
| 8 | Osimertinib | 60 mg/kg | Compound 2b, 1% | None | 2/12 (17%) | 8/12 (67%) |
| 9 | Sorafenib | 100 mg/kg | Compound 5a, 1% | None | 1/12 (8%) | 10/12 (83%) |
| 10 | Erlotinib | 70 mg/kg | Compound 5a, 1% | None | 2/12 (17%) | 9/12 (75%) |
| 11 | Afatinib | 50 mg/kg | Compound 5a, 1% | None | 1/12 (8%) | 9/12 (75%) |
| 12 | Osimertinib | 60 mg/kg | Compound 5a, 1% | None | 2/12 (17%) | 9/12 (75%) |
| 13 | Sorafenib | 100 mg/kg | Compound 8b, 1% | None | 1/12 (8%) | 8/12 (67%) |
| 14 | Erlotinib | 70 mg/kg | Compound 8b, 1% | None | 2/12 (17%) | 10/12 (83%) |
| 15 | Afatinib | 50 mg/kg | Compound 8b, 1% | None | 1/12 (8%) | 9/12 (75%) |
| 16 | Osimertinib | 60 mg/kg | Compound 8b, 1% | None | 1/12 (8%) | 9/12 (75%) |
| 17 | Sorafenib | 100 mg/kg | Compound 2, 3% | None | 1/12 (8%) | 5/12 (42%) |
| 18 | Erlotinib | 70 mg/kg | Compound 2, 3% | None | 2/12 (17%) | 4/12 (33%) |
| 19 | Afatinib | 50 mg/kg | Compound 2, 3% | None | 2/12 (17%) | 5/12 (42%) |
| 20 | Osimertinib | 60 mg/kg | Compound 2, 3% | None | 1/12 (17%) | 4/12 (33%) |
| 21 | Sorafenib | 100 mg/kg | Compound 2b, 3% | None | 2/12 (17%) | 4/12 (33%) |
| 22 | Erlotinib | 70 mg/kg | Compound 2b, 3% | None | 2/12 (17%) | 5/12 (42%) |
| 23 | Afatinib | 50 mg/kg | Compound 2b, 3% | None | 2/12 (17%) | 4/12 (33%) |
| 24 | Osimertinib | 60 mg/kg | Compound 2b, 3% | None | 2/12 (17%) | 4/12 (33%) |
| 25 | Sorafenib | 100 mg/kg | Compound 5a, 3% | None | 1/12 (8%) | 5/12 (42%) |
| 26 | Erlotinib | 70 mg/kg | Compound 5a, 3% | None | 2/12 (17%) | 5/12 (42%) |
| 27 | Afatinib | 50 mg/kg | Compound 5a, 3% | None | 1/12 (8%) | 4/12 (33%) |
| 28 | Osimertinib | 60 mg/kg | Compound 5a, 3% | None | 1/12 (8%) | 4/12 (33%) |
| 29 | Sorafenib | 100 mg/kg | Compound 8b, 3% | None | 1/12 (8%) | 3/12 (25%) |

(continued)

| Serial number | Modeling drug | Dose | Compound and its mass proportion in an ointment | | Incidence rate | |
|---|---|---|---|---|---|---|
| | | | Left paw | Right paw | 左侧足爪 | 右侧足爪 |
| 30 | Erlotinib | 70 mg/kg | Compound 8b, 3% | None | 1/12 (8%) | 4/12 (33%) |
| 31 | Afatinib | 50 mg/kg | Compound 8b, 3% | None | 1/12 (8%) | 4/12 (33%) |
| 32 | Osimertinib | 60 mg/kg | Compound 8b, 3% | None | 1/12 (8%) | 3/12 (25%) |
| 33 | Sorafenib | 100 mg/kg | Compound 2, 10% | None | 0/12 (0%) | 5/12 (42%) |
| 34 | Erlotinib | 70 mg/kg | Compound 2, 10% | None | 1/12 (8%) | 4/12 (33%) |
| 35 | Afatinib | 50 mg/kg | Compound 2, 10% | None | 1/12 (8%) | 5/12 (42%) |
| 36 | Osimertinib | 60 mg/kg | Compound 2, 10% | None | 0/12 (0%) | 3/12 (25%) |
| 37 | Sorafenib | 100 mg/kg | Compound 2b, 10% | None | 0/12 (0%) | 4/12 (33%) |
| 38 | Erlotinib | 70 mg/kg | Compound 2b, 10% | None | 1/12 (8%) | 4/12 (33%) |
| 39 | Afatinib | 50 mg/kg | Compound 2b, 10% | None | 0/12 (0%) | 3/12 (25%) |
| 40 | Osimertinib | 60 mg/kg | Compound 2b, 10% | None | 0/12 (0%) | 3/12 (25%) |
| 41 | Sorafenib | 100 mg/kg | Compound 5a, 10% | None | 0/12 (0%) | 2/12 (17% |
| 42 | Erlotinib | 70 mg/kg | Compound 5a, 10% | None | 0/12 (0%) | 0/12 (0%) |
| 43 | Afatinib | 50 mg/kg | Compound 5a, 10% | None | 0/12 (0%) | 1/12 (8%) |
| 44 | Osimertinib | 60 mg/kg | Compound 5a, 10% | None | 0/12 (0%) | 1/12 (8%) |
| 45 | Sorafenib | 100 mg/kg | Compound 8b, 10% | None | 0/12 (0%) | 1/12 (8%) |
| 46 | Erlotinib | 70 mg/kg | Compound 8b, 10% | None | 0/12 (0%) | 0/12 (0%) |
| 47 | Afatinib | 50 mg/kg | Compound 8b, 10% | None | 0/12 (0%) | 1/12 (8%) |
| 48 | Osimertinib | 60 mg/kg | Compound 8b, 10% | None | 0/12 (0%) | 0/12 (0%) |
| 49 | Sorafenib | 100 mg/kg | None | None | 9/12 (75%) | |
| 50 | Erlotinib | 70 mg/kg | None | None | 10/12 (83.3%) | |
| 51 | Afatinib | 50 mg/kg | None | None | 8/12 (66.7%) | |
| 52 | Osimertinib | 60 mg/kg | None | None | 9/12 (75%) | |

As shown in Table 3, when the sorafenib, the erlotinib, the afatinib, and the osimertinib are respectively administered once daily by gavage at doses of 100 mg/kg, 70 mg/kg, 50 mg/kg, and 60 mg/kg to the SD rats, successful modeling rates for hand-foot skin reaction are 75%, 83.3%, 66.7%, and 75%, respectively. Some compounds (such as 2, 2b, 5a, and 8b, etc.) at low doses (such as compounds at a mass content of 1%) can significantly decrease the incidence rate of hand-foot skin reaction at a drug application site. Some compounds (such as 2, 2b, 5a, and 8b, etc.) at high mass contents (such as compounds at mass contents of 3% and 10%) can significantly decrease the incidence rate of hand-foot skin reaction in all paws of the rats.

According to stratum corneum thickness measurement results shown in FIG. 8, in the successfully modeled rats using the sorafenib, the erlotinib, the afatinib, and the osimertinib, the stratum corneum thickness of the paw plantar skin is significantly higher than that of normal rats. Some compounds (such as 2, 2b, 5a, and 8b, etc.) can effectively decrease the stratum corneum thickness of the paw plantar skin of the modeled rats.

According to tissue staining results shown in FIG. 9, due to skin toxicity caused by the modeling drugs, blisters can be formed under the epidermis and in the epidermis of the paw plantar skin of the rats, inflammatory cell infiltration occurs in the dermis, and congestion symptoms occur in skin tissues. The compounds 2b and 5a can effectively improve the formation of blisters under the epidermis and in the epidermis caused by the above modeling drugs, inhibit the inflammatory cell infiltration in the dermis, and significantly improve the skin congestion phenomenon.

According to pathological scoring results shown in FIG. 10, the modeling drugs can cause the formation of blisters under the epidermis and in the epidermis at paw plantar sites of the rats, inflammatory cell infiltration occurs in the dermis, and congestion symptoms occur in skin tissues. The compounds 2, 2b, 5a, and 8b can effectively improve the formation of

blisters under the epidermis and in the epidermis caused by the above modeling drugs, inhibit the inflammatory cell infiltration in the dermis, and significantly improve the skin congestion phenomenon.

Immunohistochemical staining results also show that in the successfully modeled rats using the sorafenib, the erlotinib, the afatinib, and the osimertinib, KRT1 and Loricrin are significantly increased in tissues. However, with the addition of compounds, KRT1 and Loricrin are significantly decreased in the tissues. **In** summary, the compounds 2, 2b, 5a, and 8b can effectively inhibit the differentiation of keratinocytes both *in vitro* and *in vivo,* thus having the potential for treatment of a hyperkeratosis-related disease.

**Example 23:** Effects of compound 2b in chemotherapeutic drug-induced mouse models with hand-foot syndrome

After adaptive feeding for 7 days, SPF-grade ICR mice (male, 5-6 weeks old, with a body weight of approximately 35 g) were randomly divided into groups, including a blank control group (6 mice), a capecitabine modeling group (6 mice), a docetaxel modeling group (6 mice), a capecitabine + compound 2b group (6 mice), a docetaxel + compound 2b group (6 mice), a capecitabine + nicotinamide group (6 mice), and a docetaxel + nicotinamide group (6 mice). The groups were respectively administered corresponding modeling drugs by oral gavage (capecitabine at 200 mg/kg and docetaxel at 25 mg/kg) once daily; and the blank control group was administered an equivalent volume of normal saline. After administration with the chemotherapeutic drugs for 2 weeks, the compound ointments (at a compound content of 3%) used in Example 22 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind paws of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of $Na_2HPO_4$, and 1 M HCl, pH 7.4). The sections were permeabilized with 0.5% Triron-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin

adhesive (Solarbio, Catalog No. 20230725), and inspected under a microscope.

Mouse skin appearance results are shown in FIG. 11. After modeling with the capecitabine and the docetaxel for 14 days, the skin of the hind limb toes of the mice has obvious lesions. Compared with the normal control group, the skin of the hind limb toes of the mice in the capecitabine group and the docetaxel group has obvious erythema, rhagades, and blisters.

The degree of swelling of the hind limb toes of the mice is shown in FIG. 12. After continuous oral administration of the modeling drugs including the capecitabine and the docetaxel for 14 days, swelling of the hind limb toes of the mice is caused. Topical application of the compound 2b to the hind limb toes of the mice can significantly alleviate the swelling of the toes caused by the modeling drugs ($p<0.05$). However, it is not observed that oral administration of the nicotinamide at 100 mg/kg significantly alleviates the swelling of the hind limb toes of the mice.

Pathological staining results of toe skin tissues are shown in FIG. 13. Compared with the normal control group, after continuous oral administration of the modeling drugs including the capecitabine and the docetaxel for 14 days, the epidermal layer and the stratum corneum in the skin tissues of the hind limb toes of the mice are significantly thickened, with increased granular cells in the basal layer and infiltration of inflammatory cells observed. Topical application of the compound 2b to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of the nicotinamide at 100 mg/kg mildly alleviates the thickening of the epidermal layer and the stratum corneum in the skin tissues of the hind limb toes of the mice, with slightly increased granular cells in the basal layer and infiltration of inflammatory cells still observed.

Immunohistochemical results of the capecitabine modeling series groups are shown in FIG. 14. Compared with the normal control group, after continuous oral administration of the modeling drug capecitabine for 14 days, cytokine IL-8 in the skin tissues of the hind limb toes of the mice is significantly increased ($p<0.05$). It is indicated that oral administration of the capecitabine results in skin inflammation of the mice, which is consistent with a clinical trial observation result. Topical application of the compound 2b to the hind limb toes of the mice can significantly decrease the expression level of cytokine IL-8 in the skin tissues of the hind limb toes of the mice ($p<0.05$). However, oral administration of the nicotinamide at 100 mg/kg does not have a significant impact on the expression level of cytokine IL-8 in the skin tissues of the hind limb toes of the mice.

Immunohistochemical results of the docetaxel modeling series groups are shown in FIG. 15. Compared with the normal control group, after continuous oral administration of the modeling drug docetaxel for 14 days, cytokine IL-6 in the skin tissues of the hind limb toes of the mice is significantly increased ($p<0.05$). It is indicated that oral administration of the docetaxel results in skin inflammation of the mice, which is consistent with a clinical trial observation result. Topical application of the compound 2b to the hind limb toes of the mice can significantly decrease the expression level of cytokine IL-6 in the skin tissues of the hind limb toes of the mice ($p<0.05$). However, oral administration of the nicotinamide at 100 mg/kg does not have a significant impact on the expression level of cytokine IL-6 in the skin tissues of the hind limb toes of the mice.

**Example 24:** Effects of compound 2b in kinase inhibitor-induced mouse models with hand-foot skin reaction

After adaptive feeding for 7 days, SPF-grade ICR mice (male, 5-6 weeks old, with a body weight of approximately 35 g) were randomly divided into groups, including a blank control group (6 mice), a sorafenib modeling group (6 mice), an osimertinib modeling group (6 mice), a sorafenib + compound 2b group (6 mice), an osimertinib + compound 2b group (6 mice), a sorafenib + nicotinamide group (6 mice), and an osimertinib + nicotinamide group (6 mice). The groups were respectively administered corresponding modeling drugs by oral gavage (sorafenib at 100 mg/kg and osimertinib at 10 mg/kg) once daily; and the blank control group was administered an equivalent volume of normal saline. After administration with the chemotherapeutic drugs for 2 weeks, the compound ointments (at a compound content of 3%) used in Example 22 were respectively applied topically in the groups or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind paws of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became

transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of $Na_2HPO_4$, and 1 M HCl, pH 7.4). The sections were permeabilized with 0.5% Triron-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

Results are shown in FIG. 16. After modeling with the sorafenib and the osimertinib for 14 days, the skin of the hind limb toes of the mice has obvious lesions. Compared with the normal control group, the skin of the hind limb toes of the mice in the sorafenib group and the osimertinib group has obvious erythema and rhagades.

The degree of swelling of the hind limb toes of the mice is shown in FIG. 17. After continuous oral administration of the modeling drugs including the sorafenib and the osimertinib for 14 days, significant swelling of the hind limb toes of the mice is caused. Topical application of the compound 2b to the hind limb toes of the mice can effectively alleviate the swelling of the toes caused by the modeling drugs. However, it is not observed that oral administration of the nicotinamide at 100 mg/kg significantly alleviates the swelling of the hind limb toes of the mice.

Pathological staining results of toe skin tissues are shown in FIG. 18. Compared with the normal control group, after continuous oral administration of the modeling drugs including the sorafenib and the osimertinib for 14 days, the epidermal layer and the stratum corneum in the skin tissues of the hind limb toes of the mice are significantly thickened, with increased granular cells in the basal layer and infiltration of inflammatory cells observed. Topical application of the compound 2b to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of the nicotinamide at 100 mg/kg does not significantly alleviate the thickening of the epidermal layer and the stratum corneum in the skin tissues of the hind limb toes of the mice, with slightly restored morphology of basal layer cells and infiltration of inflammatory cells still observed.

Immunohistochemical results of the sorafenib modeling series groups are shown in FIG. 19. Compared with the normal control group, after continuous oral administration of the modeling drug sorafenib for 14 days, cytokine IL-1β in the skin tissues of the hind limb toes of the mice is significantly increased ($p<0.05$). It is indicated that oral administration of the sorafenib results in skin inflammation of the mice, which is consistent with a clinical trial observation result. Topical application of the compound 2b to the hind limb toes of the mice can significantly decrease the expression level of cytokine IL-1β in the skin tissues of the hind limb toes of the mice ($p<0.05$). However, oral administration of the nicotinamide at 100 mg/kg does not have a significant impact on the expression level of cytokine IL-1β in the skin tissues of the hind limb toes of the mice.

Immunohistochemical results of the osimertinib modeling series groups are shown in FIG. 20. Compared with the normal control group, after continuous oral administration of the modeling drug osimertinib for 14 days, cytokine IL-1β in the skin tissues of the hind limb toes of the mice is significantly increased ($p<0.05$). It is indicated that oral administration of the osimertinib results in skin inflammation of the mice, which is consistent with a clinical trial observation result. Topical

application of the compound 2b to the hind limb toes of the mice can significantly decrease the expression level of cytokine IL-1β in the skin tissues of the hind limb toes of the mice ($p<0.05$). However, oral administration of the nicotinamide at 100 mg/kg does not have a significant impact on the expression level of cytokine IL-1β in the skin tissues of the hind limb toes of the mice.

All reference documents cited herein, including publications, patent applications, and patents, are incorporated herein by reference. Each reference document is cited as if individually and specifically indicated to be incorporated herein by reference and to the same extent as if fully set forth herein.

It should be understood that the above examples are all exemplary and are not intended to encompass all possible embodiments covered by the claims. Without departing from the scope of the present disclosure, various modifications and alterations may also be made based on the above examples. Similarly, various technical features of the above examples may also be arbitrarily combined to form other examples of the present disclosure that may not have been explicitly described. Therefore, the above examples merely express several embodiments of the present disclosure and do not limit the scope of protection of the present disclosure.

## Claims

1. A compound of formula I, or a stereoisomer, a tautomer, a solvate, a hydrate, a prodrug, a stable isotope derivative, and a pharmaceutically acceptable salt thereof:

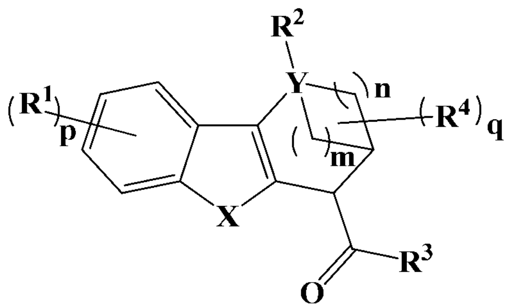

I

wherein,

X is selected from $NR^a$, O, and S;
Y is selected from C and N;
$R^1$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, wherein the substituted means substituted with one or more $Q^1$;
$R^2$ is selected from hydrogen, deuterium, halogen, $-S(O)_2-R^d$, $-C(O)-R^e$, $-NR^iR^j$, substituted or unsubstituted alkyl, alkoxy, haloalkyl, cycloalkyl, aryl, heteroaryl, and heterocyclyl, wherein the substituted means substituted with one or more $Q^2$;
$R^3$ is selected from hydrogen, deuterium, hydroxyl, $-NR^iR^j$, substituted or unsubstituted alkyl, alkoxy, haloalkyl, cycloalkyl, aryl, heteroaryl, and heterocyclyl, wherein the substituted means substituted with one or more $Q^3$;
$R^4$ is selected from hydrogen, deuterium, halogen, hydroxyl, cyano, $-OR^b$, $-SR^c$, $-NR^iR^j$, substituted or unsubstituted alkyl, alkoxy, haloalkyl, cycloalkyl, aryl, heteroaryl, and heterocyclyl, wherein the substituted means substituted with one or more $Q^4$;
$R^a$ is selected from hydrogen, deuterium, alkyl, and haloalkyl;
each $R^b$ is respectively and independently selected from hydrogen, deuterium, substituted or unsubstituted alkyl, alkoxy, haloalkyl, cycloalkyl, aryl, heteroaryl, and heterocyclyl, wherein the substituted means substituted with one or more $Q^5$;
each $R^c$ is respectively and independently selected from hydrogen, deuterium, substituted or unsubstituted alkyl, alkoxy, haloalkyl, cycloalkyl, aryl, heteroaryl, and heterocyclyl, wherein the substituted means substituted with one or more $Q^6$;
each of $R^i$ and $R^j$ is respectively and independently selected from hydrogen, deuterium, $-S(O)_2-R^d$, substituted or unsubstituted alkyl, alkoxy, haloalkyl, cycloalkyl, aryl, heteroaryl, and heterocyclyl, wherein the substituted means substituted with one or more $Q^7$;

each $R^d$ is respectively and independently selected from hydrogen, deuterium, substituted or unsubstituted alkyl, alkoxy, haloalkyl, cycloalkyl, aryl, heteroaryl, and heterocyclyl, wherein the substituted means substituted with one or more $Q^8$;
each $R^e$ is respectively and independently selected from hydrogen, deuterium, substituted or unsubstituted alkyl, alkoxy, haloalkyl, cycloalkyl, aryl, heteroaryl, and heterocyclyl, wherein the substituted means substituted with one or more $Q^9$;
each of $Q^1$, $Q^2$, $Q^3$, $Q^4$, $Q^5$, $Q^6$, $Q^7$, $Q^8$, and $Q^9$ is independently selected from hydrogen, deuterium, halogen, cyano, hydroxyl, mercapto, amino, substituted or unsubstituted alkyl, alkoxy, haloalkyl, cycloalkyl, aryl, heteroaryl, and heterocyclyl, wherein the substituted means substituted with one or more $Q^{10}$;
$Q^{10}$ is selected from hydrogen, deuterium, halogen, alkyl, alkoxy, haloalkyl, cycloalkyl, aryl, heteroaryl, and heterocyclyl;
each of m and n is independently 0, 1, or 2; and
each of p and q is independently 0, 1, 2, 3, or 4.

2. The compound of formula I, or the stereoisomer, the tautomer, the solvate, the hydrate, the prodrug, the stable isotope derivative, and the pharmaceutically acceptable salt thereof according to claim 1, wherein the compound of formula I is selected from the following structures:

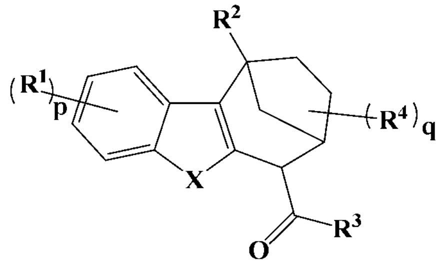

I-1 ,

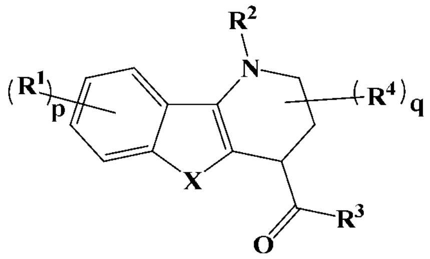

I-2 ,

and

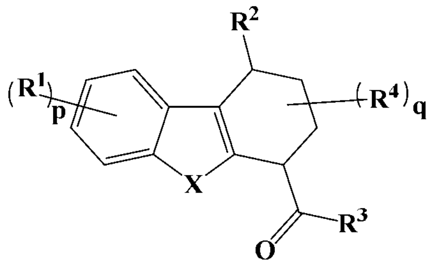

I-3 .

3. The compound of formula I, or the stereoisomer, the tautomer, the solvate, the hydrate, the prodrug, the stable isotope derivative, and the pharmaceutically acceptable salt thereof according to claim 1 or 2, wherein X is selected from $NR^a$ and O; and preferably selected from $NR^a$.

4. The compound of formula I, or the stereoisomer, the tautomer, the solvate, the hydrate, the prodrug, the stable isotope derivative, and the pharmaceutically acceptable salt thereof according to any one of claims 1-3, wherein $R^a$ is selected from hydrogen, deuterium, and alkyl; preferably selected from hydrogen and $C_{1-3}$ alkyl; and more preferably selected from hydrogen and methyl.

5. The compound of formula I, or the stereoisomer, the tautomer, the solvate, the hydrate, the prodrug, the stable isotope derivative, and the pharmaceutically acceptable salt thereof according to any one of claims 1-4, wherein $R^1$ is selected from hydrogen, deuterium, halogen, $-OR^b$, $-NR^iR^j$, substituted or unsubstituted alkyl, cycloalkyl, aryl, heteroaryl, and heterocyclyl; preferably selected from hydrogen, deuterium, halogen, $-OR^b$, $-NR^iR^i$, substituted or unsubstituted $C_{1-6}$ alkyl, $C_{3-7}$ cycloalkyl, $C_{6-10}$ aryl, 5-10 membered heteroaryl, and 3-10 membered heterocyclyl; more preferably selected from hydrogen, deuterium, fluorine, chlorine, bromine, $-OR^b$, $-NR^iR^j$, substituted or unsubstituted $C_{1-3}$ alkyl,

$C_{3-6}$ cycloalkyl, $C_{6-10}$ aryl, 5-6 membered heteroaryl, and 3-6 membered heterocyclyl; and most preferably selected from hydrogen, fluorine, chlorine, -$OR^b$, -$NR^iR^j$, substituted or unsubstituted methyl, and cyclopropyl.

6. The compound of formula I, or the stereoisomer, the tautomer, the solvate, the hydrate, the prodrug, the stable isotope derivative, and the pharmaceutically acceptable salt thereof according to any one of claims 1-5, wherein $Q^1$ is selected from hydrogen, halogen, cyano, hydroxyl, amino, and substituted or unsubstituted alkyl; preferably selected from hydrogen, halogen, and substituted or unsubstituted $C_{1-3}$ alkyl; more preferably selected from hydrogen and halogen; and most preferably selected from hydrogen.

7. The compound of formula I, or the stereoisomer, the tautomer, the solvate, the hydrate, the prodrug, the stable isotope derivative, and the pharmaceutically acceptable salt thereof according to any one of claims 1-6, wherein $R^b$ is selected from hydrogen, substituted or unsubstituted alkyl, and cycloalkyl; preferably selected from substituted or unsubstituted $C_{1-6}$ alkyl and $C_{3-7}$ cycloalkyl; more preferably selected from substituted or unsubstituted $C_{1-3}$ alkyl and $C_{3-6}$ cycloalkyl; and most preferably selected from substituted or unsubstituted methyl, cyclopropyl, and cyclohexyl.

8. The compound of formula I, or the stereoisomer, the tautomer, the solvate, the hydrate, the prodrug, the stable isotope derivative, and the pharmaceutically acceptable salt thereof according to any one of claims 1-7, wherein $Q^5$ is selected from hydrogen, halogen, cyano, hydroxyl, amino, and substituted or unsubstituted heteroaryl; preferably selected from hydrogen, halogen, and substituted or unsubstituted 5-10 membered heteroaryl; more preferably selected from hydrogen, halogen, and substituted or unsubstituted 5-6 membered heteroaryl, wherein the heteroaryl contains 1-4 heteroatoms selected from N, O, and S; and most preferably selected from hydrogen and substituted or unsubstituted pyridyl.

9. The compound of formula I, or the stereoisomer, the tautomer, the solvate, the hydrate, the prodrug, the stable isotope derivative, and the pharmaceutically acceptable salt thereof according to any one of claims 1-8, wherein $R^c$ is selected from hydrogen, substituted or unsubstituted alkyl, and cycloalkyl; preferably selected from substituted or unsubstituted $C_{1-6}$ alkyl and $C_{3-7}$ cycloalkyl; more preferably selected from substituted or unsubstituted $C_{1-3}$ alkyl and $C_{3-6}$ cycloalkyl; and most preferably selected from substituted or unsubstituted methyl, cyclopropyl, and cyclohexyl.

10. The compound of formula I, or the stereoisomer, the tautomer, the solvate, the hydrate, the prodrug, the stable isotope derivative, and the pharmaceutically acceptable salt thereof according to any one of claims 1-9, wherein $Q^6$ is selected from hydrogen, halogen, cyano, hydroxyl, amino, and substituted or unsubstituted heteroaryl; preferably selected from hydrogen, halogen, and substituted or unsubstituted 5-10 membered heteroaryl; more preferably selected from hydrogen, halogen, and substituted or unsubstituted 5-6 membered heteroaryl, wherein the heteroaryl contains 1-4 heteroatoms selected from N, O, and S; and most preferably selected from hydrogen and substituted or unsubstituted pyridyl.

11. The compound of formula I, or the stereoisomer, the tautomer, the solvate, the hydrate, the prodrug, the stable isotope derivative, and the pharmaceutically acceptable salt thereof according to any one of claims 1-10, wherein $R^i$ is selected from hydrogen, -$S(O)_2$-$R^d$, substituted or unsubstituted alkyl, cycloalkyl, aryl, and heteroaryl; preferably selected from hydrogen, -$S(O)_2$-$R^d$, substituted or unsubstituted $C_{1-6}$ alkyl, $C_{3-7}$ cycloalkyl, $C_{6-10}$ aryl, and 5-10 membered heteroaryl; more preferably selected from hydrogen, -$S(O)_2$-$R^d$, substituted or unsubstituted $C_{1-3}$ alkyl, $C_{3-6}$ cycloalkyl, $C_{6-10}$ aryl, and 5-6 membered heteroaryl, wherein the heteroaryl contains 1-4 heteroatoms selected from N, O, and S; and most preferably selected from hydrogen, -$S(O)_2$-$R^d$, substituted or unsubstituted methyl, ethyl, cyclopentyl, phenyl, pyridyl, furanyl, and imidazolyl.

12. The compound of formula I, or the stereoisomer, the tautomer, the solvate, the hydrate, the prodrug, the stable isotope derivative, and the pharmaceutically acceptable salt thereof according to any one of claims 1-11, wherein $R^j$ is selected from hydrogen, -$S(O)_2$-$R^d$, substituted or unsubstituted alkyl, cycloalkyl, aryl, and heteroaryl; preferably selected from hydrogen, -$S(O)_2$-$R^d$, substituted or unsubstituted $C_{1-6}$ alkyl, $C_{3-7}$ cycloalkyl, $C_{6-10}$ aryl, and 5-10 membered heteroaryl; more preferably selected from hydrogen, -$S(O)_2$-$R^d$, substituted or unsubstituted $C_{1-3}$ alkyl, $C_{3-6}$ cycloalkyl, $C_{6-10}$ aryl, and 5-6 membered heteroaryl, wherein the heteroaryl contains 1-4 heteroatoms selected from N, O, and S; and most preferably selected from hydrogen, -$S(O)_2$-$R^d$, substituted or unsubstituted methyl, ethyl, cyclopentyl, phenyl, pyridyl, furanyl, and imidazolyl.

13. The compound of formula I, or the stereoisomer, the tautomer, the solvate, the hydrate, the prodrug, the stable isotope derivative, and the pharmaceutically acceptable salt thereof according to any one of claims 1-12, wherein $Q^7$ is selected from hydrogen, halogen, cyano, hydroxyl, amino, and substituted or unsubstituted heteroaryl; preferably

selected from hydrogen, halogen, and substituted or unsubstituted 5-10 membered heteroaryl; more preferably selected from hydrogen, halogen, and substituted or unsubstituted 5-6 membered heteroaryl, wherein the heteroaryl contains 1-4 heteroatoms selected from N, O, and S; and most preferably selected from hydrogen and pyridyl.

**14.** The compound of formula I, or the stereoisomer, the tautomer, the solvate, the hydrate, the prodrug, the stable isotope derivative, and the pharmaceutically acceptable salt thereof according to any one of claims 1-13, wherein $R^d$ is selected from hydrogen, substituted or unsubstituted alkyl, cycloalkyl, aryl, and heteroaryl; preferably selected from hydrogen, substituted or unsubstituted $C_{1-6}$ alkyl, $C_{3-7}$ cycloalkyl, $C_{6-10}$ aryl, and 5-10 membered heteroaryl; more preferably selected from hydrogen, substituted or unsubstituted $C_{1-3}$ alkyl, $C_{3-6}$ cycloalkyl, and $C_{6-10}$ aryl; and most preferably selected from methyl, cyclopropyl, and phenyl.

**15.** The compound of formula I, or the stereoisomer, the tautomer, the solvate, the hydrate, the prodrug, the stable isotope derivative, and the pharmaceutically acceptable salt thereof according to any one of claims 1-14, wherein $Q^8$ is selected from hydrogen, halogen, cyano, hydroxyl, mercapto, and amino; preferably selected from hydrogen, halogen, and amino; more preferably selected from hydrogen, fluorine, and chlorine; and most preferably selected from hydrogen.

**16.** The compound of formula I, or the stereoisomer, the tautomer, the solvate, the hydrate, the prodrug, the stable isotope derivative, and the pharmaceutically acceptable salt thereof according to any one of claims 1-15, wherein $R^2$ is selected from hydrogen, halogen, $-S(O)_2-R^d$, $-C(O)-R^e$, $-NR^iR^j$, substituted or unsubstituted alkyl, cycloalkyl, aryl, and heteroaryl; preferably selected from hydrogen, halogen, $-S(O)_2-R^d$, $-C(O)-R^e$, substituted or unsubstituted $C_{1-6}$ alkyl, and $C_{3-7}$ cycloalkyl; more preferably selected from hydrogen, halogen, $-S(O)_2-R^d$, $- C(O)-R^e$, $C_{1-3}$ alkyl, and $C_{3-6}$ cycloalkyl; and most preferably selected from hydrogen, fluorine, $-S(O)_2-R^d$, $- C(O)-R^e$, methyl, isopropyl, and cyclopropyl.

**17.** The compound of formula I, or the stereoisomer, the tautomer, the solvate, the hydrate, the prodrug, the stable isotope derivative, and the pharmaceutically acceptable salt thereof according to any one of claims 1-16, wherein $Q^2$ is selected from hydrogen, halogen, cyano, hydroxyl, mercapto, and amino; preferably selected from hydrogen, halogen, and amino; more preferably selected from hydrogen, fluorine, and chlorine; and most preferably selected from hydrogen.

**18.** The compound of formula I, or the stereoisomer, the tautomer, the solvate, the hydrate, the prodrug, the stable isotope derivative, and the pharmaceutically acceptable salt thereof according to any one of claims 1-17, wherein $R^e$ is selected from hydrogen, substituted or unsubstituted alkyl, aryl, and heterocyclyl; preferably selected from hydrogen, substituted or unsubstituted $C_{1-6}$ alkyl, $C_{6-10}$ aryl, and 3-10 membered heterocyclyl; more preferably selected from hydrogen, substituted or unsubstituted $C_{1-3}$ alkyl, $C_{6-10}$ aryl, and 3-6 membered heterocyclyl; and most preferably selected from hydrogen, substituted or unsubstituted methyl, ethyl, phenyl, and piperidinyl.

**19.** The compound of formula I, or the stereoisomer, the tautomer, the solvate, the hydrate, the prodrug, the stable isotope derivative, and the pharmaceutically acceptable salt thereof according to any one of claims 1-18, wherein $Q^9$ is selected from hydrogen, halogen, substituted or unsubstituted alkyl, aryl, and heteroaryl; preferably selected from hydrogen, halogen, substituted or unsubstituted $C_{1-6}$ alkyl, $C_{6-10}$ aryl, and 5-10 membered heteroaryl; more preferably selected from hydrogen, halogen, substituted or unsubstituted $C_{1-3}$ alkyl, and 5-6 membered heteroaryl, wherein the heteroaryl contains 1-4 heteroatoms selected from N, O, and S; and most preferably selected from hydrogen, fluorine, methyl, and pyrrolyl.

**20.** The compound of formula I, or the stereoisomer, the tautomer, the solvate, the hydrate, the prodrug, the stable isotope derivative, and the pharmaceutically acceptable salt thereof according to any one of claims 1-19, wherein $Q^{10}$ is selected from hydrogen, halogen, and substituted or unsubstituted alkyl; preferably selected from hydrogen and halogen; and more preferably selected from hydrogen and fluorine.

**21.** The compound of formula I, or the stereoisomer, the tautomer, the solvate, the hydrate, the prodrug, the stable isotope derivative, and the pharmaceutically acceptable salt thereof according to any one of claims 1-20, wherein $R^3$ is selected from hydrogen, hydroxyl, $-NR^iR^j$, substituted or unsubstituted alkyl, and alkoxy; preferably selected from hydrogen, hydroxyl, and $-NR^iR^j$; and more preferably selected from $-NR^iR^j$.

**22.** The compound of formula I, or the stereoisomer, the tautomer, the solvate, the hydrate, the prodrug, the stable isotope derivative, and the pharmaceutically acceptable salt thereof according to any one of claims 1-21, wherein $Q^3$ is

selected from hydrogen, halogen, cyano, hydroxyl, mercapto, and amino; preferably selected from hydrogen, halogen, and amino; more preferably selected from hydrogen, fluorine, and chlorine; and most preferably selected from hydrogen.

**23.** The compound of formula I, or the stereoisomer, the tautomer, the solvate, the hydrate, the prodrug, the stable isotope derivative, and the pharmaceutically acceptable salt thereof according to any one of claims 1-22, wherein $R^4$ is selected from hydrogen, halogen, $-OR^b$, and $-NR^iR^j$; preferably selected from hydrogen and halogen; more preferably selected from hydrogen, fluorine, chlorine, and bromine; and most preferably selected from hydrogen and fluorine.

**24.** The compound of formula I, or the stereoisomer, the tautomer, the solvate, the hydrate, the prodrug, the stable isotope derivative, and the pharmaceutically acceptable salt thereof according to any one of claims 1-23, wherein $Q^4$ is selected from hydrogen, halogen, cyano, hydroxyl, amino, and substituted or unsubstituted alkyl; preferably selected from hydrogen, halogen, and substituted or unsubstituted $C_{1-3}$ alkyl; more preferably selected from hydrogen and halogen; and most preferably selected from hydrogen.

**25.** The compound of formula I, or the stereoisomer, the tautomer, the solvate, the hydrate, the prodrug, the stable isotope derivative, and the pharmaceutically acceptable salt thereof according to any one of claims 1-24, wherein m is 0 or 1.

**26.** The compound of formula I, or the stereoisomer, the tautomer, the solvate, the hydrate, the prodrug, the stable isotope derivative, and the pharmaceutically acceptable salt thereof according to any one of claims 1-25, wherein n is 1 or 2.

**27.** The compound of formula I, or the stereoisomer, the tautomer, the solvate, the hydrate, the prodrug, the stable isotope derivative, and the pharmaceutically acceptable salt thereof according to any one of claims 1-26, wherein p is 1, 2, or 3.

**28.** The compound of formula I, or the stereoisomer, the tautomer, the solvate, the hydrate, the prodrug, the stable isotope derivative, and the pharmaceutically acceptable salt thereof according to any one of claims 1-27, wherein q is 0, 1, or 2.

**29.** The compound of formula I, or the stereoisomer, the tautomer, the solvate, the hydrate, the prodrug, the stable isotope derivative, and the pharmaceutically acceptable salt thereof according to any one of claims 1-28, wherein X is selected from $NR^a$;

$R^a$ is selected from hydrogen and $C_{1-3}$ alkyl;
$R^1$ is selected from hydrogen, fluorine, chlorine, bromine, $-OR^b$, $-NR^iR^j$, substituted or unsubstituted $C_{1-3}$ alkyl, $C_{3-6}$ cycloalkyl, $C_{6-10}$ aryl, 5-6 membered heteroaryl, and 3-6 membered heterocyclyl, wherein the substituted means substituted with one or more $Q^1$;
$Q^1$ is selected from hydrogen and halogen;
$R^b$ is selected from substituted or unsubstituted $C_{1-3}$ alkyl and $C_{3-6}$ cycloalkyl, wherein the substituted means substituted with one or more $Q^5$;
$Q^5$ is selected from hydrogen, halogen, and substituted or unsubstituted 5-6 membered heteroaryl, wherein the heteroaryl contains 1-4 heteroatoms selected from N, O, and S, and the substituted means substituted with one or more $Q^{10}$;
$R^i$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, wherein the heteroaryl contains 1-4 heteroatoms selected from N, O, and S, and the substituted means substituted with one or more $Q^7$;
$R^j$ is selected from hydrogen, $-S(O)_2-R^d$, substituted or unsubstituted $C_{1-3}$ alkyl, $C_{3-6}$ cycloalkyl, $C_{6-10}$ aryl, and 5-6 membered heteroaryl, wherein the heteroaryl contains 1-4 heteroatoms selected from N, O, and S, and the substituted means substituted with one or more $Q^7$;
$Q^7$ is selected from hydrogen, halogen, and substituted or unsubstituted 5-6 membered heteroaryl, wherein the heteroaryl contains 1-4 heteroatoms selected from N, O, and S, and the substituted means substituted with one or more $Q^{10}$;
$R^d$ is selected from hydrogen, substituted or unsubstituted $C_{1-3}$ alkyl, $C_{3-5}$ cycloalkyl, and $C_{6-10}$ aryl, wherein the substituted means substituted with one or more $Q^8$;
$Q^8$ is selected from hydrogen, fluorine, and chlorine;
$R^2$ is selected from hydrogen, halogen, $-S(O)_2-R^d$, $-C(O)-R^e$, $C_{1-3}$ alkyl, and $C_{3-6}$ cycloalkyl;
$R^e$ is selected from hydrogen, substituted or unsubstituted $C_{1-3}$ alkyl, $C_{6-10}$ aryl, and 3-6 membered heterocyclyl, wherein the substituted means substituted with one or more $Q^9$;
$Q^9$ is selected from hydrogen, halogen, substituted or unsubstituted $C_{1-3}$ alkyl, and 5-6 membered heteroaryl, wherein the heteroaryl contains 1-4 heteroatoms selected from N, O, and S, and the substituted means

substituted with one or more $Q^{10}$;
$Q^{10}$ is selected from hydrogen and halogen;
$R^3$ is selected from $-NR^iR^j$;
$R^4$ is selected from hydrogen, fluorine, chlorine, and bromine;
m is 0 or 1;
n is 1 or 2;
p is 1, 2, or 3; and
q is 0, 1, or 2.

**30.** The compound of formula I, or the stereoisomer, the tautomer, the solvate, the hydrate, the prodrug, the stable isotope derivative, and the pharmaceutically acceptable salt thereof according to any one of claims 1-29, wherein X is selected from $NR^a$;

$R^a$ is selected from hydrogen and methyl;
$R^1$ is selected from hydrogen, fluorine, chlorine, $-OR^b$, $-NR^iR^j$, methyl, and cyclopropyl;
$R^b$ is selected from substituted or unsubstituted methyl, cyclopropyl, and cyclohexyl, wherein the substituted means substituted with one or more $Q^5$;
$Q^5$ is selected from hydrogen and substituted or unsubstituted pyridyl, wherein the substituted means substituted with one or more $Q^{10}$;
$R^i$ is selected from hydrogen, $-S(O)_2-R^d$, substituted or unsubstituted methyl, ethyl, cyclopentyl, phenyl, pyridyl, furanyl, and imidazolyl, wherein the substituted means substituted with one or more $Q^7$;
$R^j$ is selected from hydrogen, $-S(O)_2-R^d$, substituted or unsubstituted methyl, ethyl, cyclopentyl, phenyl, pyridyl, furanyl, and imidazolyl, wherein the substituted means substituted with one or more $Q^7$;
$Q^7$ is selected from hydrogen and pyridyl;
$R^d$ is selected from methyl, cyclopropyl, and phenyl;
$R^2$ is selected from hydrogen, fluorine, $-S(O)_2-R^d$, $-C(O)-R^e$, methyl, isopropyl, and cyclopropyl;
$R^e$ is selected from hydrogen, substituted or unsubstituted methyl, ethyl, phenyl, and piperidinyl, wherein the substituted means substituted with one or more $Q^9$;
$Q^9$ is selected from hydrogen, fluorine, methyl, and pyrrolyl;
$Q^{10}$ is selected from hydrogen and fluorine;
$R^3$ is selected from $- NR^iR^j$;
$R^4$ is selected from hydrogen and fluorine;
m is 0 or 1;
n is 1 or 2;
p is 1, 2, or 3; and
q is 0, 1, or 2.

**31.** A compound, or a stereoisomer, a tautomer, a solvate, a hydrate, a prodrug, a stable isotope derivative, and a pharmaceutically acceptable salt thereof, wherein the compound is any one of the following:

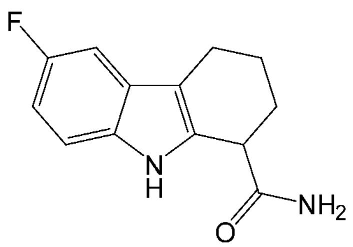 , 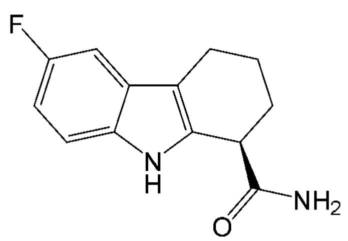 , 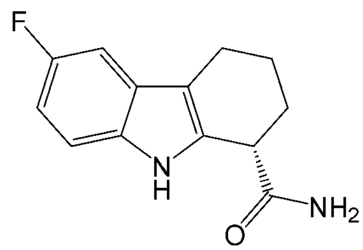 ,

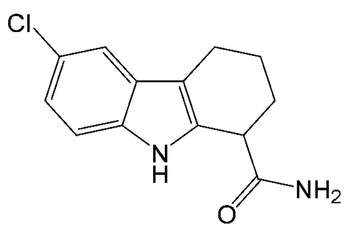 , 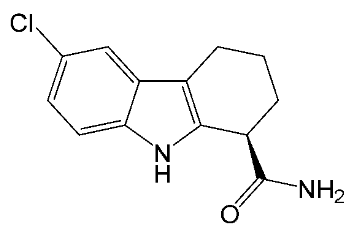 , 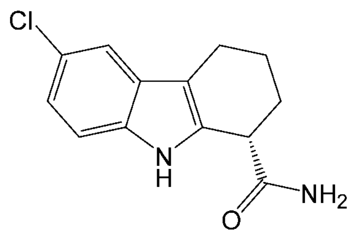 ,

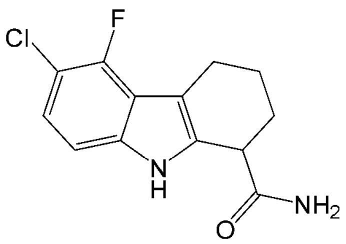 , 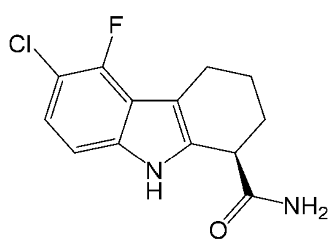 , 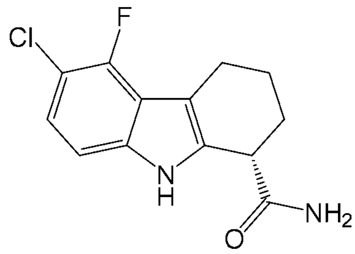 ,

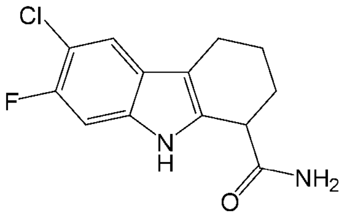 , 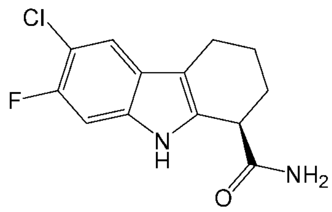 , 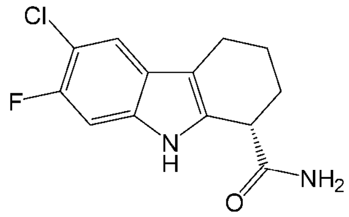 ,

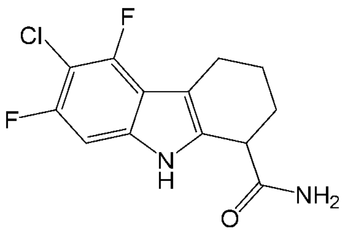 , 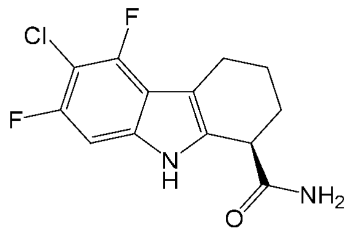 , 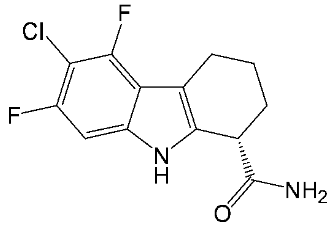 ,

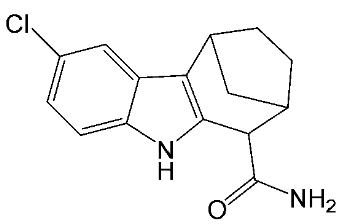 , 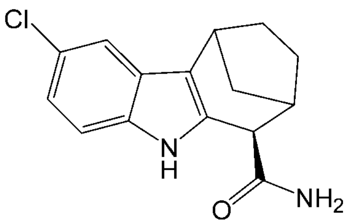 , 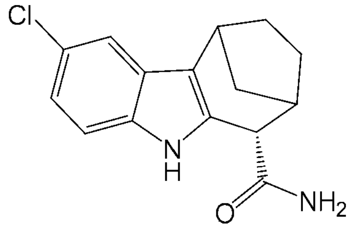 ,

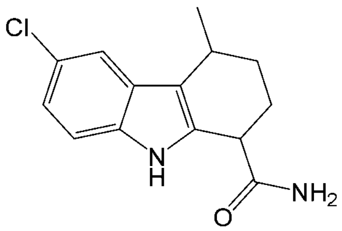 , 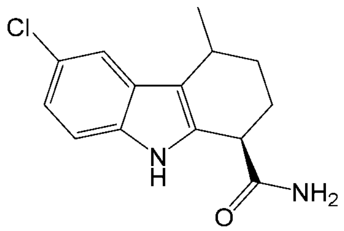 , 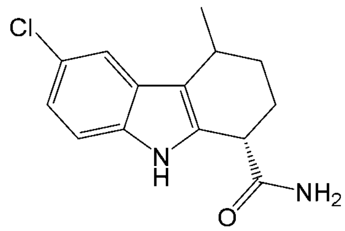 ,

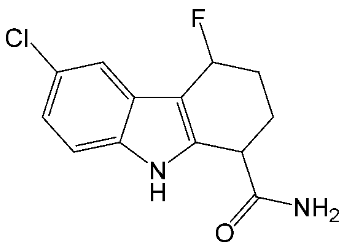 , 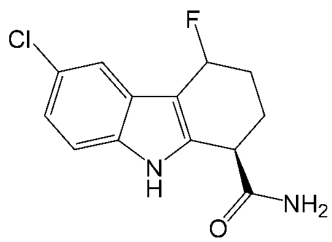 , 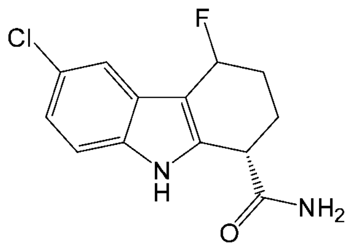 ,

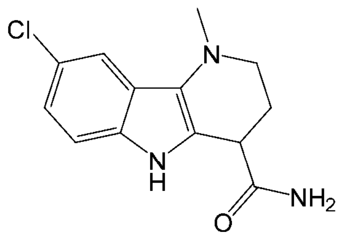 ,
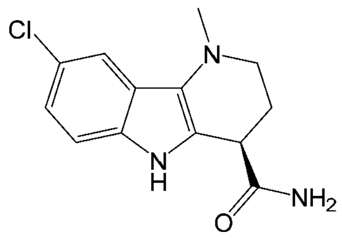 ,
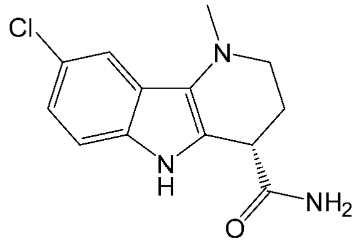 ,

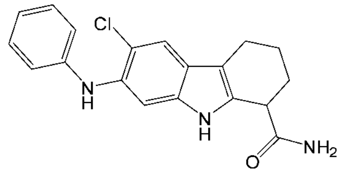 ,
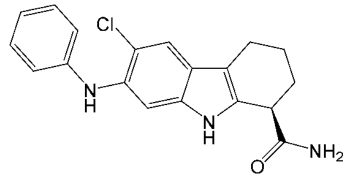 ,
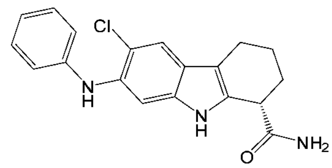 ,

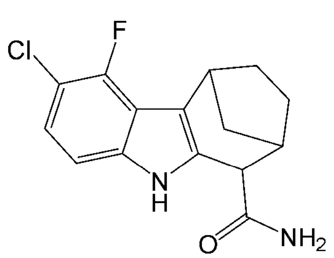 ,
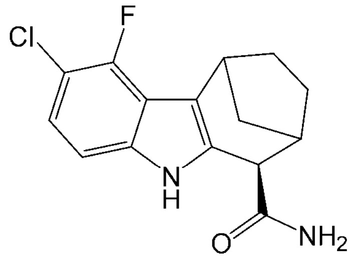 ,
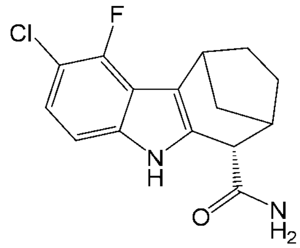 ,

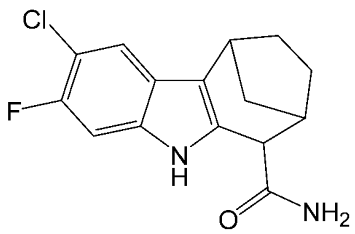 ,
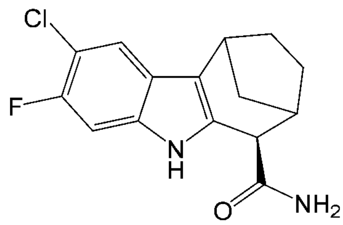 ,
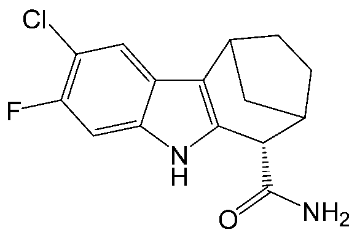 ,

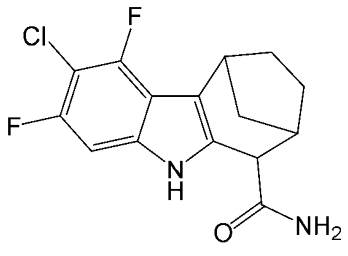 ,
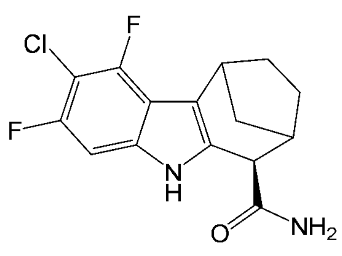 ,
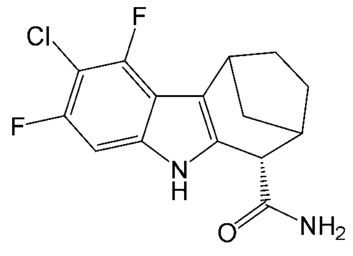 ,

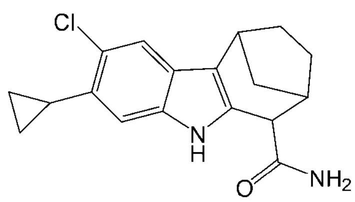 ,
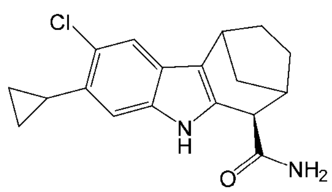 ,
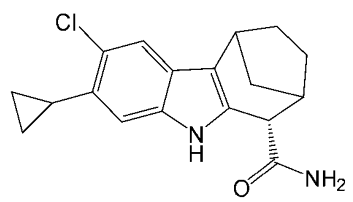 ,

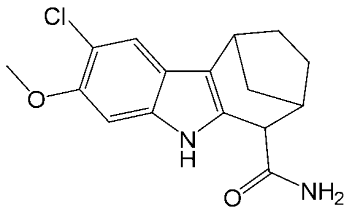 , 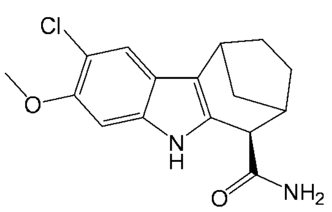 , 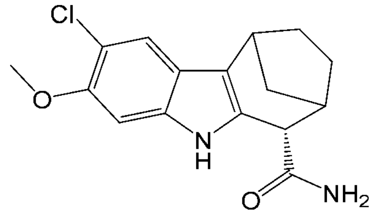 ,

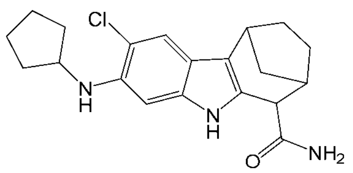 , 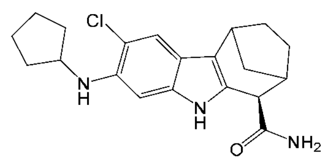 , 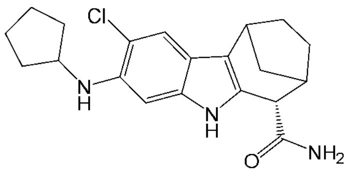 ,

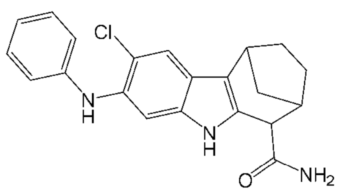 , 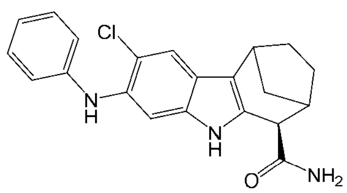 , 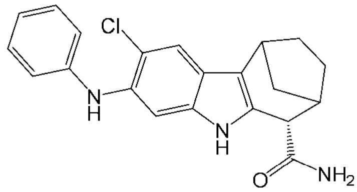 ,

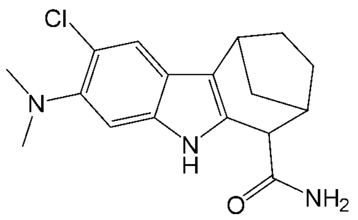 , 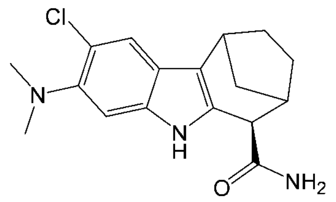 , 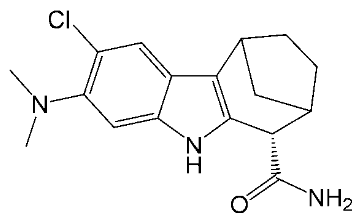 ,

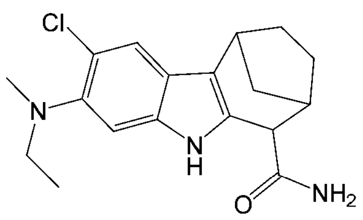 , 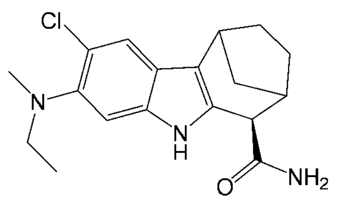 , 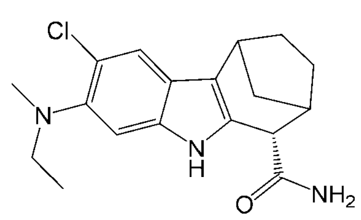 ,

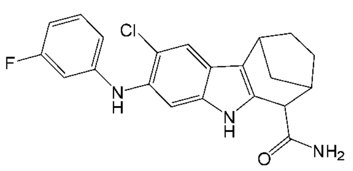 , 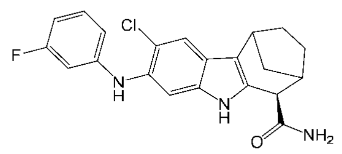 , 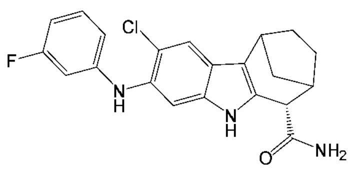 ,

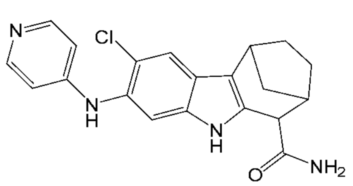 , 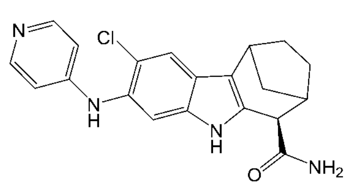 , 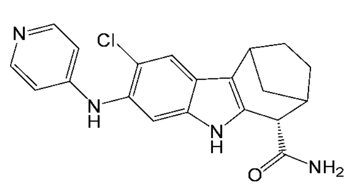 ,

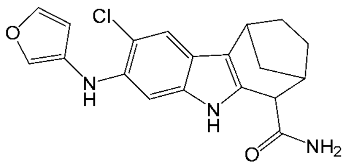 , 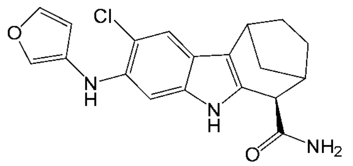 , 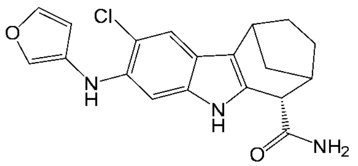 ,

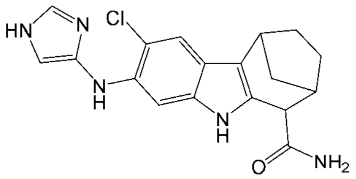 , 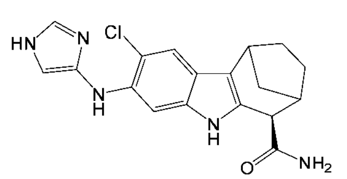 , 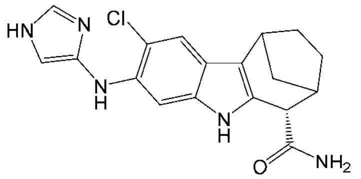 ,

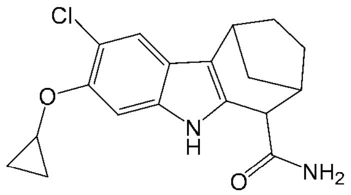 , 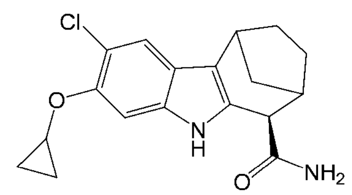 , 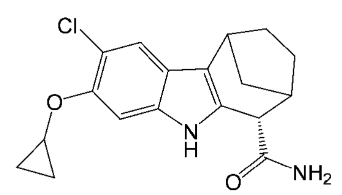 ,

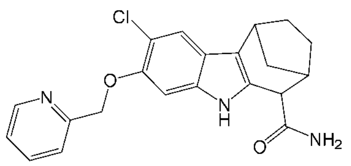 , 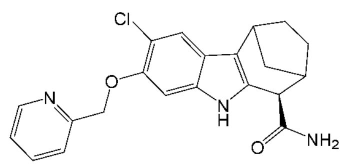 , 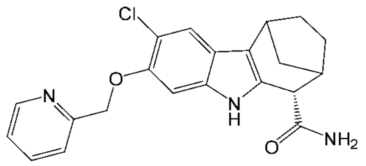 ,

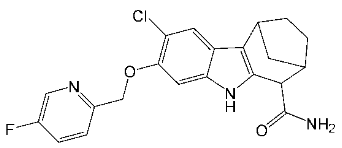 , 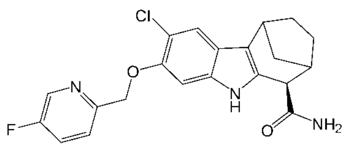 , 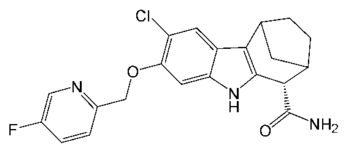 ,

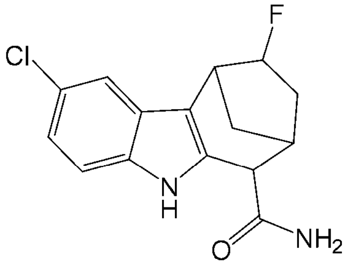 , 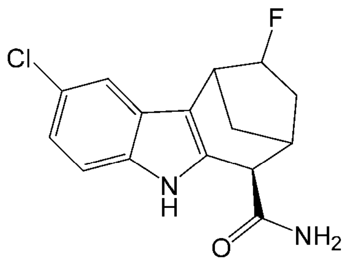 , 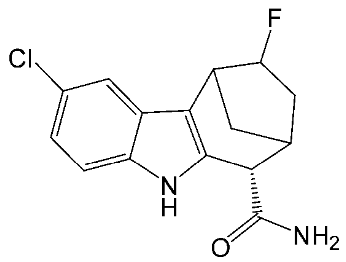 ,

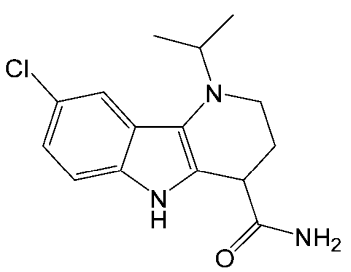 , 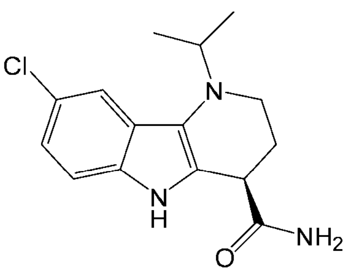 , 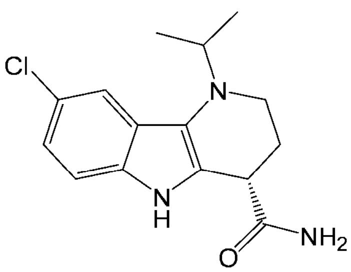 ,

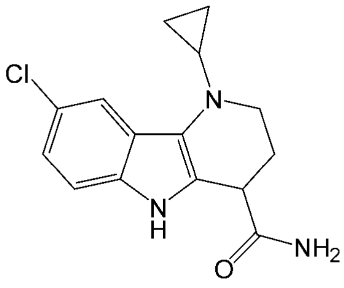 , 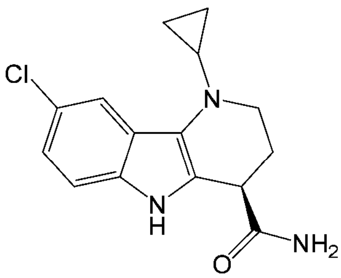 , 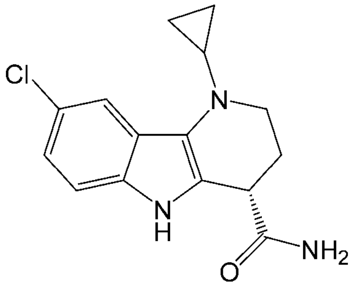 ,

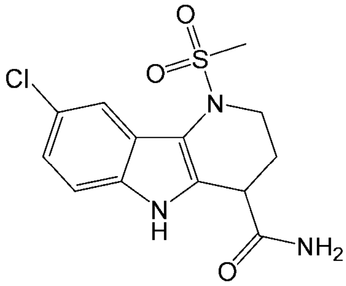 , 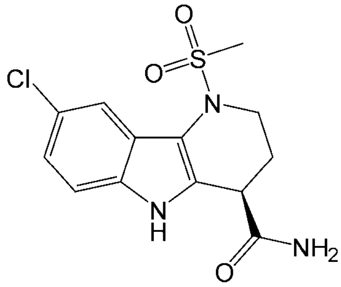 , 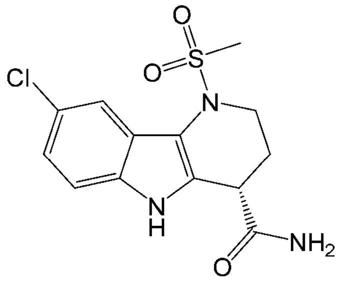 ,

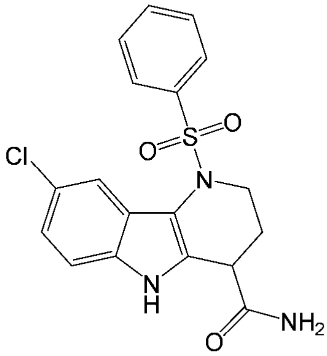 , 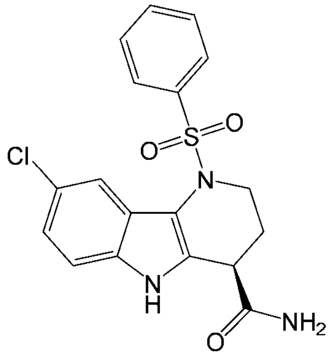 , 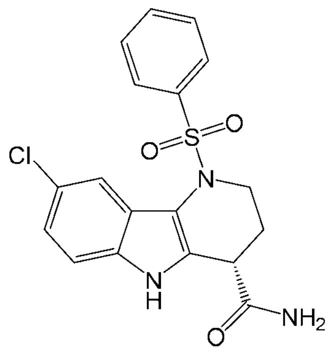 ,

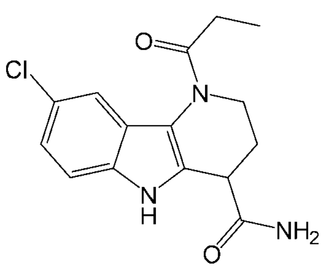 , 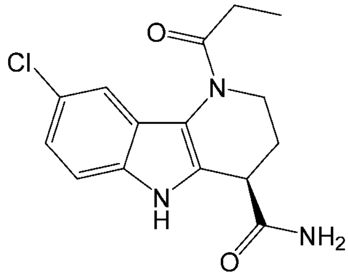 , 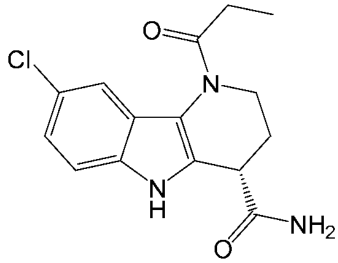 ,

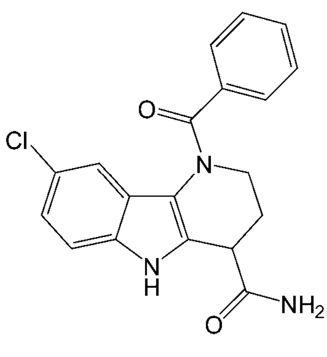 , 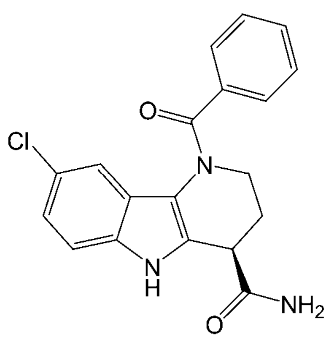 , 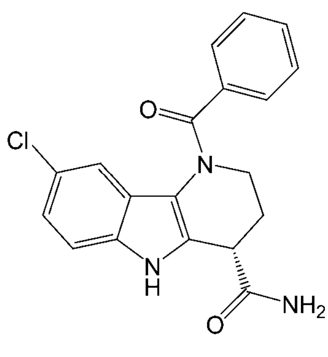 ,

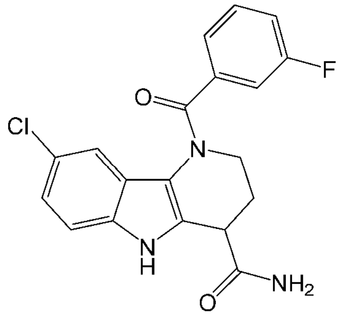 , 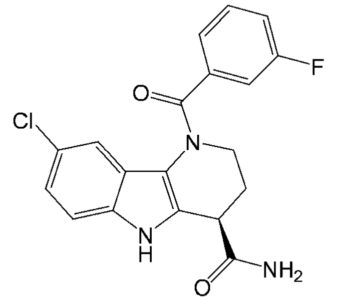 , 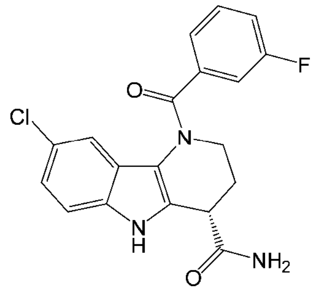 ,

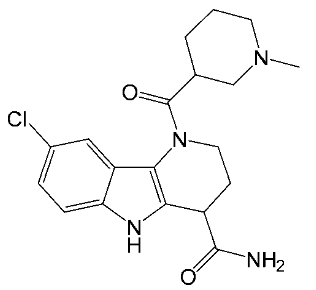 , 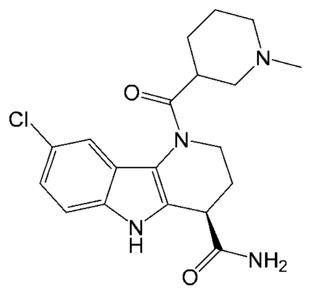 , 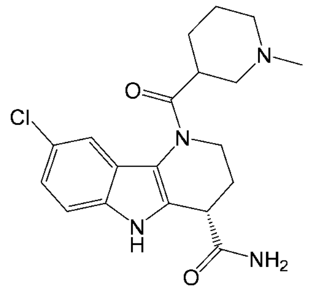 ,

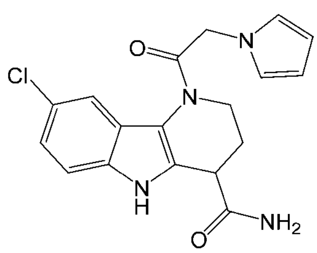 , 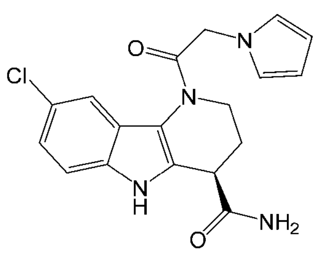 , 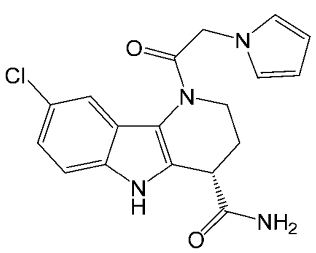 ,

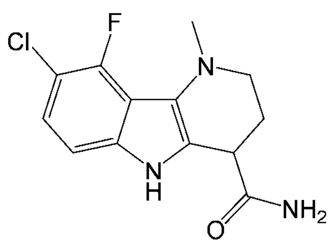 , 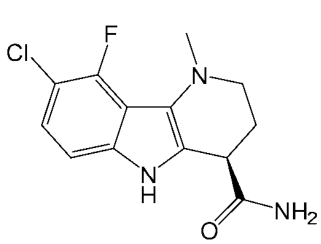 , 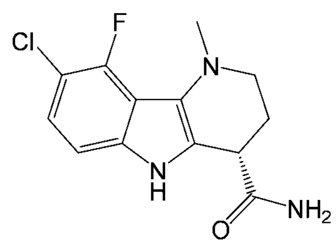 ,

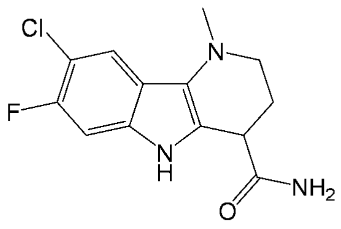 , 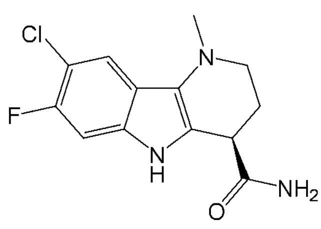 , 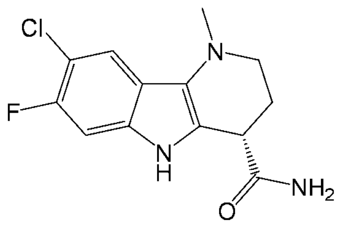 ,

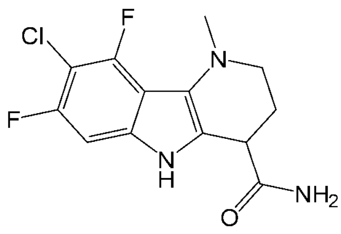 , 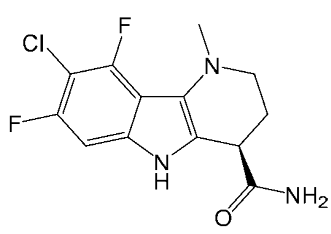 , 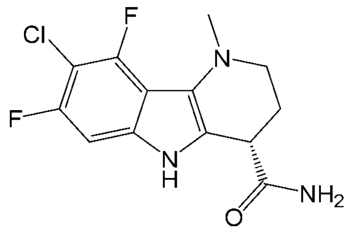 ,

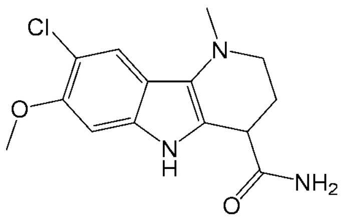 , 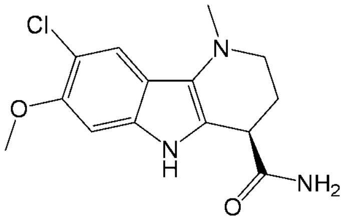 , 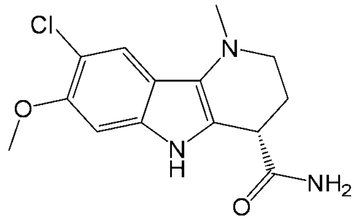 ,

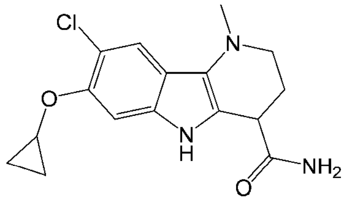 , 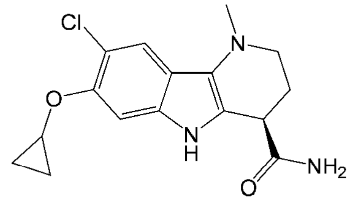 , 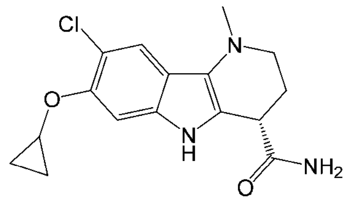 ,

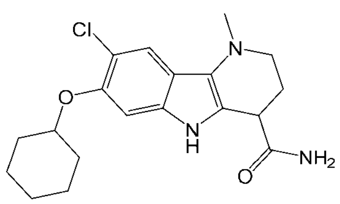 , 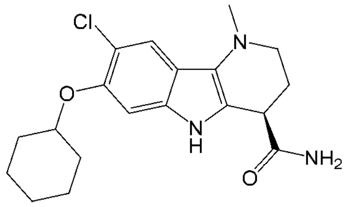 , 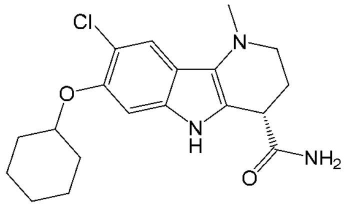 ,

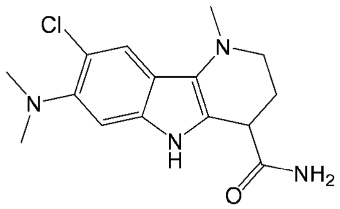 , 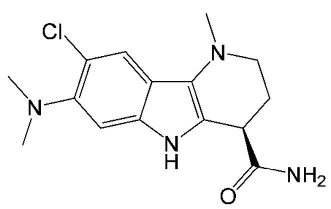 , 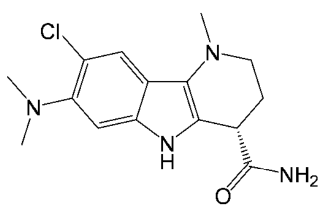 ,

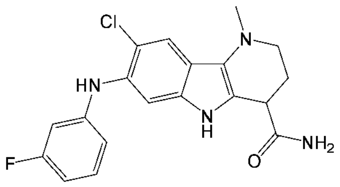 , 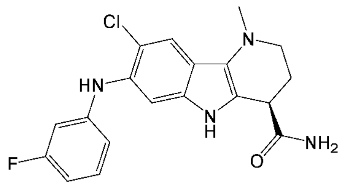 , 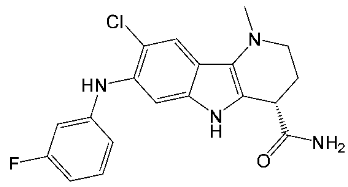 ,

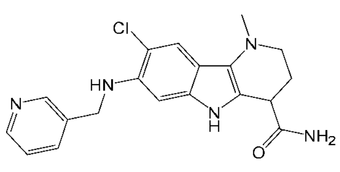 , 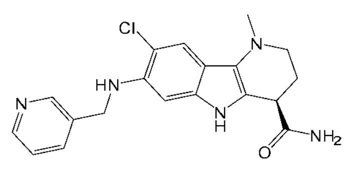 , 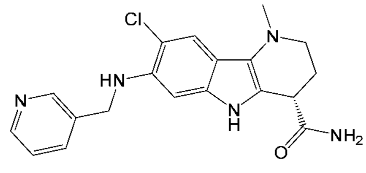 ,

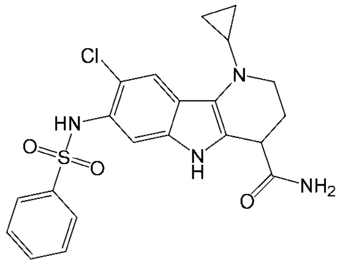 , 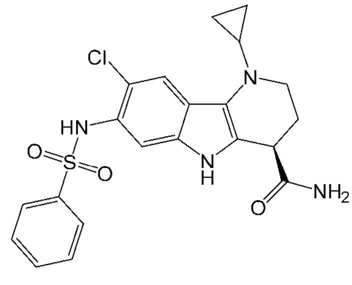 , 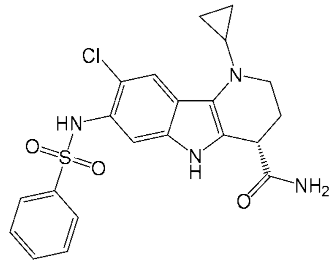 ,

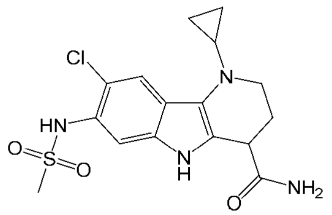 , 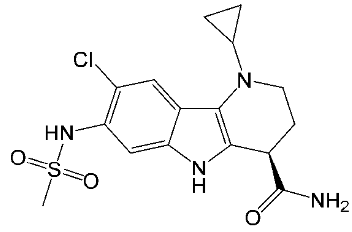 , 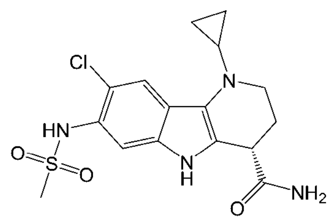 ,

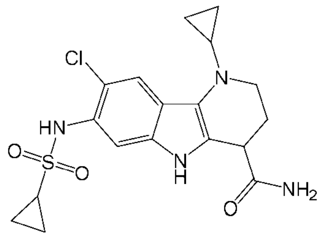 , 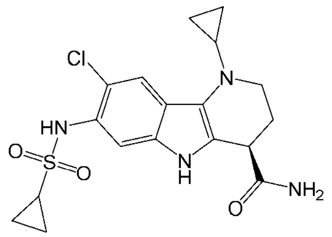 , 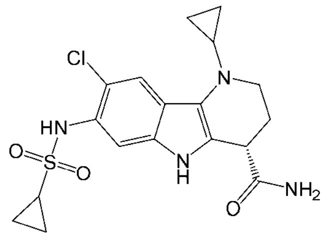 ,

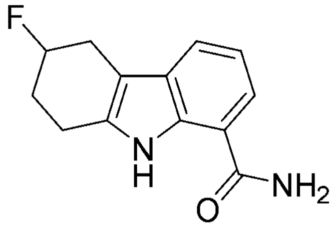 , 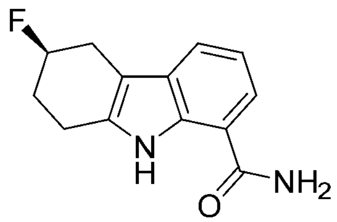 , 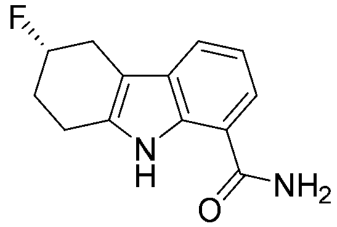 ,

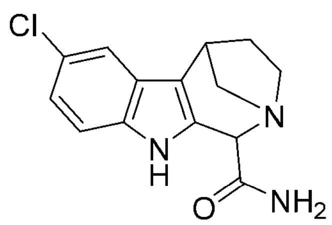 , 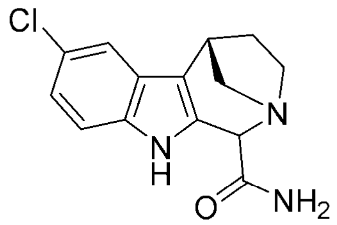 , 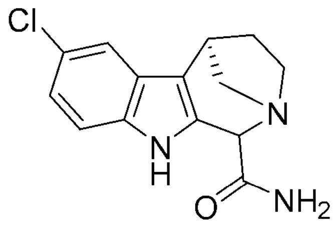 ,

and

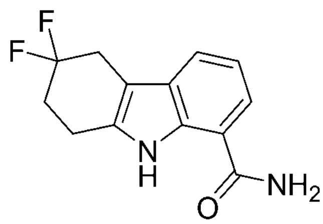 .

**32.** A pharmaceutical composition, comprising the compound, or the stereoisomer, the tautomer, the solvate, the hydrate, the prodrug, the stable isotope derivative, and the pharmaceutically acceptable salt thereof according to any one of claims 1-31.

**33.** The compound, or the stereoisomer, the tautomer, the solvate, the hydrate, the prodrug, the stable isotope derivative, and the pharmaceutically acceptable salt thereof according to any one of claims 1-31 or the pharmaceutical composition according to claim 32, for use in prevention and/or treatment of a skin-related disease, wherein

preferably, the skin-related disease is selected from a skin hyperkeratosis-related disease;
preferably, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis;

preferably, the hand-foot syndrome is caused by administration of a group consisting of one or more selected from docetaxel, capecitabine, fluorouracil, paclitaxel, cytarabine, adriamycin, oxaliplatin, and doxorubicin, and preferably caused by administration of a group consisting of one or more selected from docetaxel and capecitabine; and

preferably, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor, preferably caused by administration of a group consisting of one or more selected from an EGFR inhibitor and a VEGFR inhibitor, and preferably caused by administration of a group consisting of one or more selected from sorafenib, lenvatinib, axitinib, donafenib, regorafenib, apatinib, fruquintinib, anlotinib, erlotinib, afatinib, and osimertinib.

**34.** Use of the compound, or the stereoisomer, the tautomer, the solvate, the hydrate, the prodrug, the stable isotope derivative, and the pharmaceutically acceptable salt thereof according to any one of claims 1-31 or the pharmaceutical composition according to claim 32 in prevention and/or treatment of a skin-related disease, wherein

preferably, the skin-related disease is selected from a skin hyperkeratosis-related disease;

preferably, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis;

preferably, the hand-foot syndrome is caused by administration of a group consisting of one or more selected from docetaxel, capecitabine, fluorouracil, paclitaxel, cytarabine, adriamycin, oxaliplatin, and doxorubicin, and preferably caused by administration of a group consisting of one or more selected from docetaxel and capecitabine; and

preferably, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor, preferably caused by administration of a group consisting of one or more selected from an EGFR inhibitor and a VEGFR inhibitor, and preferably caused by administration of a group consisting of one or more selected from sorafenib, lenvatinib, axitinib, donafenib, regorafenib, apatinib, fruquintinib, anlotinib, erlotinib, afatinib, and osimertinib.

**35.** Use of the compound, or the stereoisomer, the tautomer, the solvate, the hydrate, the prodrug, the stable isotope derivative, and the pharmaceutically acceptable salt thereof according to any one of claims 1-31 or the pharmaceutical composition according to claim 32 in preparation of a drug for preventing and/or treating a skin-related disease, wherein

preferably, the skin-related disease is selected from a skin hyperkeratosis-related disease;

preferably, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis;

preferably, the hand-foot syndrome is caused by administration of a group consisting of one or more selected from docetaxel, capecitabine, fluorouracil, paclitaxel, cytarabine, adriamycin, oxaliplatin, and doxorubicin, and preferably caused by administration of a group consisting of one or more selected from docetaxel and capecitabine; and

preferably, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor, preferably caused by administration of a group consisting of one or more selected from an EGFR inhibitor and a VEGFR inhibitor, and preferably caused by administration of a group consisting of one or more selected from sorafenib, lenvatinib, axitinib, donafenib, regorafenib, apatinib, fruquintinib, anlotinib, erlotinib, afatinib, and osimertinib.

**36.** The compound, or the stereoisomer, the tautomer, the solvate, the hydrate, the prodrug, the stable isotope derivative, and the pharmaceutically acceptable salt thereof according to any one of claims 1-31 or the pharmaceutical composition according to claim 32, for use in prevention and/or treatment of an abnormal RNA m6A methylation-related disease, wherein

preferably, the abnormal RNA m6A methylation-related disease is selected from a skin-related disease;
preferably, the skin-related disease is selected from a skin hyperkeratosis-related disease;
preferably, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis;
preferably, the hand-foot syndrome is caused by administration of a group consisting of one or more selected from docetaxel, capecitabine, fluorouracil, paclitaxel, cytarabine, adriamycin, oxaliplatin, and doxorubicin, and preferably caused by administration of a group consisting of one or more selected from docetaxel and capecitabine; and
preferably, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor, preferably caused by administration of a group consisting of one or more selected from an EGFR inhibitor and a VEGFR inhibitor, and preferably caused by administration of a group consisting of one or more selected from sorafenib, lenvatinib, axitinib, donafenib, regorafenib, apatinib, fruquintinib, anlotinib, erlotinib, afatinib, and osimertinib.

**37.** Use of the compound, or the stereoisomer, the tautomer, the solvate, the hydrate, the prodrug, the stable isotope derivative, and the pharmaceutically acceptable salt thereof according to any one of claims 1-31 or the pharmaceutical composition according to claim 32 in prevention and/or treatment of an abnormal RNA m6A methylation-related disease, wherein

preferably, the abnormal RNA m6A methylation-related disease is selected from a skin-related disease;
preferably, the skin-related disease is selected from a skin hyperkeratosis-related disease;
preferably, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis;
preferably, the hand-foot syndrome is caused by administration of a group consisting of one or more selected from docetaxel, capecitabine, fluorouracil, paclitaxel, cytarabine, adriamycin, oxaliplatin, and doxorubicin, and preferably caused by administration of a group consisting of one or more selected from docetaxel and capecitabine; and
preferably, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor, preferably caused by administration of a group consisting of one or more selected from an EGFR inhibitor and a VEGFR inhibitor, and preferably caused by administration of a group consisting of one or more selected from sorafenib, lenvatinib, axitinib, donafenib, regorafenib, apatinib, fruquintinib, anlotinib, erlotinib, afatinib, and osimertinib.

**38.** Use of the compound, or the stereoisomer, the tautomer, the solvate, the hydrate, the prodrug, the stable isotope derivative, and the pharmaceutically acceptable salt thereof according to any one of claims 1-31 or the pharmaceutical composition according to claim 32 in preparation of a drug for preventing and/or treating an abnormal RNA m6A methylation-related disease, wherein

preferably, the abnormal RNA m6A methylation-related disease is selected from a skin-related disease;
preferably, the skin-related disease is selected from a skin hyperkeratosis-related disease;
preferably, the skin hyperkeratosis-related disease is selected from an abnormal skin keratosis disease; and preferably selected from rash, pruritus, folliculitis, paronychia, pigmentation disorder, desquamation, acne, urticaria, acanthosis, eczema, ichthyosis, psoriasis, keratosis, systemic lupus erythematosus, hand-foot syndrome, hand-foot skin reaction, oral ulcer, and dermatitis;
preferably, the hand-foot syndrome is caused by administration of a group consisting of one or more selected from docetaxel, capecitabine, fluorouracil, paclitaxel, cytarabine, adriamycin, oxaliplatin, and doxorubicin, and preferably caused by administration of a group consisting of one or more selected from docetaxel and capecitabine; and
preferably, the hand-foot skin reaction is caused by administration of a group consisting of one or more selected from an EGFR inhibitor, a VEGFR inhibitor, c-kit, PDGFR, an ALK inhibitor, an RET inhibitor, and an FGFR inhibitor, preferably caused by administration of a group consisting of one or more selected from an EGFR inhibitor and a VEGFR inhibitor, and preferably caused by administration of a group consisting of one or more

selected from sorafenib, lenvatinib, axitinib, donafenib, regorafenib, apatinib, fruquintinib, anlotinib, erlotinib, afatinib, and osimertinib.

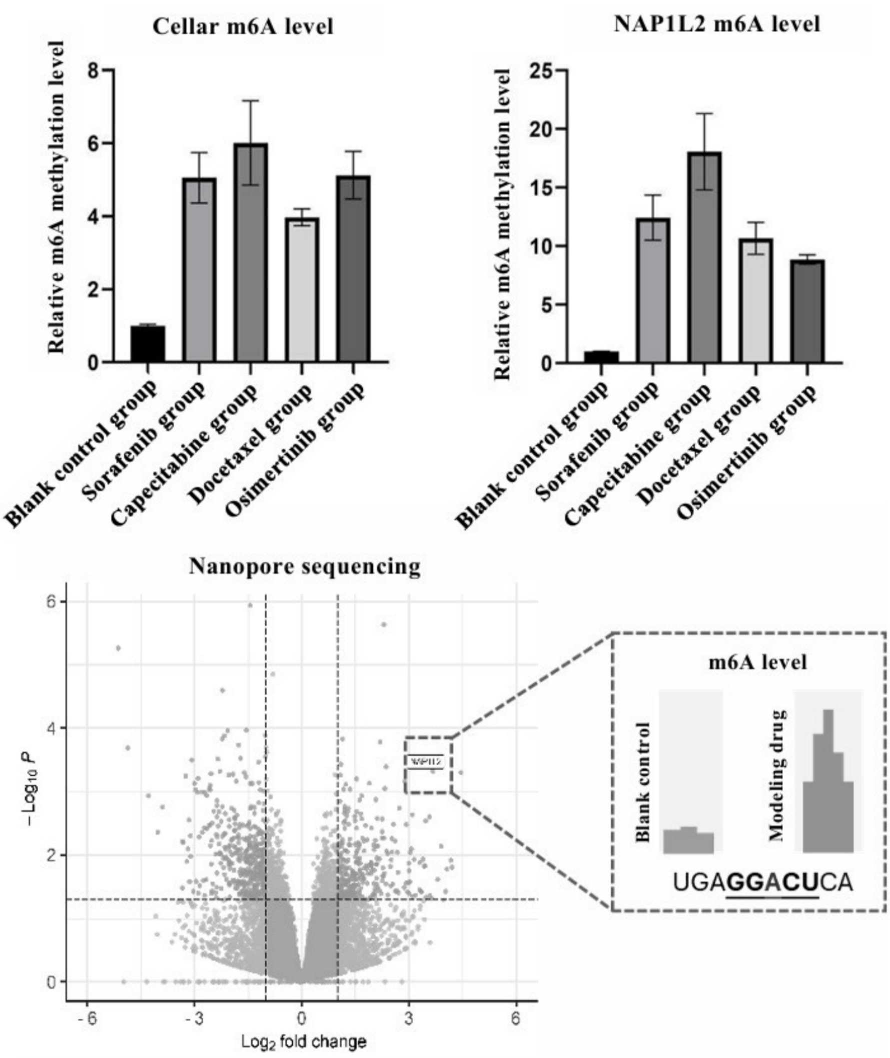
Cellar m6A level
Relative m6A methylation level
0
2
4
6
8
Blank control group
Sorafenib group
Capecitabine group
Docetaxel group
Osimertinib group
NAP1L2 m6A level
Relative m6A methylation level
0
5
10
15
20
25
Blank control group
Sorafenib group
Capecitabine group
Docetaxel group
Osimertinib group
Nanopore sequencing
$-\mathrm{Log}_{10}\,P$
0
2
4
6
-6
-3
0
3
6
$\mathrm{Log}_2$ fold change
m6A level
Blank control
Modeling drug
UGAGGACUCA

FIG. 1

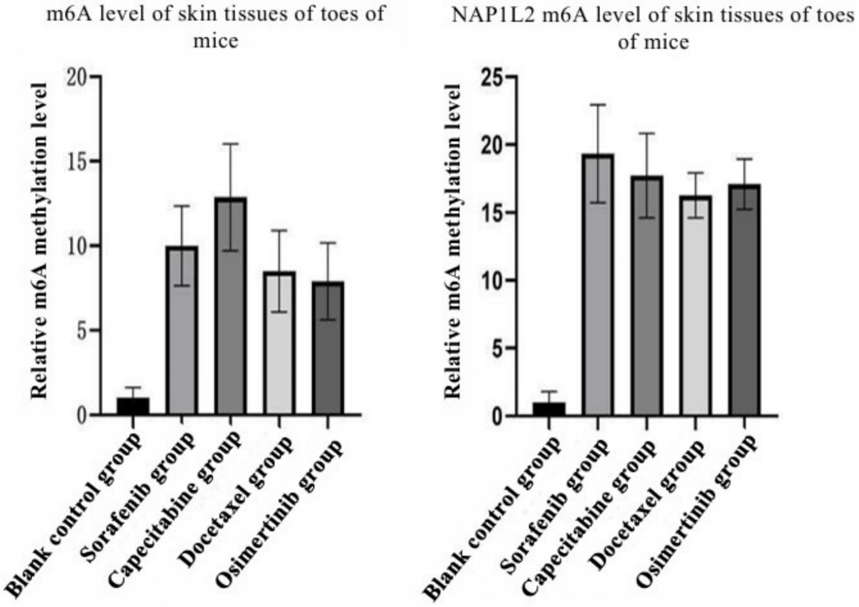
m6A level of skin tissues of toes of mice
Relative m6A methylation level
0
5
10
15
20
Blank control group
Sorafenib group
Capecitabine group
Docetaxel group
Osimertinib group
NAP1L2 m6A level of skin tissues of toes of mice
Relative m6A methylation level
0
5
10
15
20
25
Blank control group
Sorafenib group
Capecitabine group
Docetaxel group
Osimertinib group

FIG. 2

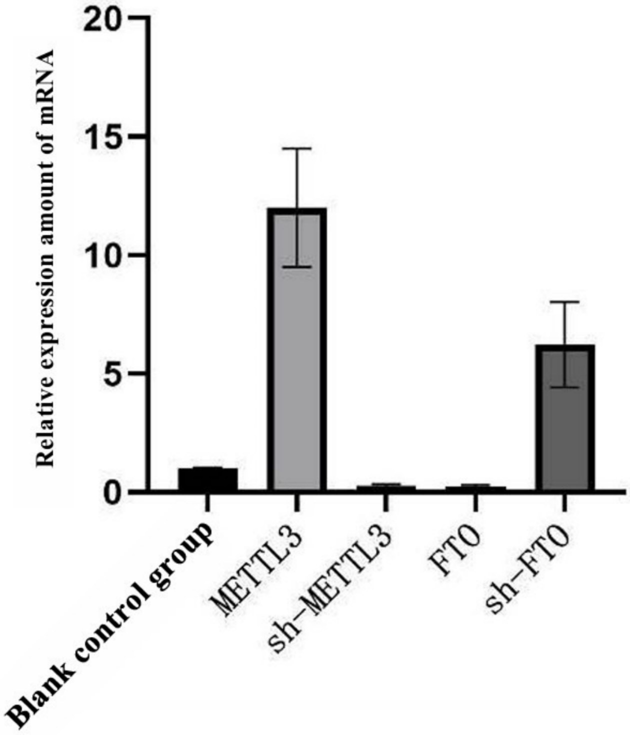
Relative expression amount of mRNA
20
15
10
5
0
Blank control group
METTL3
sh-METTL3
FTO
sh-FTO

FIG. 3

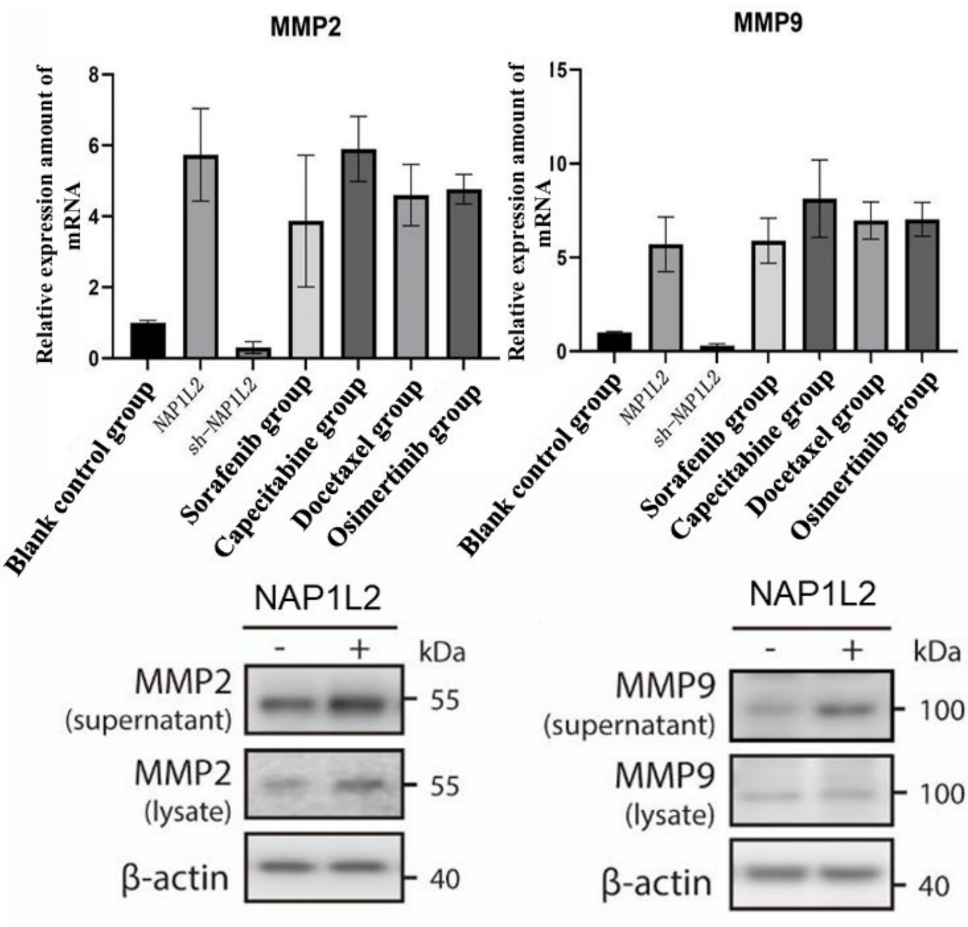
MMP2
Relative expression amount of mRNA
8
6
4
2
0
Blank control group
NAP1L2
sh-NAP1L2
Sorafenib group
Capecitabine group
Docetaxel group
Osimertinib group
MMP9
Relative expression amount of mRNA
15
10
5
0
Blank control group
NAP1L2
sh-NAP1L2
Sorafenib group
Capecitabine group
Docetaxel group
Osimertinib group
NAP1L2
-
+
kDa
MMP2 (supernatant)
55
MMP2 (lysate)
55
β-actin
40
NAP1L2
-
+
kDa
MMP9 (supernatant)
100
MMP9 (lysate)
100
β-actin
40

FIG. 4

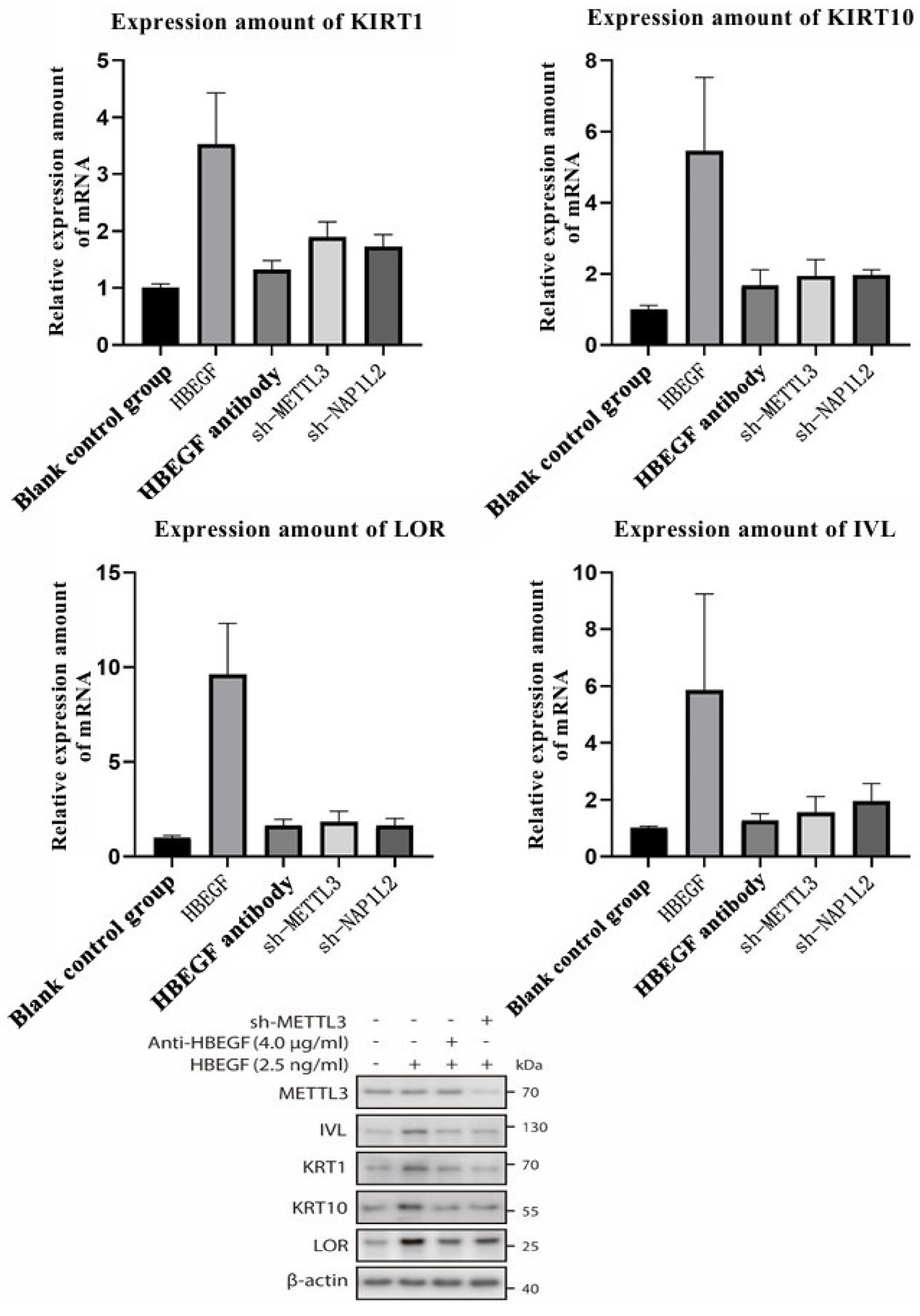
Expression amount of KIRT1
Relative expression amount of mRNA
0
1
2
3
4
5
Blank control group
HBEGF
HBEGF antibody
sh-METTL3
sh-NAP1L2
Expression amount of KIRT10
Relative expression amount of mRNA
0
2
4
6
8
Blank control group
HBEGF
HBEGF antibody
sh-METTL3
sh-NAP1L2
Expression amount of LOR
Relative expression amount of mRNA
0
5
10
15
Blank control group
HBEGF
HBEGF antibody
sh-METTL3
sh-NAP1L2
Expression amount of IVL
Relative expression amount of mRNA
0
2
4
6
8
10
Blank control group
HBEGF
HBEGF antibody
sh-METTL3
sh-NAP1L2
sh-METTL3 - - - +
Anti-HBEGF (4.0 μg/ml) - - + -
HBEGF (2.5 ng/ml) - + + +
kDa
METTL3
70
IVL
130
KRT1
70
KRT10
55
LOR
25
β-actin
40

FIG. 5

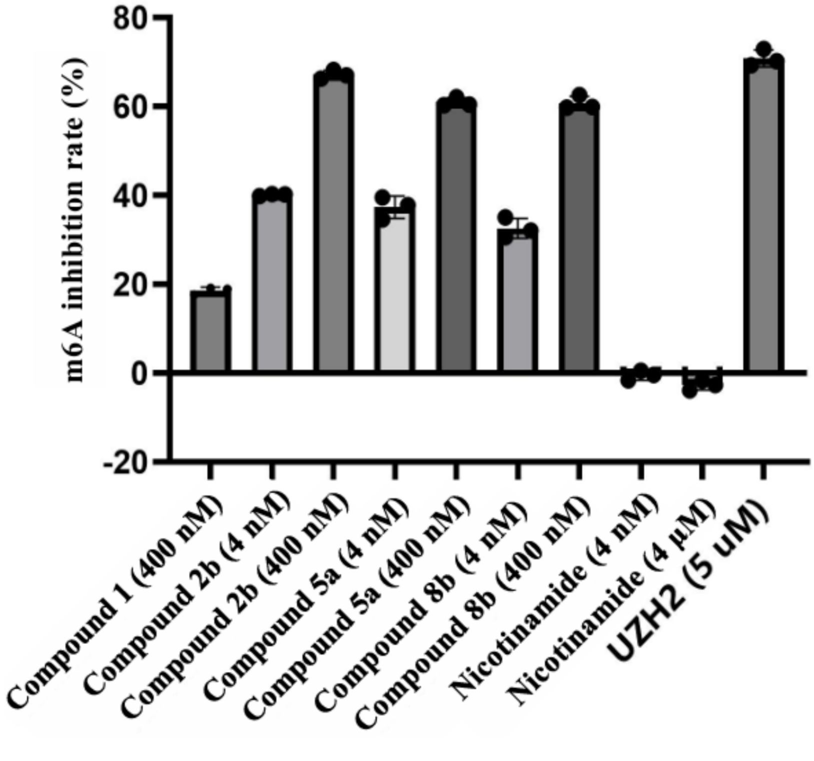
m6A inhibition rate (%)
80
60
40
20
0
-20
Compound 1 (400 nM)
Compound 2b (4 nM)
Compound 2b (400 nM)
Compound 5a (4 nM)
Compound 5a (400 nM)
Compound 8b (4 nM)
Compound 8b (400 nM)
Nicotinamide (4 nM)
Nicotinamide (4 μM)
UZH2 (5 uM)

FIG. 6

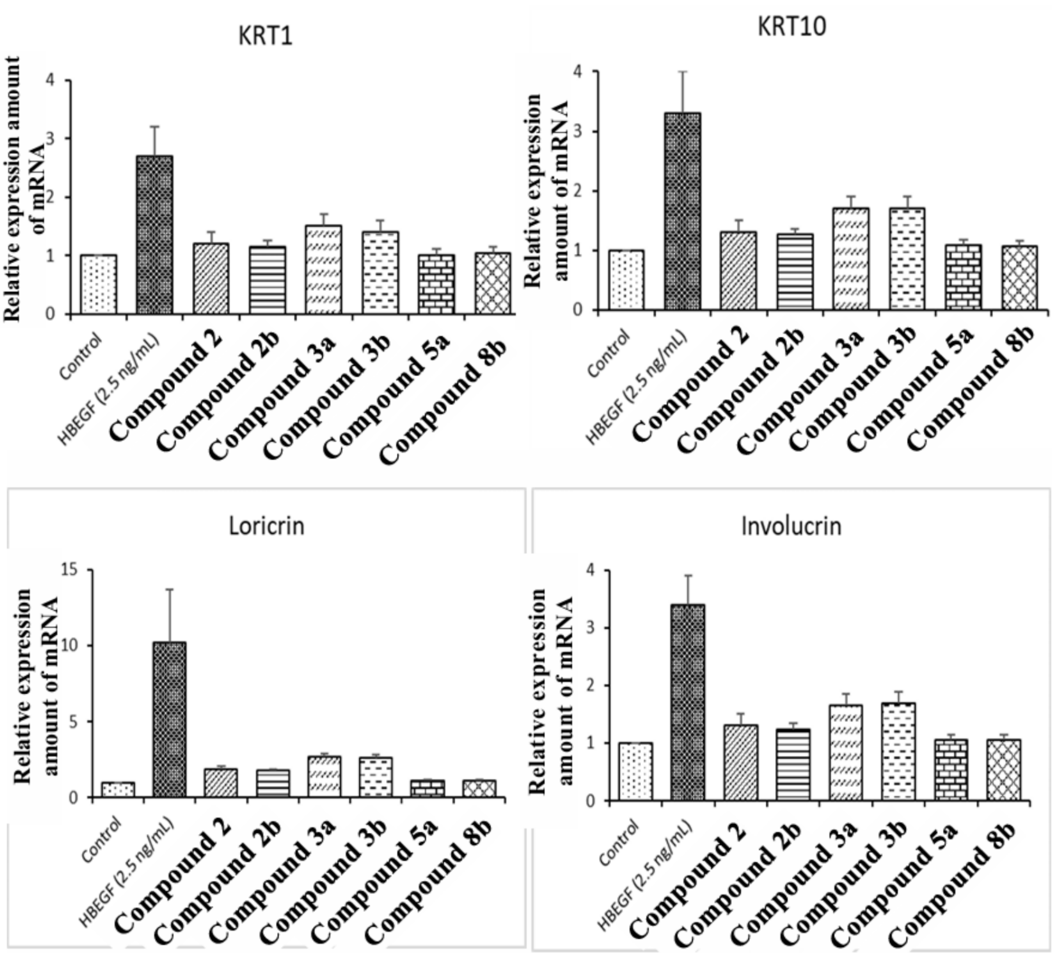
KRT1
KRT10
Loricrin
Involucrin
Relative expression amount of mRNA
Control
HBEGF (2.5 ng/mL)
Compound 2
Compound 2b
Compound 3a
Compound 3b
Compound 5a
Compound 8b
0
1
2
3
4
5
10
15

FIG. 7

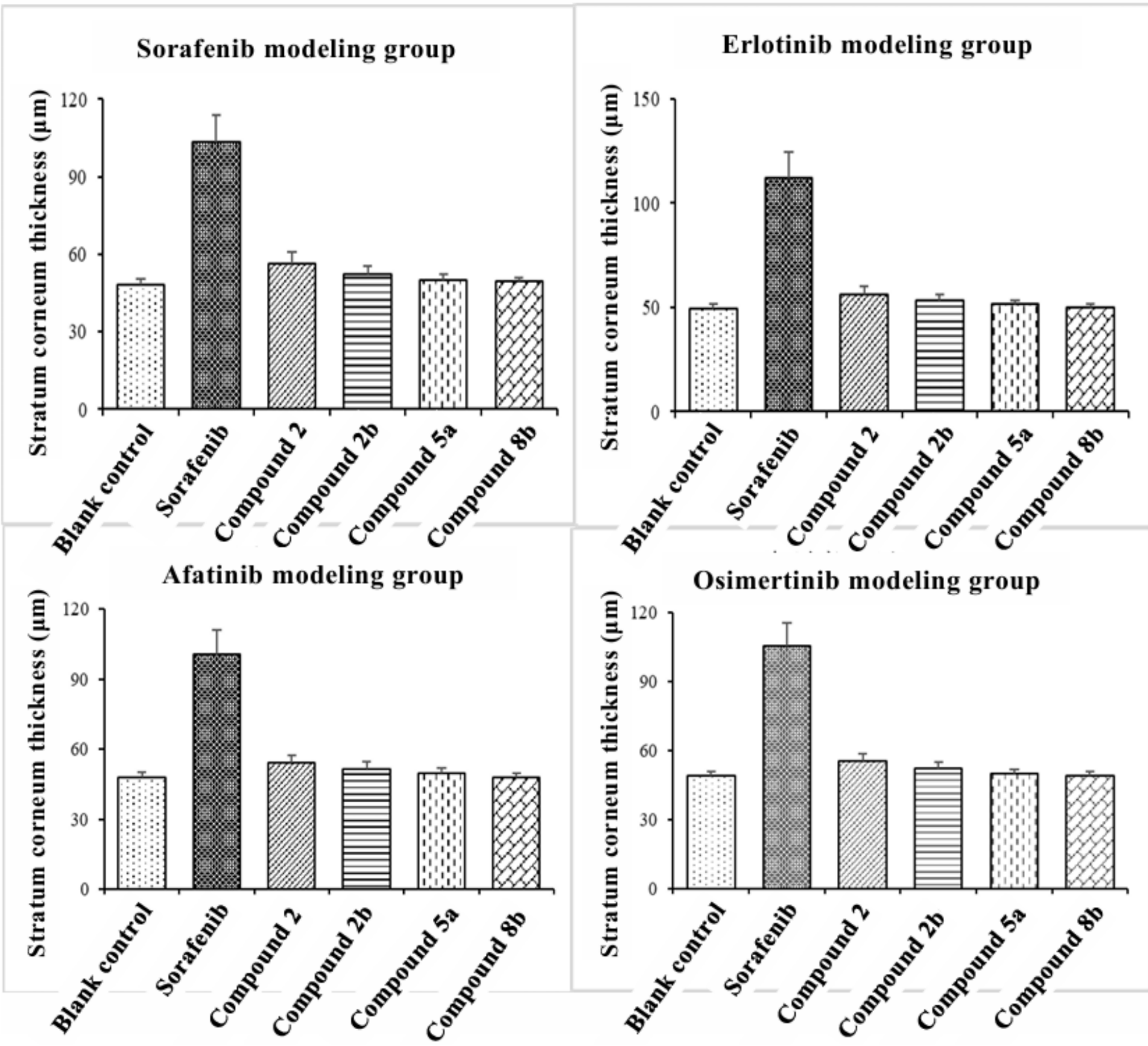
Sorafenib modeling group
Stratum corneum thickness (μm)
0
30
60
90
120
Blank control
Sorafenib
Compound 2
Compound 2b
Compound 5a
Compound 8b
Erlotinib modeling group
Stratum corneum thickness (μm)
0
50
100
150
Blank control
Sorafenib
Compound 2
Compound 2b
Compound 5a
Compound 8b
Afatinib modeling group
Stratum corneum thickness (μm)
0
30
60
90
120
Blank control
Sorafenib
Compound 2
Compound 2b
Compound 5a
Compound 8b
Osimertinib modeling group
Stratum corneum thickness (μm)
0
30
60
90
120
Blank control
Sorafenib
Compound 2
Compound 2b
Compound 5a
Compound 8b

FIG. 8

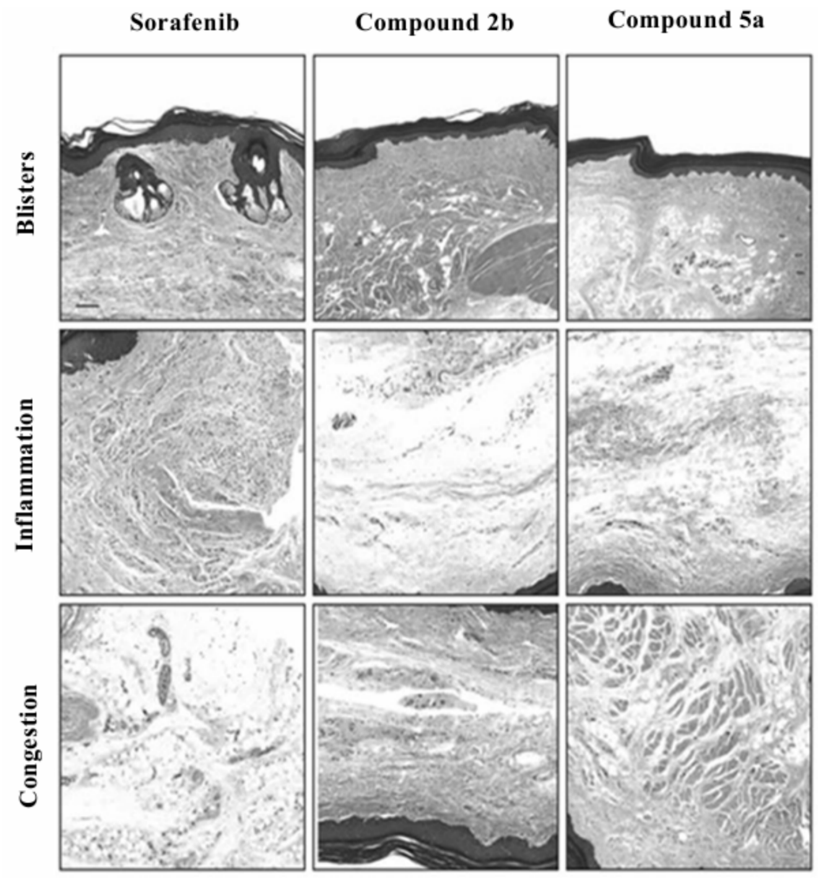
Sorafenib
Compound 2b
Compound 5a
Blisters
Inflammation
Congestion

FIG. 9

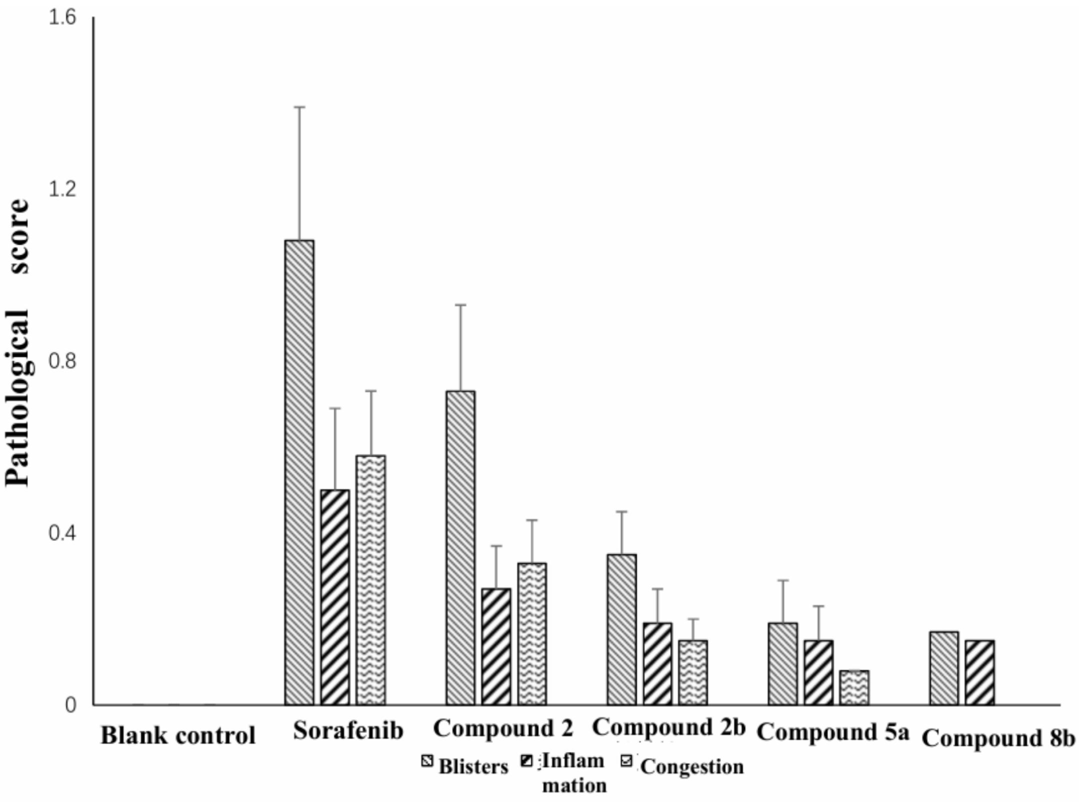
1.6
1.2
0.8
0.4
0
Pathological score
Blank control
Sorafenib
Compound 2
Compound 2b
Compound 5a
Compound 8b
Blisters
Inflam mation
Congestion

FIG. 10

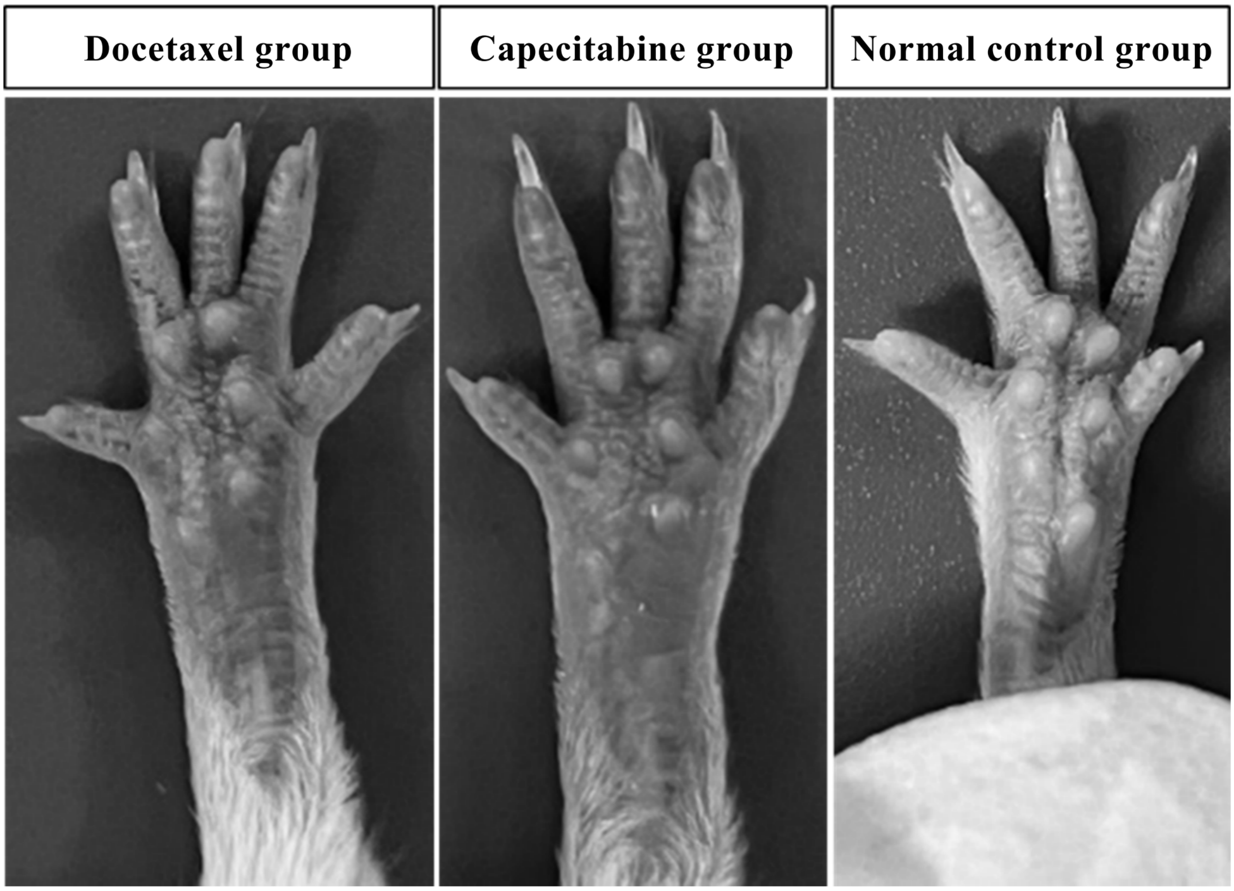


FIG. 11

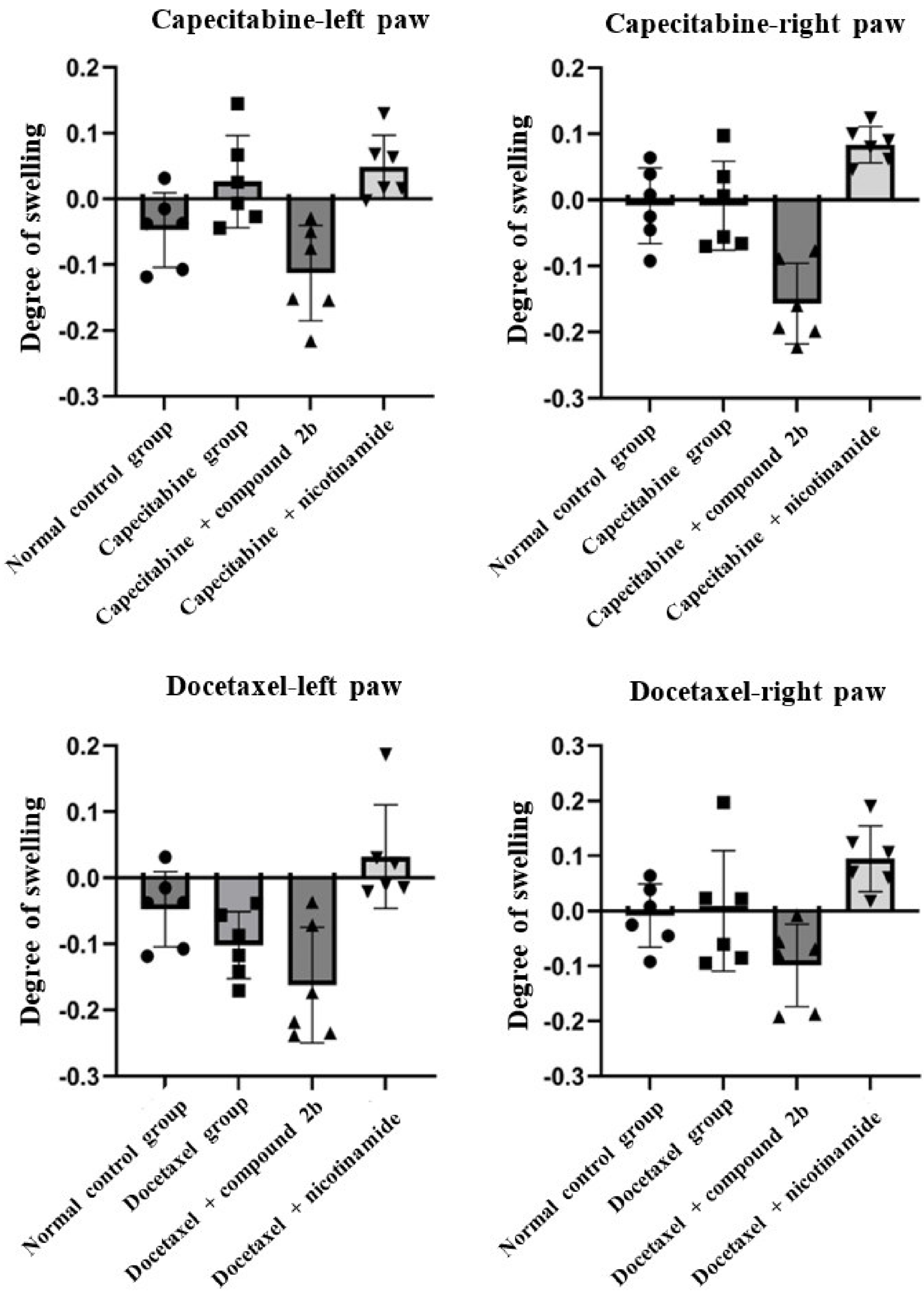
Capecitabine-left paw
Degree of swelling
0.2
0.1
0.0
-0.1
-0.2
-0.3
Normal control group
Capecitabine group
Capecitabine + compound 2b
Capecitabine + nicotinamide
Capecitabine-right paw
Degree of swelling
0.2
0.1
0.0
-0.1
-0.2
-0.3
Normal control group
Capecitabine group
Capecitabine + compound 2b
Capecitabine + nicotinamide
Docetaxel-left paw
Degree of swelling
0.2
0.1
0.0
-0.1
-0.2
-0.3
Normal control group
Docetaxel group
Docetaxel + compound 2b
Docetaxel + nicotinamide
Docetaxel-right paw
Degree of swelling
0.3
0.2
0.1
0.0
-0.1
-0.2
-0.3
Normal control group
Docetaxel group
Docetaxel + compound 2b
Docetaxel + nicotinamide

FIG. 12

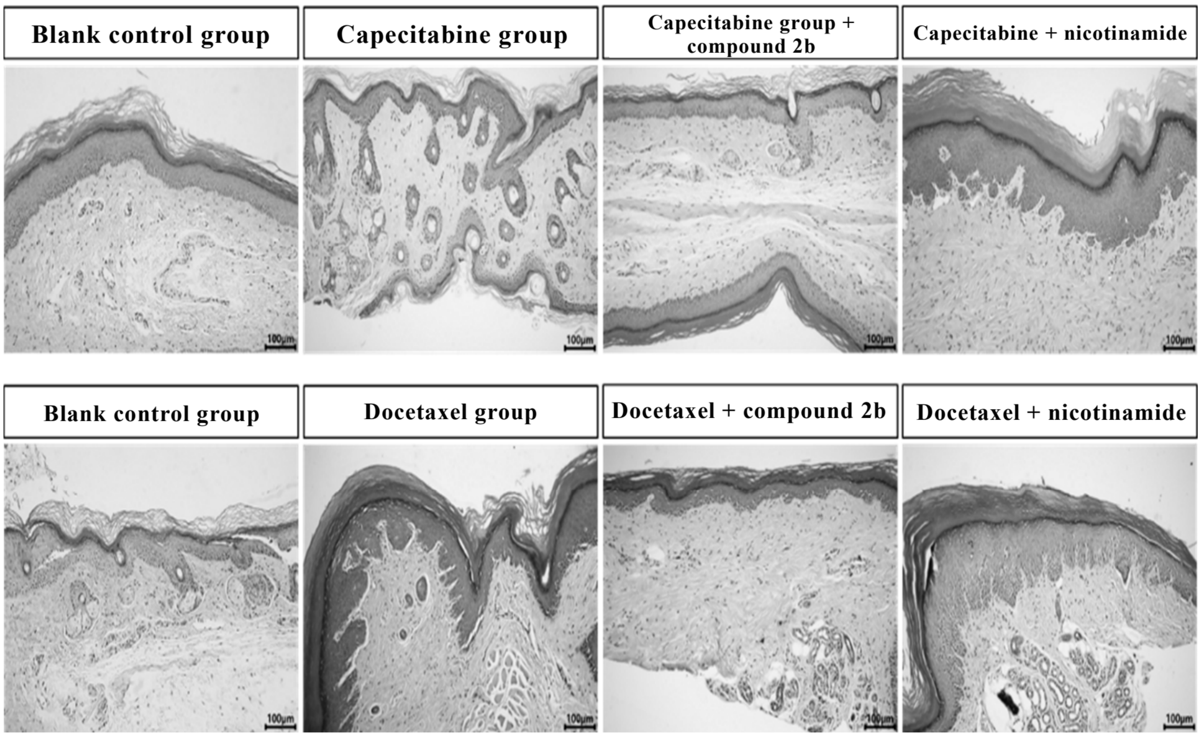
Blank control group
Capecitabine group
Capecitabine group + compound 2b
Capecitabine + nicotinamide
Blank control group
Docetaxel group
Docetaxel + compound 2b
Docetaxel + nicotinamide
100μm

FIG. 13

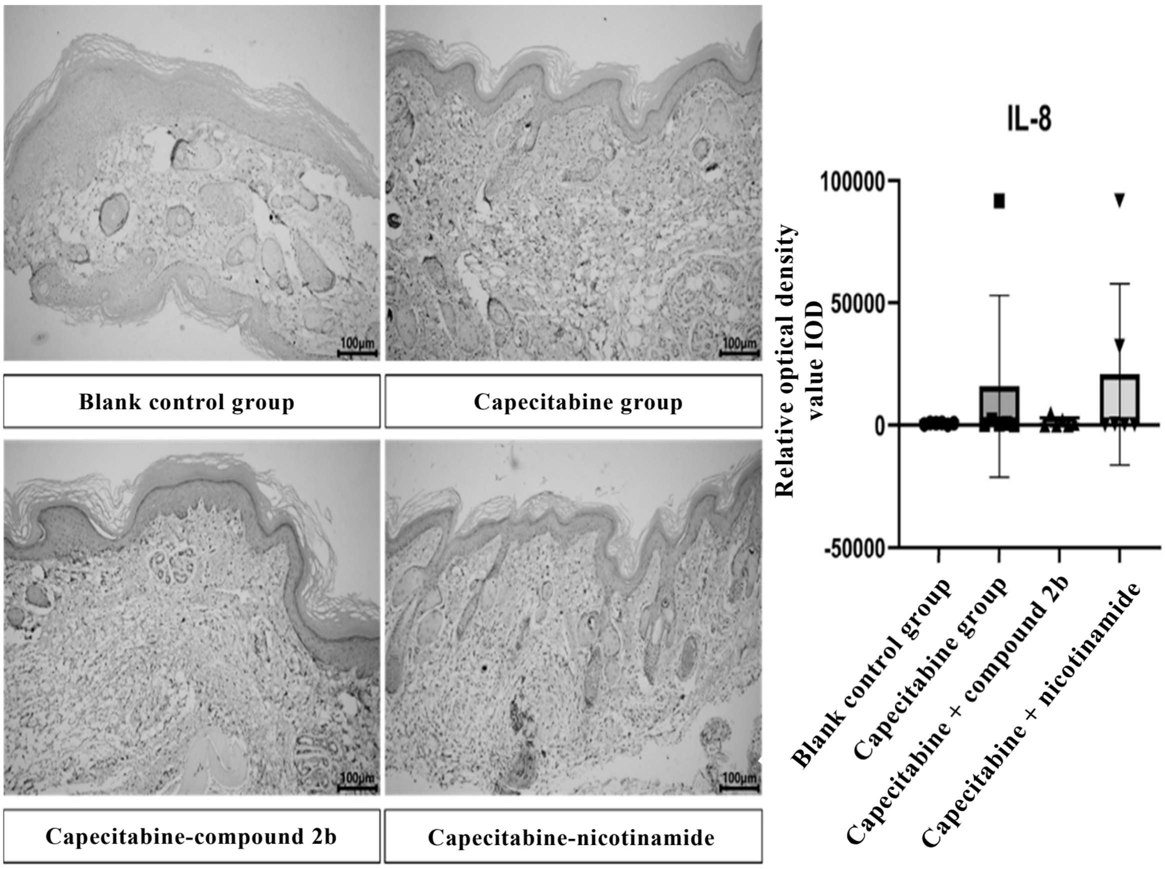
Blank control group
Capecitabine group
Capecitabine-compound 2b
Capecitabine-nicotinamide
100μm
IL-8
Relative optical density value IOD
100000
50000
0
-50000
Blank control group
Capecitabine group
Capecitabine + compound 2b
Capecitabine + nicotinamide

FIG. 14

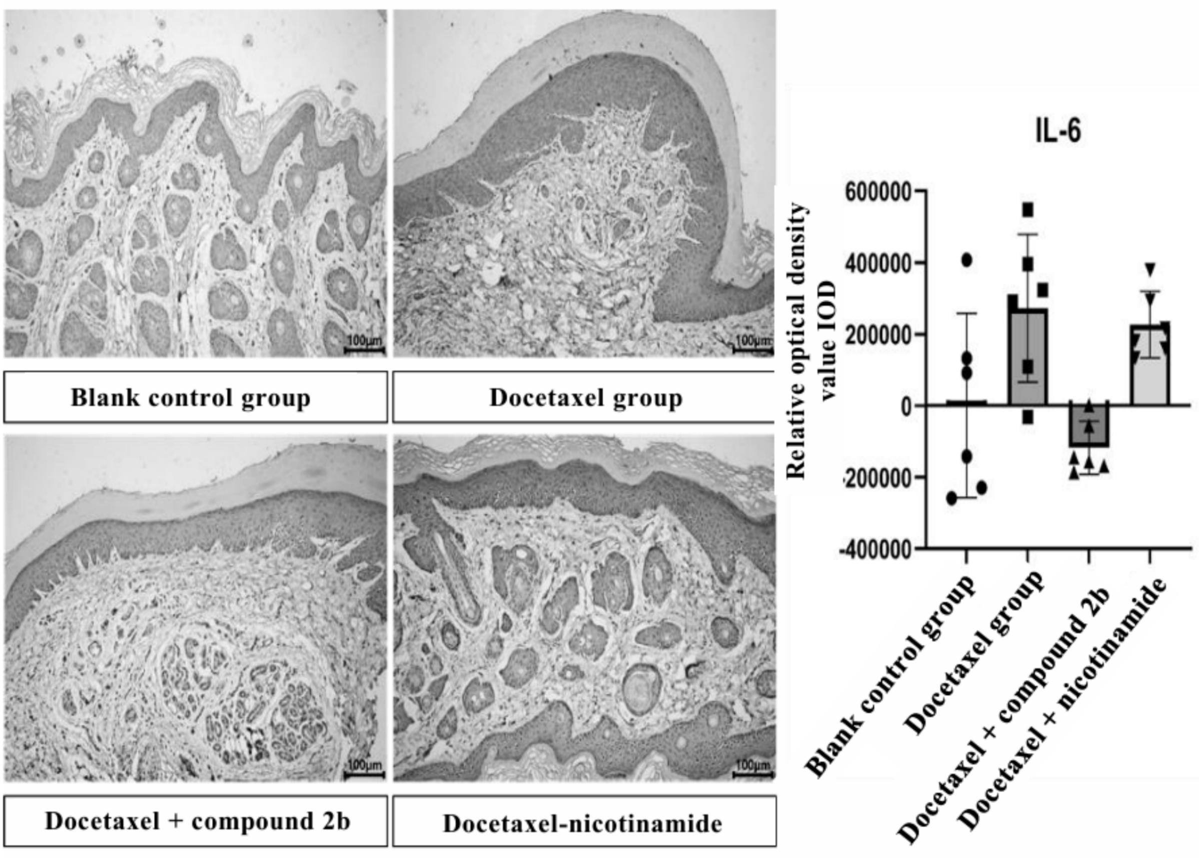
Blank control group
Docetaxel group
Docetaxel + compound 2b
Docetaxel-nicotinamide
100μm
IL-6
Relative optical density value IOD
600000
400000
200000
0
-200000
-400000
Blank control group
Docetaxel group
Docetaxel + compound 2b
Docetaxel + nicotinamide

FIG. 15

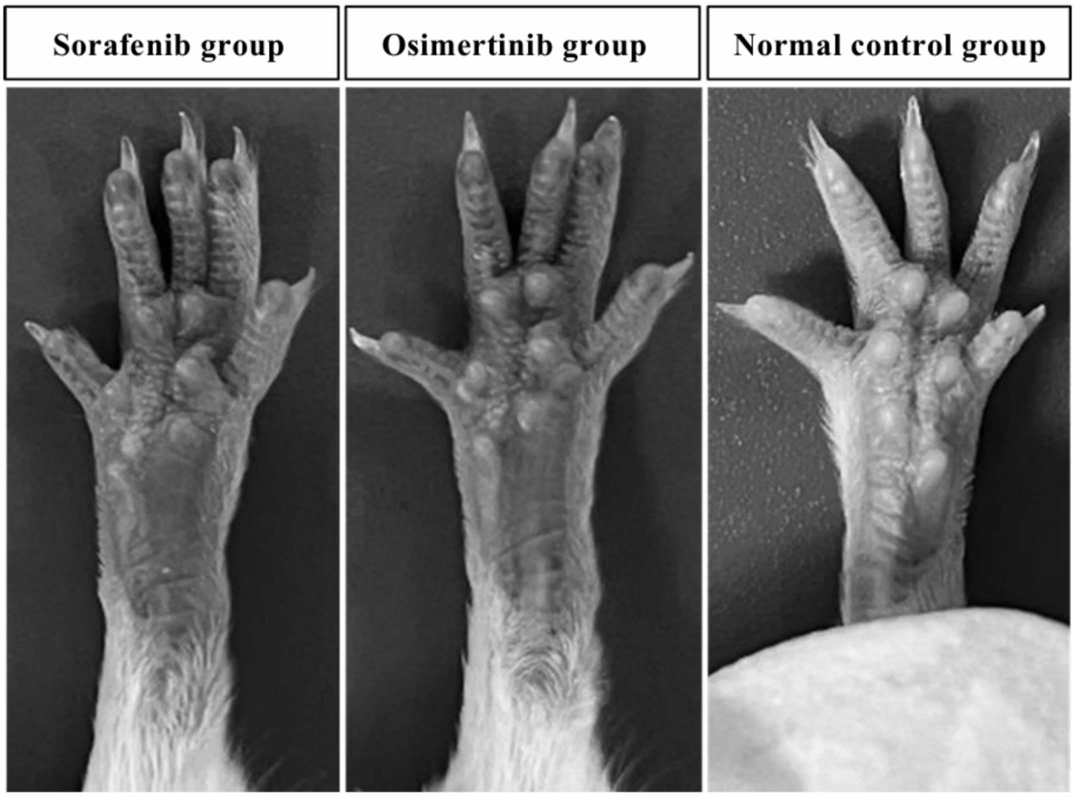
Sorafenib group
Osimertinib group
Normal control group

FIG. 16

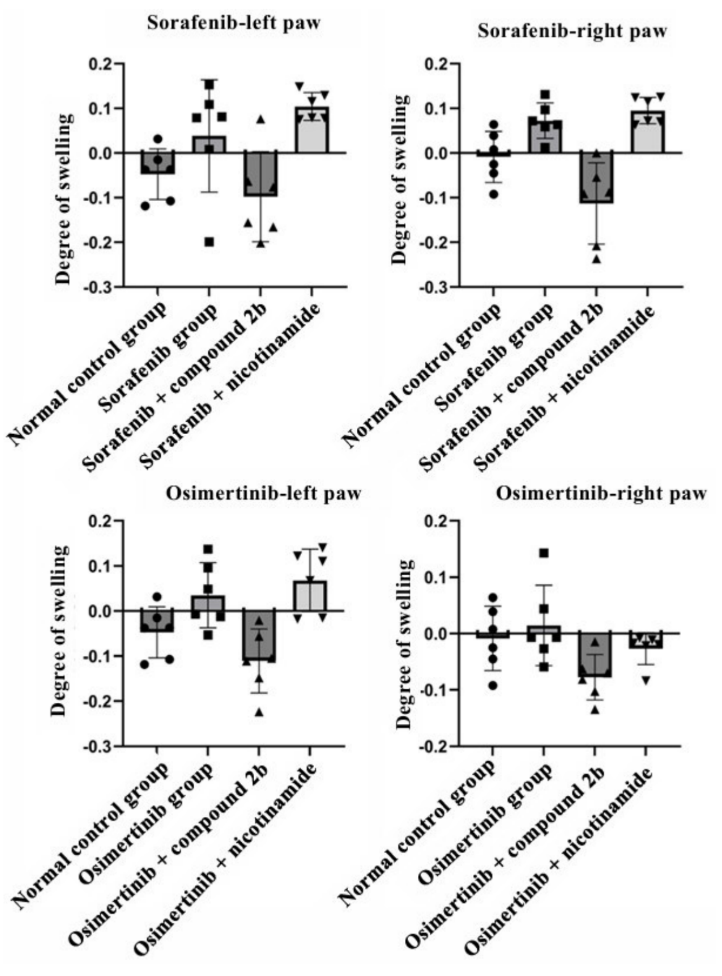
Sorafenib-left paw
Sorafenib-right paw
Degree of swelling
0.2
0.1
0.0
-0.1
-0.2
-0.3
Normal control group
Sorafenib group
Sorafenib + compound 2b
Sorafenib + nicotinamide
Osimertinib-left paw
Osimertinib-right paw
Osimertinib group
Osimertinib + compound 2b
Osimertinib + nicotinamide

FIG. 17

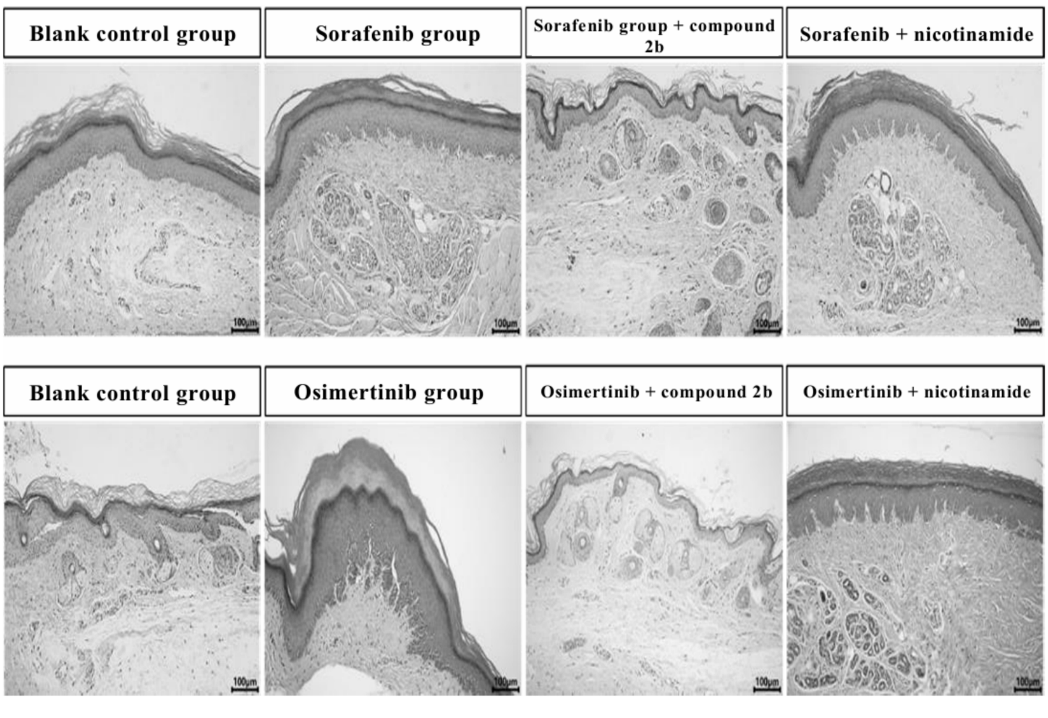
Blank control group
Sorafenib group
Sorafenib group + compound 2b
Sorafenib + nicotinamide
Blank control group
Osimertinib group
Osimertinib + compound 2b
Osimertinib + nicotinamide

FIG. 18

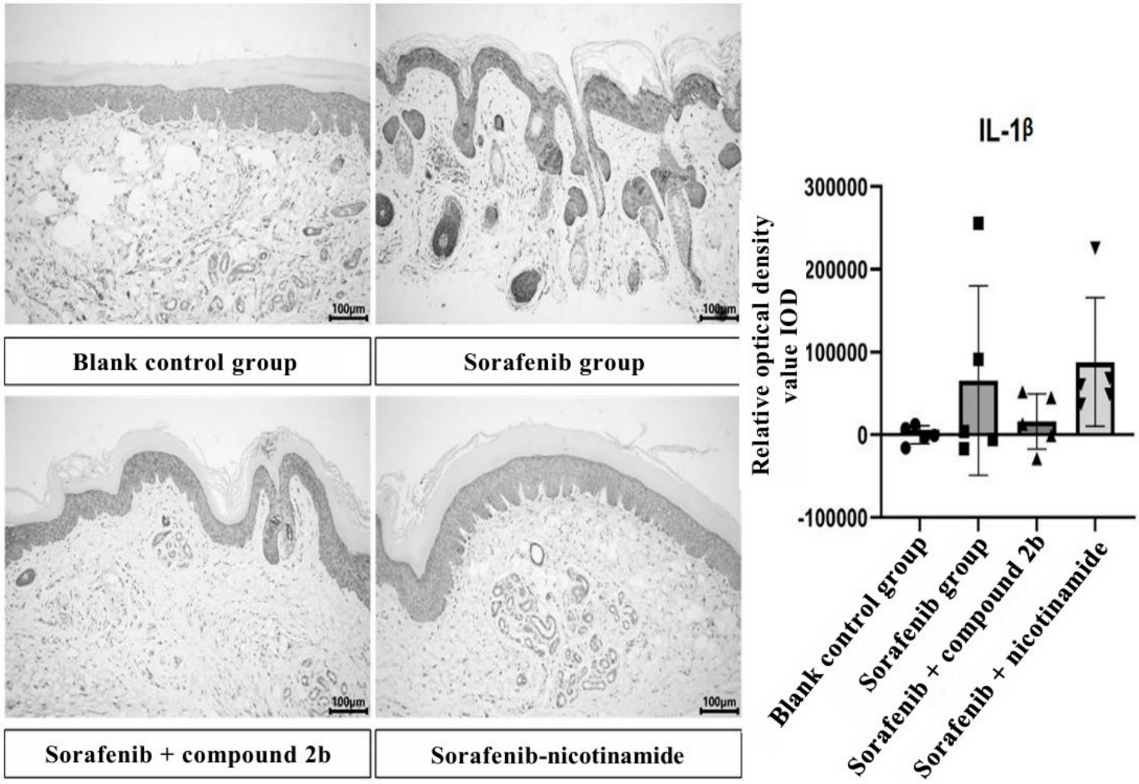

Blank control group
Sorafenib group
Sorafenib + compound 2b
Sorafenib-nicotinamide
100μm
IL-1β
Relative optical density value IOD
300000
200000
100000
0
-100000
Blank control group
Sorafenib group
Sorafenib + compound 2b
Sorafenib + nicotinamide


FIG. 19

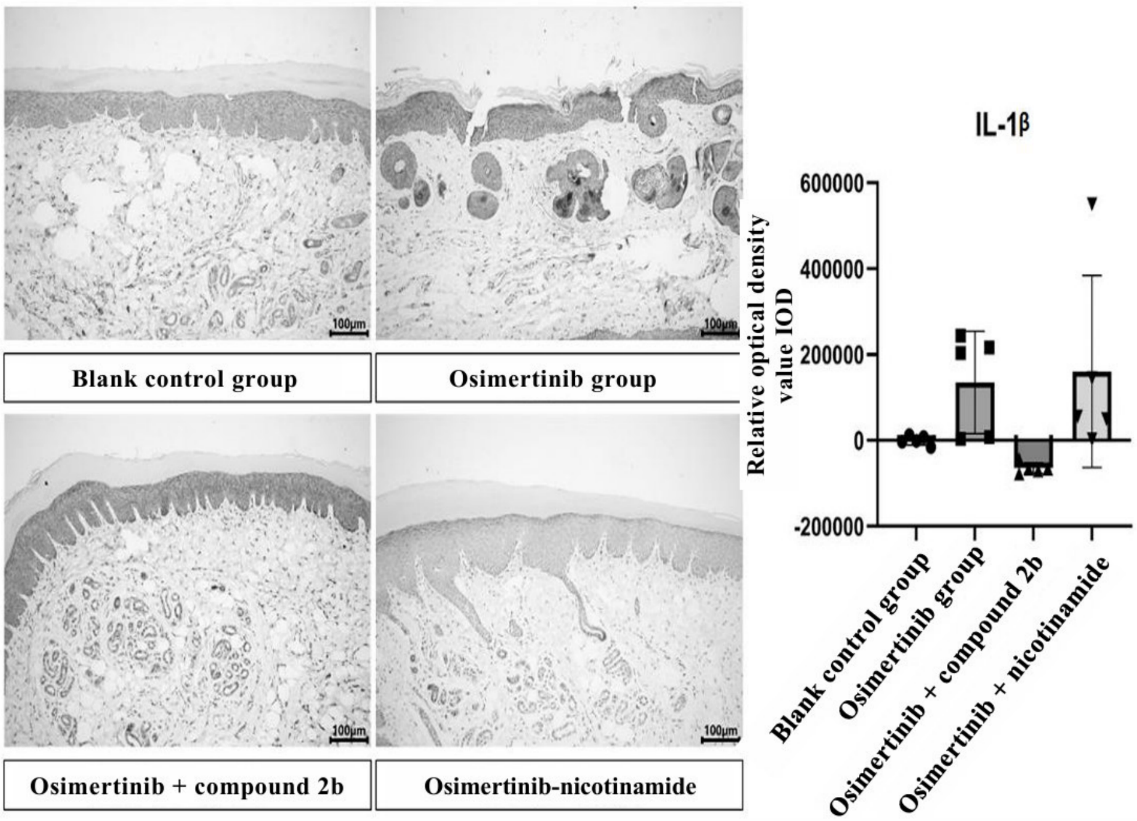

Blank control group
Osimertinib group
Osimertinib + compound 2b
Osimertinib-nicotinamide
100μm
IL-1β
Relative optical density value IOD
600000
400000
200000
0
-200000
Blank control group
Osimertinib group
Osimertinib + compound 2b
Osimertinib + nicotinamide


FIG. 20

## INTERNATIONAL SEARCH REPORT

International application No.
**PCT/CN2024/103648**

**A. CLASSIFICATION OF SUBJECT MATTER**

C07D209/88(2006.01)i; C07D209/90(2006.01)i; A61K31/403(2006.01)i; A61P17/00(2006.01)i

According to International Patent Classification (IPC) or to both national classification and IPC

**B. FIELDS SEARCHED**

Minimum documentation searched (classification system followed by classification symbols)

IPC: C07D: A61K; A61P

Documentation searched other than minimum documentation to the extent that such documents are included in the fields searched

Electronic data base consulted during the international search (name of data base and, where practicable, search terms used)

CNTXT, ENTXT, DWPI, CNKI, Caplus(STN), Registry(STN): 苏州湃芮生物, 王靖方, 彭程, 宋治东, 王莹, 仝思雨, 吲哚, 咔唑, 苯, 皮肤, indole, carbazole, benzene, skin, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2007047604 A2 (ELIXIR PHARMACEUTICALS, INC. et al.) 26 April 2007 (2007-04-26)<br>description, pages 32-37 and 56, and claims 1-29 | 1-33, 36 |
| X | US 2009022694 A1 (DISTEFANO PETER) 22 January 2009 (2009-01-22)<br>description, paragraphs 50, 192 and 231 | 1-33, 36 |
| X | STN. "CAS RN 2328285-59-0 et al."<br>*Registry*, 11 June 2019 (2019-06-11),<br>1-133 | 1, 3-25, 27-28, 33, 36 |
| PX | STN. "CAS RN 3035145-48-0 et al."<br>*Registry*, 30 April 2024 (2024-04-30),<br>1-2 | 1, 3-25, 27-28, 33, 36 |
| A | CN 104311472 A (ARENA PHARMACEUTICALS, INC.) 28 January 2015 (2015-01-28)<br>description, paragraphs 371-375, claims 1-53, and abstract | 1-38 |
| A | CN 101945862 A (ARENA PHARMACEUTICALS, INC.) 12 January 2011 (2011-01-12)<br>description, paragraphs 479-496, claims 1-51, and abstract | 1-38 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 September 2024** | **23 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/103648**

| C. DOCUMENTS CONSIDERED TO BE RELEVANT | | |
|---|---|---|
| Category* | Citation of document, with indication, where appropriate, of the relevant passages | Relevant to claim No. |
| A | WO 2012099952 A2 (ALBANY MOLECULAR RESEARCH, INC. et al.) 26 July 2012 (2012-07-26)<br>entire document | 1-38 |

Form PCT/ISA/210 (second sheet) (July 2022)

**INTERNATIONAL SEARCH REPORT**

International application No.
**PCT/CN2024/103648**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **34, 37**
because they relate to subject matter not required to be searched by this Authority, namely:

Claims 34 and 37 relate to a disease diagnosis and treatment method (PCT Rule 39.1(iv)), and the following search is performed on the basis of the pharmaceutical use of the compound or composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/103648**

| Patent document cited in search report | | | Publication date (day/month/year) | Patent family member(s) | | | Publication date (day/month/year) |
|---|---|---|---|---|---|---|---|
| WO | 2007047604 | A2 | 26 April 2007 | None | | | |
| US | 2009022694 | A1 | 22 January 2009 | WO | 2007047604 | A2 | 26 April 2007 |
| | | | | WO | 2007047604 | A3 | 13 September 2007 |
| CN | 104311472 | A | 28 January 2015 | None | | | |
| CN | 101945862 | A | 12 January 2011 | AU | 2008338965 | A1 | 25 June 2009 |
| | | | | ATE | 531711 | T1 | 15 November 2011 |
| | | | | EP | 2222668 | A1 | 01 September 2010 |
| | | | | EP | 2222668 | B1 | 02 November 2011 |
| | | | | US | 2010273806 | A1 | 28 October 2010 |
| | | | | JP | 2011506600 | A | 03 March 2011 |
| | | | | CA | 2707513 | A1 | 25 June 2009 |
| | | | | WO | 2009078983 | A1 | 25 June 2009 |
| WO | 2012099952 | A2 | 26 July 2012 | CA | 2823955 | A1 | 26 July 2012 |
| | | | | WO | 2012099952 | A3 | 24 January 2013 |
| | | | | EP | 2668191 | A2 | 04 December 2013 |
| | | | | EP | 2668191 | A4 | 20 August 2014 |
| | | | | AU | 2012207335 | A1 | 25 July 2013 |
| | | | | US | 2012184531 | A1 | 19 July 2012 |
| | | | | US | 9067949 | B2 | 30 June 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310814278
- CN 202410257182
- CN 202410340315

### Non-patent literature cited in the description


- **MARIA VASTARELLA et al.** *Drug Safety*, 2020, vol. 43 (5), 395-408
- **AUSTIN GRECO et al.** *Cancers*, 2019, vol. 11 (12), 1950
- **SAMANTHA R. ELLIS et al.** *Journal of the American Academy of Dermatology*, 2022, vol. 83 (4), 1130-1143
- **FRYE M. et al.** *Science*, 2018, vol. 361, 2073-2092
- **YANG C. et al.** *Cell Death & Disease*, 2020, vol. 11, 960
- **MEYER K.D.** ; **JAFFREY S.R.** *Nature Review Molecular Cell Biology*, 2014, vol. 15, 313-326
- **HSU P.J. et al.** *Journal of Biological Chemistry*, 2019, vol. 294, 19889-19895
- **YAO Y. et al.** *FASEB Journal*, 2019, vol. 33, 7529-7544
- **LI T.D. et al.** *Cancer Cell*, 2020, vol. 20, 239
- **CUI Y.H. et al.** *Nature Communications*, 2021, vol. 12, 2183
- **LEE J. et al.** *EMBO J.*, 2021, vol. 40 (8), e106276
- **LIANG D.F. et al.** *Autophage*, 2022, vol. 18 (6), 1318-1337
- **WANG Y. et al.** *Front. Cell. Dev. Biol.*, 2020, vol. 8, 591629
- **CUI L. et al.** *J. Investig. Dermatol.*, 2020, vol. 140 (7), S6
- **LI L. et al.** *Mol. Immunol.*, 2018, vol. 93, 55-63
- **KUMPER M. et al.** *Am. J. Physiol. Cell. Physiol.*, 2022, vol. 323 (4), 1290-1303